# EUROPÄISCHE PATENTSCHRIFT

(11) **EP 3 024 822 B1**
(45) Veröffentlichungstag und Bekanntmachung des Hinweises auf die Patenterteilung: **03.05.2017**
(21) Anmeldenummer: 14741869.3
(22) Anmeldetag: 21.07.2014
(51) Int. Cl.: C07D 215/227, C07D 221/04, C07D 401/12, C07D 413/12, C07D 471/04, A61K 31/435, A61K 31/4704, A61K 31/4709, A61P 1/00, A61P 7/02, A61P 7/10, A61P 27/02, A61P 29/00

(54) **SUBSTITUIERTE OXOPYRIDIN-DERIVATE UND IHRE VERWENDUNG ALS FAKTOR XIA UND PLASMAKALLIKREIN INHIBITOREN**
SUBSTITUTED OXOPYRIDINE DERIVATIVES AND THEIR USE AS FACTOR XIA AND PLASMA KALLIKREIN INHIBITORS
DÉRIVÉS D'OXOPYRIDINE SUBSTITUÉS ET LEUR UTILISATION EN TANT QU'INHIBITEURS DU FACTEUR XIA ET DE LA KALLICRÉINE PLASMATIQUE

(30) Priorität: 23.07.2013 EP 13177605
(43) Veröffentlichungstag der Anmeldung: 01.06.2016
(73) Patentinhaber: Bayer Pharma Aktiengesellschaft, 13353 Berlin (DE)
(72) Erfinder: RÖHRIG, Susanne, 40724 Hilden (DE); HILLISCH, Alexander, 42651 Solingen (DE); STRASSBURGER, Julia, 42115 Wuppertal (DE); HEITMEIER, Stefan, 42489 Wülfrath (DE); SCHMIDT, Martina, Victoria, 51063 Köln (DE); SCHLEMMER, Karl-Heinz, 42113 Wuppertal (DE); BUCHMÜLLER, Anja, 45259 Essen (DE); GERDES, Christoph, 51063 Köln (DE); TELLER, Henrik, 18258 Schwaan (DE); SCHÄFER, Martina, 13465 Berlin (DE); TERSTEEGEN, Adrian, 42111 Wuppertal (DE)
(74) Vertreter: BIP Patents
(86) Internationale Anmeldenummer: PCT/EP2014/065608
(87) Internationale Veröffentlichungsnummer: WO 2015/011087

(56) Entgegenhaltungen:
- WO-A1-2013/093484

## Beschreibung

Die Erfindung betrifft substituierte Oxopyridin-Derivate und Verfahren zu ihrer Herstellung sowie ihre Verwendung zur Herstellung von Arzneimitteln zur Behandlung und/oder Prophylaxe von Krankheiten, insbesondere von Herz-Kreislauf-Erkrankungen, vorzugsweise von thrombotischen beziehungsweise thromboembolischen Erkrankungen sowie von Ödemen, als auch von ophthalmologischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden beziehungsweise minimiert werden. Die Blutstillung nach Gefäßverletzung erfolgt im Wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommen den Faktoren Xa und IIa (Thrombin) Schlüsselrollen zu: Faktor Xa bündelt die Signale der beiden Gerinnungswege, da er sowohl über Faktor VIIa/Tissue Factor (extrinsischer Weg) wie auch den Tenase Komplex (intrisischer Weg) durch Umsetzung von Faktor X entsteht. Die aktivierte Serinprotease Xa spaltet Prothrombin zu Thrombin, das über eine Reihe von Umsetzungen die Impulse aus der Kaskade auf den Gerinnungsstatus des Blutes überträgt.

In der jüngeren Vergangenheit ist die traditionelle Theorie der zwei getrennten Bereiche der Koagulationkaskade (extrinsischer beziehungsweise intrinsischer Pfad) aufgrund neuer Erkenntnisse modifiziert worden: In diesen Modellen wird die Koagulation durch Bindung von aktiviertem Faktor VIIa an Tissue Faktor (TF) initiiert. Der entstandene Komplex aktiviert Faktor X, was wiederum zur Thrombin-Generierung mit anschließender Herstellung von Fibrin und Thrombozyten-Aktivierung (via PAR-1) als verletzungsverschließende Endprodukte der Hämostase führt. Im Vergleich zur anschließenden Amplifikations-/Propagationsphase ist die Geschwindigkeit der Thrombinherstellung in dieser ersten Phase klein und durch das Auftreten von TFPI als Hemmer des TF-FVIIa-FX-Komplexes zeitlich begrenzt.

Ein zentraler Bestandteil des Übergangs von der Initiation zur Amplifikation und Propagation der Koagulation ist Faktor XIa: Thrombin aktiviert in positiven Rückkopplungsschleifen neben Faktor V und Faktor VIII auch Faktor XI zu Faktor XIa, der Faktor IX zu Faktor IXa umsetzt und über den so generierten Faktor IXa/Faktor VIIIa-Komplex die Faktor X-Aktivierung und damit wiederum die Thrombinbildung stark stimuliert, was zu starkem Thrombuswachstum führt und den Thrombus stabilisiert.

Darüber hinaus ist in den Blickpunkt gerückt, dass neben der Stimulation über Tissue Faktor die Aktivierung des Gerinnungssystems an insbesondere negativ geladenen Oberflächen erfolgen kann, zu denen neben Oberflächenstrukturen körperfremder Zellen (z.B. Bakterien) auch artifizielle Oberflächen wie Gefäßprothesen, Stents und extrakorporale Kreisläufe gehören. Auf der Oberfläche findet zunächst die Aktivierung von Faktor XII (FXII) zu Faktor XIIa statt, der im Folgenden an Zelloberflächen gebundenen Faktor XI zu Faktor XIa aktiviert. Dieser führt wie zuvor beschrieben zur weiteren Aktivierung der Gerinnungskaskade. Daneben aktiviert Faktor XIIa ebenfalls gebundenes Plasmaprokallikrein zu Plasmakallikrein (PK), das zum einen zu weiterer Faktor XII-Aktivierung im Rahmen einer Potentierungsschleife führt, was insgesamt eine Verstärkung der Initiation der Gerinnungskaskade zur Folge hat. Zusätzlich stellt PK eine wichtige Bradikinin-freisetzende Protease dar, die somit unter anderem zum Anstieg der endothelialen Permeabilität führt. Als weitere Substrate wurden Prorenin und Prourokinase beschrieben, deren Aktivierung die regulatorischen Prozesse des Renin-Angiotensin-Systems und der Fibrinolyse beeinflussen kann. Damit stellt die Aktivierung von PK ein wichtiges Bindeglied zwischen koagulativen und inflammatorischen Prozessen dar.

Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Darüber hinaus kann eine systemische Hyperkoagulabilität zur systemweiten Bildung von Thromben und schließlich zu einer Verbrauchskoagulopathie im Rahmen einer disseminierten intravasalen Gerinnung führen. Thromboembolische Komplikationen können ferner in extrakorporalen Blutkreisläufen, wie z. B. während einer Hämodialyse, sowie in Gefäß- beziehungsweise Herzklappenprothesen und Stents auftreten.

Im Verlauf vieler Herzkreislauf- und Stoffwechselerkrankungen kommt es infolge systemischer Faktoren, wie z.B. Hyperlipidämie, Diabetes oder Rauchen, infolge von Blutflußveränderungen mit Stase, wie z.B. beim Vorhofflimmern, oder infolge pathologischer Gefäßwandveränderungen, z.B. endothelialer Dysfunktionen oder Atherosklerose, zu einer erhöhten Neigung von Gerinnungs- und Thrombozytenaktivierung. Diese unerwünschte und überschießende Aktivierung der Gerinnung kann durch Bildung fibrin- und plättchenreicher Thromben zu thromboembolischen Erkrankungen und thrombotischen Komplikationen mit lebensbedrohlichen Zuständen führen. Hierbei können auch entzündliche Prozesse involviert sein. Thromboembolische Erkrankungen gehören daher nach wie vor zu den häufigsten Ursachen von Morbidität und Mortalität in den meisten industrialisierten Ländern.

Die aus dem Stand der Technik bekannten Antikoagulantien, d.h. Stoffe zur Hemmung oder Verhinderung der Blutgerinnung, weisen verschiedene, Nachteile auf. Eine effiziente Behandlungsmethode beziehungsweise Prophylaxe von thrombotischen/thromboembolischen Erkrankungen erweist sich in der Praxis deshalb als sehr schwierig und unbefriedigend.

In der Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im Folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen. Niedermolekulare Heparine besitzen zwar eine geringere Wahrscheinlichkeit zur Ausbildung einer Heparin-induzierten Thrombocytopenie, sind aber auch nur subkutan applizierbar. Dies gilt auch für Fondaparinux, einem synthetisch hergestellten, selektiven Faktor Xa Inhibitor mit einer langen Halbwertszeit.

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig. Darüber hinaus sind weitere Nebenwirkungen wie gastrointestinale Störungen, Haarausfall und Hautnekrosen beschrieben.

Neuere Ansätze für orale Antikoagulantien befinden sich in verschiedenen Phasen der klinischen Erprobung beziehungsweise im klinischen Einsatz und haben ihre Wirksamkeit in verschiedenen Studien unter Beweis gestellt. Allerdings kann es auch unter der Einnahme dieser Arzneimittel insbesondere bei prädisponierten Patienten zu Blutungskomplikationen kommen. Daher ist bei antithrombotischen Arzneimitteln ist die therapeutische Breite von zentraler Bedeutung: Der Abstand zwischen der therapeutisch wirksamen Dosis zur Gerinnungshemmung und der Dosis, bei der Blutungen auftreten können, sollte möglichst groß sein, so dass eine maximale therapeutische Wirksamkeit bei minimalem Risikoprofil erreicht wird.

In verschiedenen in-vitro und in-vivo Modellen mit beispielsweise Antikörpern als Faktor XIa Inhibitoren, aber auch in Faktor XIa-Knock-out-Modellen, wurde der anti-thrombotische Effekt bei geringer/keiner Verlängerung der Blutungszeit oder Vergrößerung des Blutvolumens belegt. In klinischen Studien waren erhöhte Faktor XIa-Spiegel mit einer gesteigerten Ereignisrate assoziiert. Dagegen führte Faktor XI-Defizienz (Hämophilie C) nicht zu spontanen Blutungen und fiel nur im Rahmen von Operationen und Traumen auf, zeigte aber eine Protektion gegenüber bestimmten thromboembolischen Ereignissen.

Daneben ist Plasmakallikrein (PK) mit weiteren Erkrankungen assoziert, die mit erhöhten Gefäßpermeabilitäten oder chronisch-entzündlichen Erkrankungen einhergehen, wie es z.B. bei der diabetischen Retinopathie, dem Makulaödem und dem hereditären Angioödem beziehungsweise chronisch-entzündlichen Darmerkrankungen der Fall ist. Der diabetischen Retinopathie liegt in erster Linie eine Mikrogefäßschwäche zu Grunde, in deren Folge es zu einer Basalmembranverdickung der Gefäße und zum Verlust von gefäß-ummantelnden Perizyten, später zum Gefäßverschluss mit retinaler Ischämie kommt, welche aufgrund der hervorgerufenen retinale Hypoxie zu verstärkter Gefäßpermeabilität mit nachfolgender Ausbildung eines Makulaödems und aufgrund aller vorliegender Prozesse zur Erblindung des Patienten führen kann. Beim Hereditären Angioödem (HAE) kommt es durch verminderte Bildung des physiologischen Kallikrein-Inhibitors C1-Esterase-Inhibitors zur unkontrollierten Plasmakallikrein-Aktivierung zu Entzündungen mit fulminanter Ödembildung und starken Schmerzen. Aus tierexperimentellen Ansätzen gibt es Hinweise, dass die Inhibition von Plasmakallikrein die erhöhte Gefäßpermeabilität inhibiert und somit die Ausbildung eines Makulaödems beziehungsweise der diabetischen Retinopathie verhindern beziehungsweise die akute Symptomatik des HAE verbessern kann. Orale Plasmakallikrein-Inhibitoren könnten ebenfalls zur Prophylaxe des HAE eingesetzt werden.

Bei der Progression von chronisch-entzündlichen Darmerkrankungen (CED) haben vor allem die mittels Plasmakallikrein generierten Kinine eine tragende Rolle. Deren pro-inflammatorische Wirkung über Aktivierung von Bradykinin-Rezeptoren induziert und potenziert den Krankheitverlauf. Studien an Morbus Crohn-Patienten zeigen eine Korrelation zwischen der Kallikrein-Konzentration im Darmepithel und dem Grad der Darmentzündung. Eine Aktivierung des Kallikrein-Kinin-Systems wurde ebenfalls in tierexperimentellen Studien beobachtet. Eine Hemmung der Bradykinin-Synthese durch Kallikrein-Inhibitoren könnte demnach auch zur Prophylaxe und/oder Therapie von chronisch-entzündlichen Darmerkrankungen eingesetzt werden.

Weiterhin kann auch die Kombination von antithrombotischen und antiinflammtorischen Prinzipien für viele Erkrankungen besonders attraktiv sein, um die wechselseitige Verstärkung von Koagulation und Inflammation zu unterbinden.

Eine Aufgabe der vorliegenden Erfindung ist daher die Bereitstellung neuer Verbindungen zur Behandlung von Herz-Kreislauf-Erkrankungen, insbesondere von thrombotischen beziehungsweise thromboembolischen Erkrankungen, und/oder ödematösen Erkrankungen, und/oder ophthalmologischen Erkrankungen, insbesondere von diabetischer Retinopathie beziehungsweise des Makulaödems, bei Menschen und Tieren, die eine große therapeutische Bandbreite aufweisen.

WO 2006/030032 beschreibt unter anderem substituierte Pyridinone als allosterische Modulatoren des mGluR2 Rezeptors und WO 2008/079787 beschreibt substituierte Pyridin-2-one und ihre Verwendung als Glucokinase Aktivatoren.

Gegenstand der Erfindung sind Verbindungen der Formel 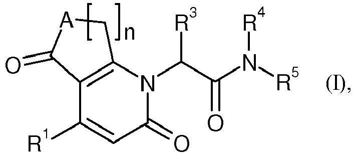 in welcher
- n: für die Zahl 1 oder 2 steht,
- A: für -N(R²)- oder -CH₂- steht,
wobei
R² für Wasserstoff oder C₁-C₄-Alkyl steht,
- R¹: für eine Gruppe der Formel 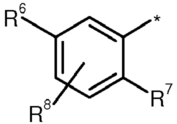 steht,
wobei * die Anknüpfstelle an den Oxopyridinring ist,
R⁶ für Brom, Chlor, Fluor, Methyl, Difluormethyl, Trifluormethyl, Methoxy, Difluormethoxy oder Trifluormethoxy steht,
R⁷ für Wasserstoff, Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
R⁸ für Wasserstoff, Chlor oder Fluor steht,
- R³: für Wasserstoff steht,
- R⁴: für Wasserstoff steht,
- R⁵: für eine Gruppe der Formel 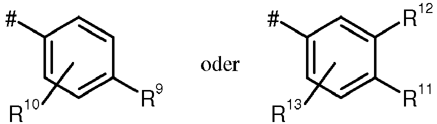 steht,
wobei # die Anknüpfstelle an das Stickstoffatom ist,
- R⁹: für Hydroxycarbonyl oder 5-gliedriges Heterocyclyl steht,
wobei Heterocyclyl substituiert sein kann mit 1 bis 2 Substituenten unabhängig voneinander ausgewählt aus der Gruppe bestehend aus Oxo, Hydroxy, Thioxo, Sulfanyl, Methyl, Difluormethyl, Trifluormethyl, 2-Hydroxycarbonyl-1,1,2,2-tetrafluorethyl und 2-Methoxycarbonyl-1,1,2,2-tetrafluorethyl,
worin Methyl substituiert sein kann mit einem Substituenten Methoxy,
- R¹⁰: für Wasserstoff, Chlor, Fluor oder Methyl steht,
- R¹¹ und R¹²: zusammen mit den Kohlenstoffatomen an die sie gebunden sind einen 5-gliedrigen Heterocyclus bilden,
wobei der Heterocyclus substituiert sein kann mit 1 bis 2 Substituenten unabhängig voneinander ausgewählt aus der Gruppe bestehend aus Oxo, Chlor, Hydroxy, Hydroxycarbonyl, Methyl, Difluormethyl, Trifluormethyl, 1,1,2,2,2-Pentafluorethyl, 2-Hydroxycarbonyl-1,1,2,2-tetrafluorethyl und 2-Methoxy-carbonyl-1,1,2,2-tetrafluorethyl,
- R¹³: für Wasserstoff, Chlor, Fluor, Methyl oder Methoxy steht,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler beziehungsweise chiraler Phase.

Sofern die erfindungsgemäßen Verbindungen in tautomeren Formen vorkommen können, umfasst die vorliegenden Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin, N-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Beschrieben werden auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Im Rahmen der vorliegenden Erfindung haben die Substituenten, soweit nicht anders spezifiziert, die folgende Bedeutung:
Alkyl steht für einen linearen oder verzweigten Alkylrest mit 1 bis 5 Kohlenstoffatomen, bevorzugt 1 bis 3 Kohlenstoffatomen, beispielhaft und vorzugsweise für Methyl, Ethyl, n-Propyl, iso-Propyl, 2-Methyl-prop-1-yl, n-Butyl, *tert.*-Butyl und 2,2-Dimethylprop-1-yl.

Cycloalkyl steht für eine monocyclische Cycloalkylgruppe mit 3 bis 6 Kohlenstoffatomen, beispielhaft und vorzugsweise für Cycloalkyl seien genannt Cyclopropyl, Cyclobutyl, Cyclopentyl und Cyclohexyl.

5-gliedriges Heterocyclyl in der Definition des Restes R⁹ steht für einen gesättigten, teilweise ungesättigten oder aromatischen monocyclischen Rest mit 5 Ringatomen und bis zu 4 Heteroatomen aus der Reihe S, O und N, wobei ein Stickstoffatom auch ein N-Oxid bilden kann, beispielhaft und vorzugsweise für Thienyl, Furyl, Pyrrolyl, Thiazolyl, Oxazolyl, Isoxazolyl, Oxadiazolyl, Pyrazolyl, Imidazolyl, Triazolyl und Tetrazolyl.

5-gliedrige Heterocyclus in der Definition der Reste R¹¹ und R¹² steht für einen gesättigten, teilweise ungesättigten oder aromatischen monocyclischen Rest mit 5 Ringatomen und bis zu 2 Heteroatomen aus der Reihe S, O und N, wobei ein Stickstoffatom auch ein N-Oxid bilden kann.

Dieser 5-gliedrige Heterocyclus steht zusammen mit dem Phenylring, an den er gebunden ist, beispielhaft und vorzugsweise für 2,3-Dihydro-1-benzothiophen-5-yl, 1,3-Dihydro-2-benzothiophen-5-yl, 2,3-Dihydro-1-benzofuran-5-yl, 1,3-Dihydro-2-benzofuran-5-yl, Indolin-5-yl, Isoindolin-5-yl, 2,3-Dihydro-1H-indazol-5-yl, 2,3-Dihydro-1H-benzimidazol-5-yl, 1,3-Dihydro-2,1-benzoxazol-5-yl, 2,3-Dihydro-1,3-benzoxazol-5-yl, 1,3-Dihydro-2,1-benzothiazol-5-yl, 2,3-Dihydro-1,3-benzothiazol-5-yl, 1H-Benzimidazol-5-yl, 1H-Indazol-5-yl, 1,2-Benzoxazol-5-yl, Indol-5-yl, Isoindol-5-yl, Benzofuran-5-yl, Benzothiophen-5-yl, 2,3-Dihydro-1-benzothiophen-6-yl, 1,3-Dihydro-2-benzothiophen-6-yl, 2,3-Dihydro-1-benzofuran-6-yl, 1,3-Dihydro-2-benzofuran-6-yl, Indolin-6-yl, Isoindolin-6-yl, 2,3-Dihydro-1H-indazol-6-yl, 2,3-Dihydro-1H-benzimidazol-6-yl, 1,3-Dihydro-2,1-benzoxazol-6-yl, 2,3-Dihydro-1,3-benzoxazol-6-yl, 1,3-Dihydro-2,1-benzothiazol-6-yl, 2,3-Dihydro-1,3-benzothiazol-6-yl, 1H-Benzimidazol-6-yl, 1H-Indazol-6-yl, 1,2-Benzoxazol-6-yl, Indol-6-yl, Isoindol-6-yl, Benzofuran-6-yl und Benzothiophen-6-yl.

4- bis 6-gliedrige Oxo-Heterocyclyl in der Definition des Restes R³ steht für einen gesättigten monocyclischen Rest mit 4 bis 6 Ringatomen, in dem ein Ringatom ein Sauerstoffatom ist, beispielhaft und vorzugsweise für Oxetanyl, Tetrahydrofuranyl und Tetrahydro-2H-pyranyl.

In den Formeln der Gruppe, die für R¹ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Atom, an das R¹ gebunden ist.

In den Formeln der Gruppe, die für R⁵ stehen kann, steht der Endpunkt der Linie, neben der jeweils ein # steht, nicht für ein Kohlenstoffatom beziehungsweise eine CH₂-Gruppe sondern ist Bestandteil der Bindung zu dem Atom, an das R⁵ gebunden ist.

Bevorzugt sind Verbindungen der Formel (I), in welcher
- n: für die Zahl 1 oder 2 steht,
- A: für -N(R²)- oder -CH₂- steht,
wobei
R² für Wasserstoff oder C₁-C₄-Alkyl steht,
- R¹: für eine Gruppe der Formel 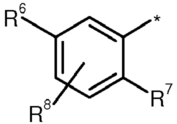 steht,
wobei * die Anknüpfstelle an den Oxopyridinring ist,
R⁶ für Brom, Chlor, Fluor, Methyl, Difluormethyl, Trifluormethyl, Methoxy, Difluormethoxy oder Trifluormethoxy steht,
R⁷ für Wasserstoff, Brom, Chlor, Fluor, Cyano, Nitro, Hydroxy, Methyl, Difluormethyl, Trifluormethyl, Methoxy, Difluormethoxy, Trifluormethoxy, Ethinyl, 3,3,3-Trifluorprop-1-in-1-yl oder Cyclopropyl steht,
R⁸ für Wasserstoff, Chlor oder Fluor steht,
- R³: für Wasserstoff steht,
- R⁴: für Wasserstoff steht,
- R⁵: für eine Gruppe der Formel 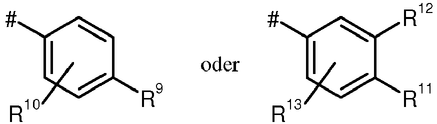 steht,
wobei # die Anknüpfstelle an das Stickstoffatom ist,
- R⁹: für Hydroxycarbonyl oder 5-gliedriges Heterocyclyl steht,
wobei Heterocyclyl substituiert sein kann mit 1 bis 2 Substituenten unabhängig voneinander ausgewählt aus der Gruppe bestehend aus Oxo, Hydroxy, Methyl, Difluormethyl und Trifluormethyl,
worin Methyl substituiert sein kann mit einem Substituenten Methoxy,
- R¹⁰: für Wasserstoff, Chlor, Fluor oder Methyl steht,
- R¹¹ und R¹²: zusammen mit den Kohlenstoffatomen an die sie gebunden sind einen 5-gliedrigen Heterocyclus bilden,
wobei der Heterocyclus substituiert sein kann mit 1 bis 2 Substituenten unabhängig voneinander ausgewählt aus der Gruppe bestehend aus Oxo, Hydroxy, Methyl, Difluormethyl, Trifluormethyl und 1,1,2,2,2-Pentafluorethyl,
- R¹³: für Wasserstoff, Chlor, Fluor oder Methyl steht,
und ihre Salze, ihre Solvate und die Solvate ihrer Salze.

Bevorzugt sind auch Verbindungen der Formel (I), in welcher
- n: für die Zahl 1 oder 2 steht,
- A: für -N(R²)- oder -CH₂- steht,
wobei
R² für Wasserstoff oder Methyl steht,
- R¹: für eine Gruppe der Formel 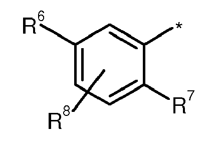 steht,
wobei * die Anknüpfstelle an den Oxopyridinring ist,
R⁶ für Chlor steht,
R⁷ für Wasserstoff, Brom, Chlor, Cyano, Methyl, Difluormethyl, Trifluormethyl, Difluormethoxy oder Trifluormethoxy steht,
R⁸ für Wasserstoff oder Fluor steht,
- R³: für Wasserstoff steht,
- R⁴: für Wasserstoff steht,
- R⁵: für eine Gruppe der Formel 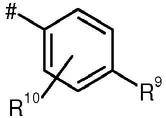 steht,
wobei # die Anknüpfstelle an das Stickstoffatom ist,
R⁹ für Hydroxycarbonyl, Oxadiazolyl, Pyrazolyl, Imidazolyl, Triazolyl oder Tetrazolyl steht,
wobei Oxadiazolyl, Pyrazolyl, Imidazolyl und Triazolyl substituiert sein können mit 1 bis 2 Substituenten unabhängig voneinander ausgewählt aus der Gruppe bestehend aus Oxo, Hydroxy, Methyl und Trifluormethyl,
R¹⁰ für Wasserstoff steht,
und ihre Salze, ihre Solvate und die Solvate ihrer Salze.

Bevorzugt sind auch Verbindungen der Formel (I), in welcher
- n: für die Zahl 1 oder 2 steht,
- A: für -CH₂- steht,
- R¹: für eine Gruppe der Formel 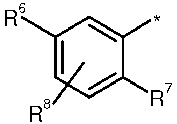 steht,
wobei * die Anknüpfstelle an den Oxopyridinring ist,
R⁶ für Chlor steht,
R⁷ für Brom oder Cyano steht,
R⁸ für Wasserstoff steht,
- R³: für Wasserstoff steht,
- R⁴: für Wasserstoff steht,
- R⁵: für eine Gruppe der Formel 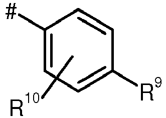 steht,
wobei # die Anknüpfstelle an das Stickstoffatom ist,
R⁹ für Hydroxycarbonyl, Oxadiazolyl, Pyrazolyl oder Tetrazolyl steht,
wobei Oxadiazolyl und Pyrazolyl substituiert sein können mit 1 bis 2 Substituenten unabhängig voneinander ausgewählt aus der Gruppe bestehend aus Oxo, Hydroxy und Trifluormethyl,
R¹⁰ für Wasserstoff steht,
und ihre Salze, ihre Solvate und die Solvate ihrer Salze.

Bevorzugt sind auch Verbindungen der Formel (I), in welcher n für die Zahl 1 steht.

Bevorzugt sind auch Verbindungen der Formel (I), in welcher A für -CH₂- steht.

Bevorzugt sind auch Verbindungen der Formel (I), in welcher
- R¹: für eine Gruppe der Formel 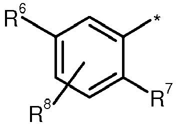 steht,
wobei * die Anknüpfstelle an den Oxopyridinring ist,
R⁶ für Chlor steht,
R⁷ für Brom oder Cyano steht,
R⁸ für Wasserstoff steht.

Bevorzugt sind auch Verbindungen der Formel (I), in welcher
- R¹: für eine Gruppe der Formel 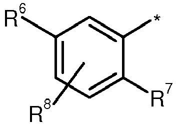 steht,
wobei * die Anknüpfstelle an den Oxopyridinring ist,
R⁶ für Chlor steht,
R⁷ für Cyano oder Difluormethoxy steht,
R⁸ für Wasserstoff steht.

Bevorzugt sind auch Verbindungen der Formel (I), in welcher R³ für Wasserstoff steht.

Bevorzugt sind auch Verbindungen der Formel (I), in welcher
- R⁵: für eine Gruppe der Formel 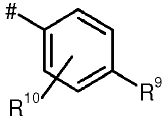 steht,
wobei # die Anknüpfstelle an das Stickstoffatom ist,
R⁹ für Hydroxycarbonyl, Oxadiazolyl, Pyrazolyl, Imidazolyl, Triazolyl oder Tetrazolyl steht,
wobei Oxadiazolyl, Pyrazolyl, Imidazolyl und Triazolyl substituiert sein können mit 1 bis 2 Substituenten unabhängig voneinander ausgewählt aus der Gruppe bestehend aus Oxo, Hydroxy, Methyl und Trifluormethyl,
R¹⁰ für Wasserstoff steht.

Bevorzugt sind auch Verbindungen der Formel (I), in welcher
- R⁵: für eine Gruppe der Formel 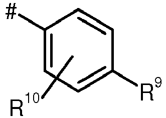 steht,
wobei # die Anknüpfstelle an das Stickstoffatom ist,
R⁹ für Hydroxycarbonyl, Oxadiazolyl, Pyrazolyl oder Tetrazolyl steht,
wobei Oxadiazolyl und Pyrazolyl substituiert sein können mit 1 bis 2 Substituenten unabhängig voneinander ausgewählt aus der Gruppe bestehend aus Oxo, Hydroxy und Trifluormethyl,
R¹⁰ für Wasserstoff steht.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), oder ihrer Salze, ihrer Solvate oder der Solvate ihrer Salze, wobei
[A] die Verbindungen der Formel 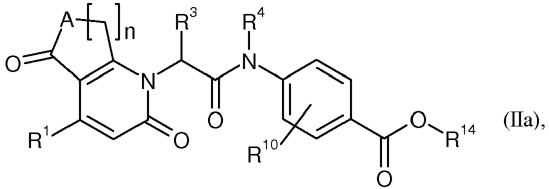 in welcher
   n, A, R¹, R³, R⁴ und R¹⁰ die oben angegebene Bedeutung haben, und
   R¹⁴ für tert-Butyl steht,
   mit einer Säure zu Verbindungen der Formel 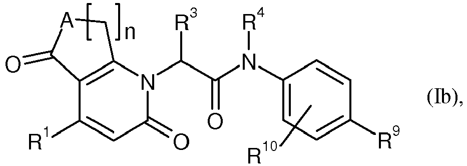 in welcher
   n, A, R¹, R³, R⁴ und R¹⁰ die oben angegebene Bedeutung haben, und
   R⁹ für Hydroxycarbonyl steht,
   umgesetzt werden,
   oder
[B] die Verbindungen der Formel 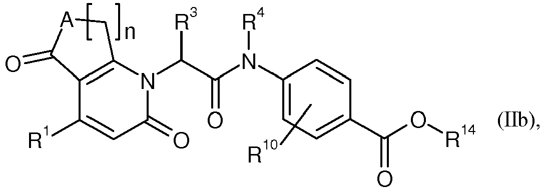 in welcher
   n, A, R¹, R³, R⁴ und R¹⁰ die oben angegebene Bedeutung haben, und
   R¹⁴ für Methyl oder Ethyl steht,
   mit einer Base zu Verbindungen der Formel 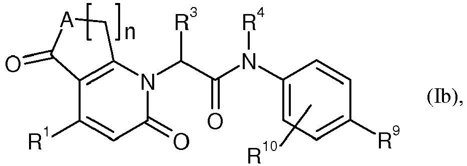 in welcher
   n, A, R¹, R³, R⁴ und R¹⁰ die oben angegebene Bedeutung haben, und
   R⁹ für Hydroxycarbonyl steht,
   umgesetzt werden,
   oder
[C] die Verbindungen der Formel 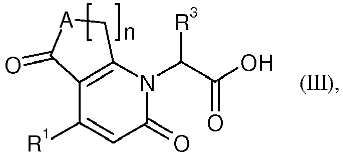 in welcher
   n, A, R¹ und R³ die oben angegebene Bedeutung haben,
   mit Verbindungen der Formel 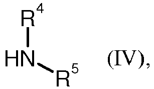 in welcher
   R⁴ und R⁵ die oben angegebene Bedeutung haben,
   in Gegenwart eines Dehydratisierungsreagenzes zu Verbindungen der Formel (I) umgesetzt werden,
   oder
[D] die Verbindungen der Formel 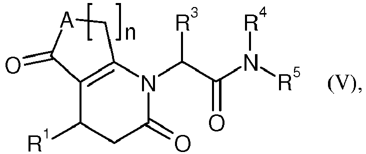 in welcher
   n, A, R¹, R³, R⁴ und R⁵ die oben angegebene Bedeutung haben,
   mit Oxidationsmitteln umgesetzt werden.

Die Verbindungen der Formel (Ib) sind eine Teilmenge der Verbindungen der Formel (I).

Die Verbindungen der Formeln (IIa) und (IIb) bilden zusammen die Menge der Verbindungen der Formel (II).

Die Umsetzung nach Verfahren [A] erfolgt im Allgemeinen in inerten Lösungsmitteln, bevorzugt in einem Temperaturbereich von Raumtemperatur bis 60°C bei Normaldruck.

Inerte Lösungsmittel sind beispielsweise Halogenkohlenwasserstoffe wie Dichlormethan, Trichlormethan, Tetrachlormethan oder 1,2-Dichlorethan, oder Ether wie Tetrahydrofuran oder Dioxan, bevorzugt ist Dichlormethan.

Säuren sind beispielsweise Trifluoressigsäure oder Chlorwasserstoff in Dioxan, bevorzugt ist Trifluoressigsäure.

Die Umsetzung nach Verfahren [B] erfolgt im Allgemeinen in inerten Lösungsmitteln, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist ein Gemisch aus Tetrahydrofuran und Wasser.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Alkoholate wie Kalium- oder Natrium-tert-butylat, bevorzugt ist Lithiumhydroxid.

Die Umsetzung nach Verfahren [C] erfolgt im Allgemeinen in inerten Lösungsmitteln, gegebenenfalls in Gegenwart einer Base, bevorzugt in einem Temperaturbereich von 0°C bis Raumtemperatur bei Normaldruck.

Als Dehydratisierungsreagenzien eignen sich hierbei beispielsweise Carbodiimide wie z.B. *N,N'-*Diethyl-, *N,N'*-Dipropyl-, *N,N'*-Diisopropyl-, *N,N'*-Dicyclohexylcarbodiimid, *N*-(3-Dimethylamino-isopropyl)-*N'*-ethylcarbodiimid-Hydrochlorid (EDC) (gegebenenfalls in Gegenwart von Pentafluorphenol (PFP)), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N,N,N',N'*-tetra-methyluronium-hexafluorophosphat (HBTU), 2-(2-Oxo-1-(2H)-pyridyl)-1,1,3,3-tetramethyluroniumtetrafluoroborat (TPTU), (Benzotriazol-1-yloxy)bisdimethylaminomethyliumfluoroborat (TBTU) oder *O*-(7-Azabenzotriazol-1-yl)-*N,N,N',N'*-tetramethyl-uroniumhexafluorophosphat (HATU), oder 1-Hydroxybenztriazol (HOBt), oder Benzotriazol-1-yloxy-tris(dimethylamino)-phosphoniumhexafluorophosphat (BOP), oder Hydroxyiminocyanessigsäureethylester/N,N'-Diisopropylcarbodiimid, oder Mischungen aus diesen, bevorzugt ist HATU.

Basen sind beispielsweise Alkalicarbonate, wie z.B. Natrium- oder Kaliumcarbonat, oder -hydrogencarbonat, oder organische Basen wie Trialkylamine, z.B. Triethylamin, *N*-Methylmorpholin, *N*-Methylpiperidin, 4-Dimethylaminopyridin oder Diisopropylethylamin, bevorzugt ist Diisopropylethylamin.

Inerte Lösungsmittel sind beispielsweise Halogenkohlenwasserstoffe wie Dichlormethan oder Trichlormethan, Kohlenwasserstoffe wie Benzol, oder andere Lösungsmittel wie Nitromethan, Dioxan, Dimethylformamid, Dimethylsulfoxid oder Acetonitril. Ebenso ist es möglich, Gemische der Lösemittel einzusetzen, bevorzugt ist Dimethylformamid.

Die Umsetzung nach Verfahren [D] erfolgt im Allgemeinen in inerten Lösungsmitteln, bevorzugt in einem Temperaturbereich von Raumtemperatur bis 60°C bei Normaldruck.

Inerte Lösungsmittel sind beispielsweise Halogenkohlenwasserstoffe wie Dichlormethan, Trichlormethan, Tetrachlormethan oder 1,2-Dichlorethan, oder Ether wie Tetrahydrofuran oder Dioxan, oder Gemische von Lösungsmitteln, oder Gemische von Lösungsmittel mit Wasser, bevorzugt ist ein Gemisch aus Dioxan und Wasser.

Oxidationsmittel sind beispielsweise Ammoniumcer(IV)nitrat, 4,5-Dichlor-3,6-dioxocyclohexa-1,4-dien-1,2-dicarbonitril (DDQ), Mangan(IV)oxid, Kaliumpermanganat, Brom, *N-*Bromsuccinimid/Dibenzoylperoxid, bevorzugt ist Ammoniumcer(IV)nitrat.

Die Verbindungen der Formel (IV) sind bekannt, lassen sich nach bekannten Verfahren aus den entsprechenden Ausgangsverbindungen synthetisieren oder können analog den im Beispielteil beschriebenen Verfahren hergestellt werden.

Die Verbindungen der Formel (II) sind bekannt oder können hergestellt werden, indem Verbindungen der Formel 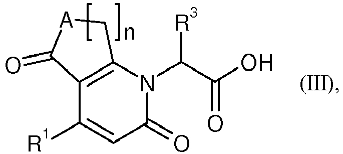 in welcher
n, A, R¹ und R³ die oben angegebene Bedeutung haben,
mit Verbindungen der Formel 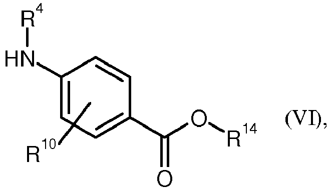 in welcher
R⁴ und R¹⁰ die oben angegebene Bedeutung haben, und
R¹⁴ für Methyl, Ethyl oder tert-Butyl steht,
in Gegenwart eines Dehydratisierungsreagenzes umgesetzt werden.

Die Umsetzung erfolgt wie für Verfahren [C] beschrieben.

Die Verbindungen der Formel (VI) sind bekannt, lassen sich nach bekannten Verfahren aus den entsprechenden Ausgangsverbindungen synthetisieren oder können analog den im Beispielteil beschriebenen Verfahren hergestellt werden.

Die Verbindungen der Formel (III) sind bekannt oder können hergestellt werden, indem

### [E] Verbindungen der Formel

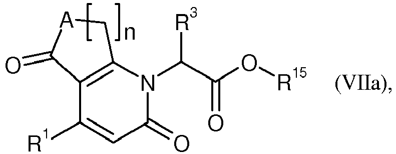

in welcher
n, A, R¹ und R³ die oben angegebene Bedeutung haben, und
R¹⁵ für tert-Butyl steht,
mit einer Säure umgesetzt werden,
oder

### [F] Verbindungen der Formel

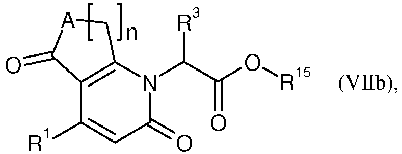

in welcher
n, A, R¹ und R³ die oben angegebene Bedeutung haben, und
R¹⁵ für Methyl oder Ethyl steht,
mit einer Base umgesetzt werden.

Die Verbindungen der Formeln (VIIa) und (VIIb) bilden zusammen die Menge der Verbindungen der Formel (VII).

Die Umsetzung nach Verfahren [E] erfolgt wie für Verfahren [A] beschrieben.

Die Umsetzung nach Verfahren [F] erfolgt wie für Verfahren [B] beschrieben.

Die Verbindungen der Formel (VII) sind bekannt oder können hergestellt werden, indem

### [G] Verbindungen der Formel

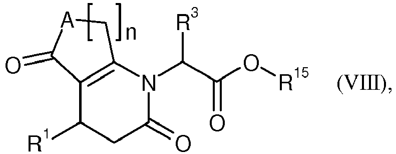

in welcher
n, A, R¹ und R³ die oben angegebene Bedeutung haben, und
R¹⁵ für Methyl, Ethyl oder tert-Butyl steht,
mit Oxidationsmitteln umgesetzt werden.
oder

### [H] Verbindungen der Formel

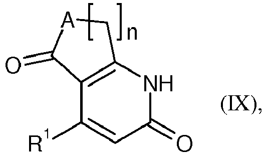

in welcher
n, A und R¹ die oben angegebene Bedeutung haben,
mit Verbindungen der Formel 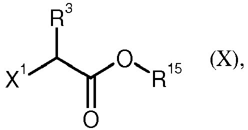 in welcher
R³ die oben angegebene Bedeutung hat,
R¹⁵ für Methyl, Ethyl oder tert-Butyl steht, und
X¹ für Chlor, Brom, Iod, Methansulfonyloxy oder Trifluormethansulfonyloxy steht,
umgesetzt werden,
oder

### [I] Verbindungen der Formel

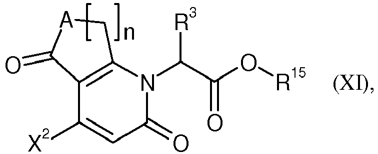

in welcher
n, A und R³ die oben angegebene Bedeutung haben,
R¹⁵ für Methyl, Ethyl oder tert-Butyl steht, und
X² für (Trifluormethyl)sulfonyloxy steht,
mit Verbindungen der Formel

R¹-Q (XII),

in welcher
R¹ die oben angegebene Bedeutung hat, und
Q für -B(OH)₂, einen Boronsäure-Ester, bevorzugt Boronsäurepinakolester, oder -BF₃⁻K⁺ steht,
unter Suzuki-Kupplungsbedingungen umgesetzt werden.

Die Umsetzung nach Verfahren [G] erfolgt wie für Verfahren [D] beschrieben.

Die Umsetzung nach Verfahren [H] erfolgt im Allgemeinen in inerten Lösungsmitteln, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Dichlormethan, Trichlormethan, Tetrachlormethan oder 1,2-Dichlorethan, Alkohole wie Methanol oder Ethanol, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist Dimethylformamid.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Kalium- oder Natrium-tert-butylat, Natriumhydrid oder eine Mischung aus diesen Basen oder eine Mischung aus Natriumhydrid und Lithiumbromid, bevorzugt ist Kaliumcarbonat oder Natriumhydrid oder eine Mischung aus Natriumhydrid und Lithiumbromid.

Die Umsetzung nach Verfahren [I] erfolgt im Allgemeinen in inerten Lösungsmitteln, in Gegenwart eines Katalysators, gegebenenfalls in Gegenwart eines Zusatzreagenzes, gegebenenfalls in einer Mikrowelle, bevorzugt in einem Temperaturbereich von Raumtemperatur bis 150°C bei Normaldruck bis 3 bar.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)acetat/Triscyclohexylphosphin, Tris(dibenzylidenaceton)dipalladium, Bis-(diphenylphosphanferrocenyl)-palladium-(II)-chlorid, 1,3-Bis(2,6-diisopropylphenyl)imidazol-2-yliden(1,4-napthtochinon)palladiumdimer, Allyl(chlor)-(1,3-dimesityl-1,3-dihydro-2H-imidazol-2-yliden)palladium, Palladium(II)acetat/Dicyclohexyl-(2',4',6'-triisopropyl-biphenyl-2-yl)-phosphin, [1,1-Bis-(diphenylphosphino)-ferrocen]-palladium(II)chlorid-Monodichlormethan-addukt oder XPhos Prekatalysator [(2'-Aminobiphenyl-2-yl)(chlor)palladium-Dicyclohexyl(2',4',6'-triisopropylbiphenyl-2-yl)phosphan (1:1)], bevorzugt ist Tetrakistriphenylphosphin-palladium(0), [1,1-Bis-(diphenylphosphino)-ferrocen]-palladium(II)chlorid-Monodichlormethan-addukt oder XPhos Prekatalysator [(2'-Aminobiphenyl-2-yl)(chlor)palladium-Dicyclohexyl(2',4',6'-triisopropylbiphenyl-2-yl)phosphan (1:1)].

Zusatzreagenzien sind beispielsweise Kaliumacetat, Cäsium-, Kalium- oder Natriumcarbonat, Kalium-tert.-butylat, Cäsiumfluorid oder Kaliumphosphat, wobei diese in wässriger Lösung vorliegen können, bevorzugt sind Zusatzreagenzien wie Kaliumcarbonat oder wässrige Kaliumphosphat-Lösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon oder Acetonitril, oder Gemische der Lösungsmittel mit Alkoholen wie Methanol oder Ethanol und/oder Wasser, bevorzugt ist Tetrahydrofuran, Dioxan oder Acetonitril.

Die Verbindungen der Formeln (X) und (XII) sind bekannt oder lassen sich nach bekannten Verfahren aus den entsprechenden Ausgangsverbindungen synthetisieren.

Die Verbindungen der Formel (VIII) sind bekannt oder können hergestellt werden, indem

### [J] Verbindungen der Formel

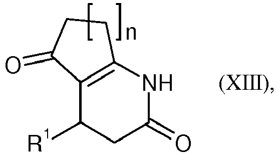

in welcher
n und R¹ die oben angegebene Bedeutung haben,
mit Verbindungen der Formel (X) zu Verbindungen der Formel 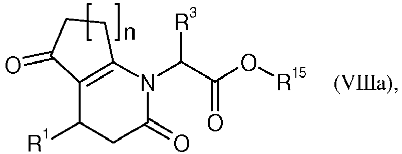 in welcher
n, R¹ und R³ die oben angegebene Bedeutung haben, und
R¹⁵ für Methyl, Ethyl oder tert-Butyl steht,
umgesetzt werden,
oder

### [K] Verbindungen der Formel

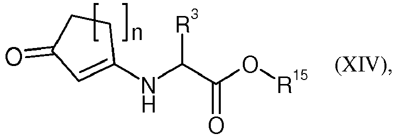

in welcher
n und R³ die oben angegebene Bedeutung haben, und
R¹⁵ für Methyl, Ethyl oder tert-Butyl steht,
mit Verbindungen der Formel 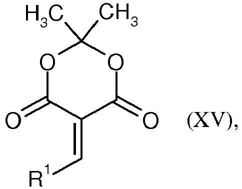 in welcher
R¹ die oben angegebene Bedeutung hat,
zu Verbindungen der Formel 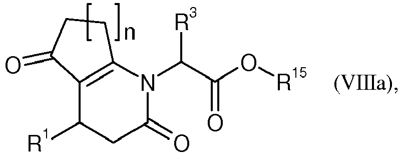 in welcher
n, R¹ und R³ die oben angegebene Bedeutung haben, und
R¹⁵ für Methyl, Ethyl oder tert-Butyl steht,
umgesetzt werden,
oder

### [L] Verbindungen der Formel

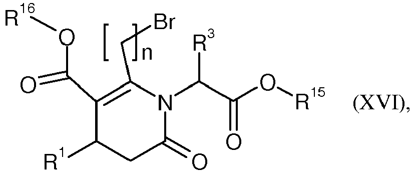

in welcher
n, R¹ und R³ die oben angegebene Bedeutung haben,
R¹⁵ für Methyl, Ethyl oder tert-Butyl steht, und
R¹⁶ für Methyl oder Ethyl steht
mit Verbindungen der Formel

R²-NH₂ (XVII),

in welcher
R² die oben angegebene Bedeutung hat,
zu Verbindungen der Formel 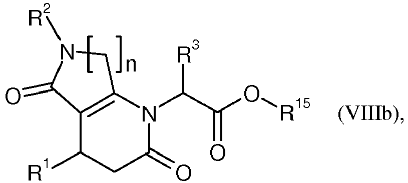 in welcher
n, R¹, R² und R³ die oben angegebene Bedeutung haben, und
R¹⁵ für Methyl, Ethyl oder tert-Butyl steht,
umgesetzt werden.

Die Verbindungen der Formeln (VIIIa) und (VIIIb) bilden zusammen die Menge der Verbindungen der Formel (VIII).

Die Umsetzung nach Verfahren [J] erfolgt wie für Verfahren [H] beschrieben.

Die Umsetzung nach Verfahren [K] erfolgt im Allgemeinen in inerten Lösungsmitteln, bevorzugt in einem Temperaturbereich von Raumtemperatur bis zum Rückfluss der Lösungsmittel, bevorzugt in einem Temperaturbereich von 60°C bis 80°C, bei Normaldruck.

Inerte Lösungsmittel sind beispielsweise Ether wie Dioxan oder Tetrahydrofuran, oder Alkohole wie Ethanol, bevorzugt ist Ethanol.

Die Umsetzung nach Verfahren [L] erfolgt im Allgemeinen in inerten Lösungsmitteln, bevorzugt in einem Temperaturbereich von Raumtemperatur bis 120°C, bevorzugt Raumtemperatur bis 80°C, bei Normaldruck.

Inerte Lösungsmittel sind beispielsweise Ether wie Dioxan oder Tetrahydrofuran, oder Acetonitril, oder Alkohole wie Methanol oder Ethanol, bevorzugt ist Tetrahydrofuran oder Acetonitril.

Die Verbindungen der Formeln (XIV), (XV), (XVI) und (XVII) sind bekannt, lassen sich nach bekannten Verfahren aus den entsprechenden Ausgangsverbindungen synthetisieren oder können analog den im Beispielteil beschriebenen Verfahren hergestellt werden.

Die Verbindungen der Formel (XIII) sind bekannt oder können hergestellt werden, indem Verbindungen der Formel 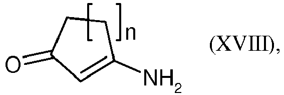 in welcher
n die oben angegebene Bedeutung hat,
in der ersten Stufe mit Verbindungen der Formel (XV) umgesetzt werden,
und gegebenfalls wird in einer zweiten Stufe mit einem Dehydratisierungsreagenz umgesetzt.

Die Umsetzung der ersten Stufe erfolgt im Allgemeinen in inerten Lösungsmitteln, bevorzugt in einem Temperaturbereich von Raumtemperatur bis zum Rückfluss der Lösungsmittel, bevorzugt unter Rückfluss der Lösungsmittel, bei Normaldruck.

Inerte Lösungsmittel sind beispielsweise Ether wie Dioxan oder Tetrahydrofuran, oder Alkohole wie Ethanol, bevorzugt ist Dioxan.

Die Umsetzung der zweiten Stufe erfolgt wie für Verfahren [C] beschrieben.

Die Verbindungen der Formel (XVIII) sind bekannt, lassen sich nach bekannten Verfahren aus den entsprechenden Ausgangsverbindungen synthetisieren oder können analog den im Beispielteil beschriebenen Verfahren hergestellt werden.

Die Verbindungen der Formel (V) sind bekannt oder können hergestellt werden, indem Verbindungen der Formel 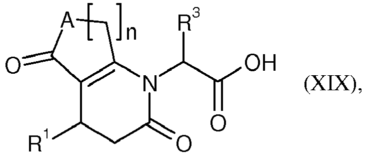 in welcher
n, A, R¹ und R³ die oben angegebene Bedeutung haben,
mit Verbindungen der Formel (IV) in Gegenwart eines Dehydratisierungsreagenzes umgesetzt werden.

Die Umsetzung erfolgt wie für Verfahren [C] beschrieben.

Die Verbindungen der Formel (XIX) sind bekannt oder können hergestellt werden, indem

### [M] Verbindungen der Formel

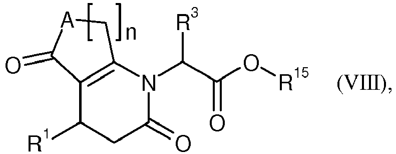

in welcher
n, A, R¹ und R³ die oben angegebene Bedeutung haben, und
R¹⁵ für tert-Butyl steht,
mit einer Säure umgesetzt werden,
oder

### [N] Verbindungen der Formel

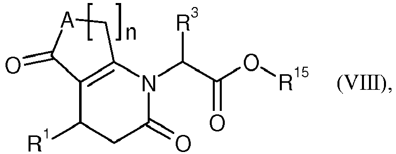

in welcher
n, A, R¹ und R³ die oben angegebene Bedeutung haben, und
R¹⁵ für Methyl oder Ethyl steht,
mit einer Base umgesetzt werden.

Die Umsetzung nach Verfahren [M] erfolgt wie für Verfahren [A] beschrieben.

Die Umsetzung nach Verfahren [N] erfolgt wie für Verfahren [B] beschrieben.

Die Verbindungen der Formel (IX) sind bekannt oder können hergestellt werden, indem Verbindungen der Formel 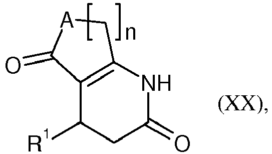 in welcher
n, A und R¹ die oben angegebene Bedeutung haben,
mit Oxidationsmitteln umgesetzt werden.

Die Umsetzung erfolgt wie für Verfahren [D] beschrieben.

Die Verbindungen der Formel (XX) sind bekannt, lassen sich nach bekannten Verfahren aus den entsprechenden Ausgangsverbindungen synthetisieren oder können analog den im Beispielteil beschriebenen Verfahren hergestellt werden.

Die Verbindungen der Formel (XI) sind bekannt oder können hergestellt werden, indem Verbindungen der Formel 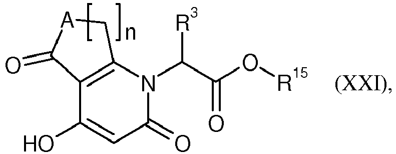 in welcher
n, A und R³ die oben angegebene Bedeutung haben, und
R¹⁵ für Methyl, Ethyl oder tert-Butyl steht,
mit Trifluormethansulfonsäureanhydrid oder *N,N*-Bis(trifluormethansulfonyl)anilin umgesetzt werden.

Die Umsetzung erfolgt im Allgemeinen in inerten Lösungsmitteln, in Gegenwart einer Base, bevorzugt in einem Temperaturbereich von -78°C bis Raumtemperatur, bevorzugt 0°C, bei Normaldruck.

Inerte Lösungsmittel sind beispielsweise Dichlormethan, Chloroform, Tetrohydrofuran oder Dimethylformamid, bevorzugt ist Dichlormethan.

Basen sind beispielsweise Pyridin, 2,6-Dimethylpyridin, Triethylamin, Diisopropylethylamin, bevorzugt ist 2,6-Dimethylpyridin oder Triethylamin.

Besonders bevorzug ist die Umsetzung mit Trifluormethansulfonsäureanhydrid in Gegenwart von 2,6-Dimethylpyridin oder *N,N*-Bis(trifluormethansulfonyl)anilin in Gegenwart von Triethylamin.

Die Verbindungen der Formel (XXI) sind bekannt, lassen sich nach bekannten Verfahren aus den entsprechenden Ausgangsverbindungen synthetisieren oder können analog den im Beispielteil beschriebenen Verfahren hergestellt werden.

Die Herstellung der Ausgangsverbindungen und der Verbindungen der Formel (I) kann durch die folgenden Syntheseschemata verdeutlicht werden.

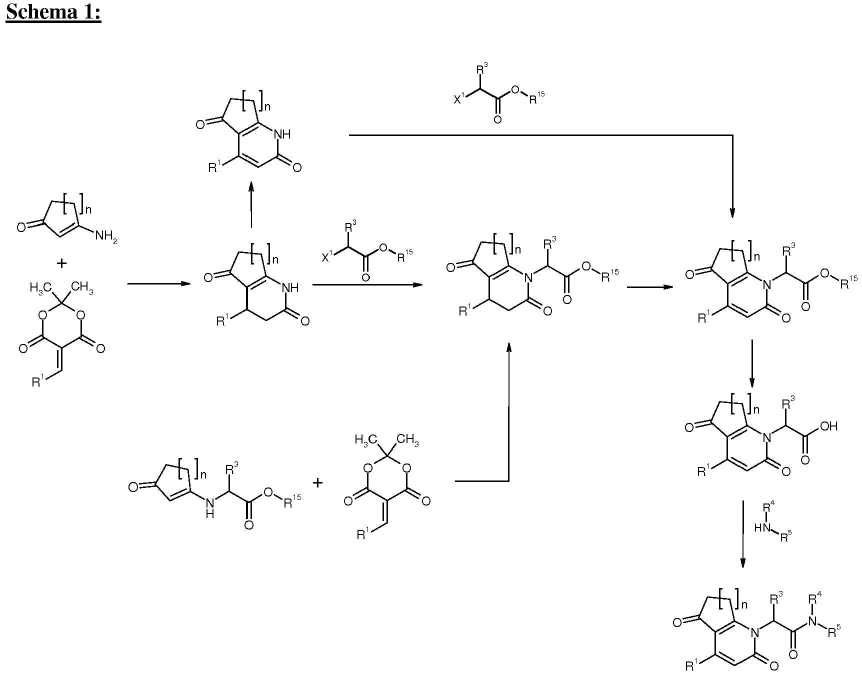

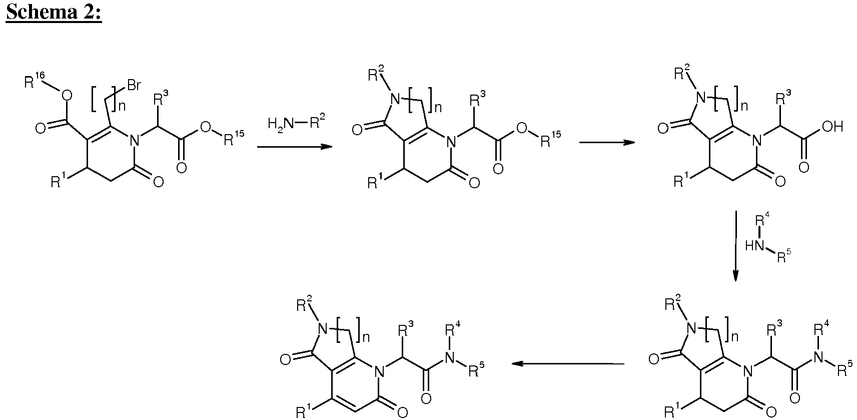

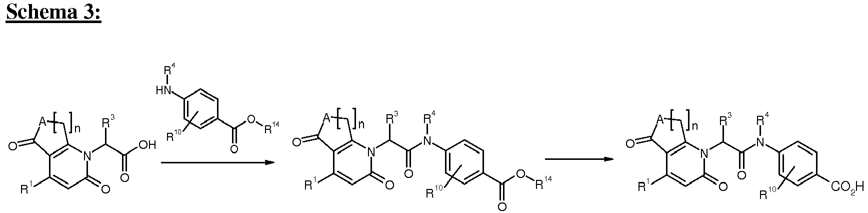

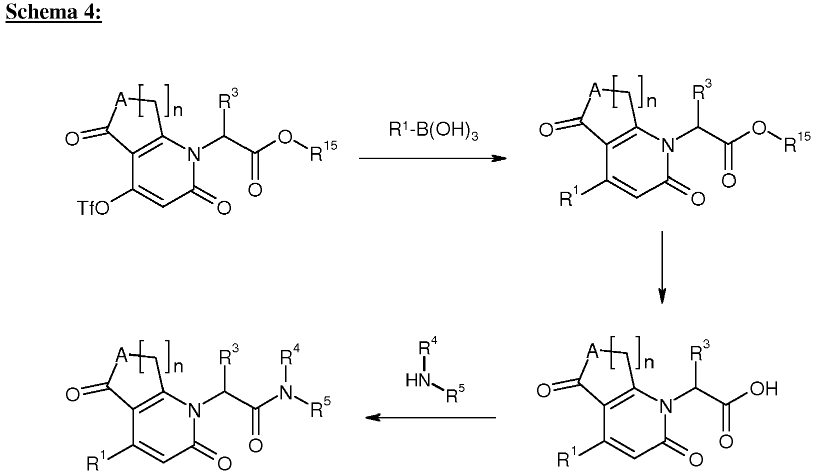

Die erfindungsgemäßen Verbindungen zeigen ein nicht vorhersehbares, wertvolles pharmakologisches Wirkspektrum und ein gutes pharmakokinetisches Verhalten. Es handelt sich dabei um Verbindungen, die die proteolytische Aktivität der Serinprotease Faktor XIa (FXIa) und/oder der Serinprotease Plasmakallikrein (PK) beeinflussen. Die erfindungsgemäßen Verbindungen hemmen die von FXIa und/oder PK katalysierte enzymatische Spaltung von Substraten, die wesentliche Rollen in der Aktivierung der Blutgerinnung, in der Aggregation von Blutplättchen via Reduktion des für die PAR-1-Aktivierung der Plättchen notwendigen Thrombins, und in inflammatorischen Prozessen einnehmen, die insbesondere eine Steigerung der Gefäßpermeabilität miteinschließen. Sie eigenen sich daher zur Verwendung als Arzneimittel zur Behandlung und/oder Prophylaxe von Krankheiten bei Menschen und Tieren. Beschrieben wird der Einsatz der erfindungsgemäßen Verbindungen zur Behandlung und/oder Prophylaxe von Erkrankungen, insbesondere von Herz-Kreislauf-Erkrankungen, vorzugsweise von thrombotischen beziehungsweise thromboembolischen Erkrankungen und/oder thrombotischen beziehungsweise thromboembolischen Komplikationen, und/oder ophthalmologischen Erkrankungen, insbesondere von diabetischer Retinopathie beziehungsweise des Makulaödems, und/oder entzündlichen Erkrankungen, insbesondere diejenigen, die mit übermäßiger Plasmakallikrein-Aktivität in Zusammenhang stehen, wie z.B. das hereditäre Angioödem (HAE) oder chronisch-entzündliche Erkrankungen, insbesondere des Darms, wie z. B. Morbus Crohn.

Faktor XIa (FXIa) ist ein wichtiges Enzym im Rahmen der Koagulation, das sowohl durch Thrombin als auch Faktor XIIa (FXIIa) aktiviert werden kann und somit in zwei wesentlichen Prozessen der Koagulation involviert ist: Es ist ein zentraler Bestandteil des Übergangs von der Initiation zur Amplifikation und Propagation der Koagulation: Thrombin aktiviert in positiven Rückkopplungsschleifen neben Faktor V und Faktor VIII auch Faktor XI zu Faktor XIa, der Faktor IX zu Faktor IXa umsetzt und über den so generierten Faktor IXa/ Faktor VIIIa-Komplex die Faktor X-Aktivierung und damit wiederum die Thrombinbildung stark stimuliert, was zu starkem Thrombuswachstum führt und den Thrombus stabilisiert.

Darüber hinaus ist Faktor XIa wichtiger Bestandteil der intrinsischen Initiation der Gerinnung: Neben der Stimulation über Gewebefaktor (tissue factor, TF) kann die Aktivierung des Gerinnungssystems auch auf insbesondere negativ geladenen Oberflächen erfolgen, zu denen neben Oberflächenstrukturen körperfremder Zellen (z.B. Bakterien) auch artifizielle Oberflächen wie Gefäßprothesen, Stents und extrakorporale Kreisläufe gehören. Auf der Oberfläche findet zunächst die Aktivierung von Faktor XII (FXII) zu Faktor XIIa (FXIIa) statt, der im Folgenden an Zelloberflächen gebundenen FXI zu FXIa aktiviert. Dieser führt wie zuvor beschrieben zur weiteren Aktivierung der Gerinnungskaskade.

Dagegen bleibt die Thrombingenerierung in der Initiationsphase über TF/Faktor VIIa und Faktor X-Aktivierung und letztlich Thrombinbildung, die physiologische Reaktion auf Gefäßverletzungen, unbeeinflußt. Dies könnte erklären, warum keine Verlängerungen der Blutungszeiten in FXIa-Knock- out-Mäusen, wie auch in Kaninchen und anderen Spezies unter FXIa-Inhibitor-Gabe gefunden wurde. Diese niedrige durch die Substanz hervorgerufene Blutungsneigung ist für die Anwendung am Menschen insbesondere bei Patienten mit erhöhtem Blutungsrisiko von großem Vorteil.

Daneben aktiviert Faktor XIIa im Rahmen der intrinsischen Aktivierung ebenfalls Plasmaprokallikrein zu Plasmakallikrein (PK), das unter anderem zu weiterer Faktor XII-Aktivierung im Rahmen einer Potentierungsschleife führt, was insgesamt eine Verstärkung der Initiation der Gerinnungskaskade an Oberflächen zur Folge hat. Eine PK-hemmende Aktivität einer erfindungsgemäßen Verbindung wird also die Gerinnung via Oberflächenaktivierung reduzieren und somit antikoagulatorisch wirken. Ein Vorteil könnte in der Kombination von Faktor XIa- und PK-inhibitorischer Aktivität liegen, die eine balancierte antithrombotische Wirkung zuläßt.

Die erfindungsgemäßen Verbindungen eignen sich daher zur Behandlung und/oder Prophylaxe von Erkrankungen oder Komplikationen, die durch Gerinnselbildung entstehen können.

Zu den "thrombotischen beziehungsweise thromboembolischen Erkrankungen" im Sinne der vorliegenden Erfindung zählen Erkrankungen, die sowohl im arteriellen als auch im venösen Gefäßbett auftreten und mit den erfindungsgemäßen Verbindungen behandelt werden können, insbesondere Erkrankungen in den Koronararterien des Herzens, wie das akute Koronarsyndrom (ACS), Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie, Stentimplantation oder aortokoronarem Bypass, aber auch thrombotische beziehungsweise thromboembolische Erkrankungen in weiteren Gefäßen, die zu peripheren arteriellen Verschlusskrankheiten, Lungenembolien, venösen Thromboembolien, venösen Thrombosen, insbesondere in tiefen Beinvenen und Nierenvenen, transitorische ischämische Attacken sowie thrombotischer Hirnschlag und thromboembolischer Hirnschlag führen.

Die Stimulation des Gerinnungssystems kann durch verschiedene Ursachen beziehungsweise Begleiterkrankungen erfolgen. Unter anderem kann im Rahmen von chirurgischen Eingriffen, Immobilität, Bettlägerigkeit, Infektionen, Inflammation oder einer Krebserkrankung beziehungsweise Krebstherapie das Gerinnungssystem stark angeregt sein und es zu thrombotischen Komplikationen, insbesondere venösen Thrombosen, kommen. Die erfindungsgemäßen Verbindungen eignen sich daher zur Thromboseprophylaxe im Rahmen von chirurgischen Eingriffen bei Patienten, die eine Krebserkrankung haben. Die erfindungsgemäßen Verbindungen eignen sich daher auch zur Thromboseprophylaxe in Patienten mit aktiviertem Gerinnungssystem, beispielsweise unter den beschriebenen Stimulationssituationen.

Die erfindungsgemäßen Verbindungen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und bei Patienten, die sich einer Kardioversion unterziehen, ferner bei Patienten mit Herzklappen-Erkrankungen oder mit künstlichen Herzklappen.

Darüber hinaus sind die erfindungsgemäßen Verbindungen zur Behandlung und Prävention einer disseminierten intravasalen Gerinnung (DIC) geeignet, die unter anderem im Rahmen einer Sepsis, aber auch infolge von Operationen, Tumorerkrankungen, Verbrennungen oder anderen Verletzungen auftreten und durch Mikrothrombosierungen zu schweren Organschäden führen kann.

Thromboembolische Komplikationen treten ferner auf bei mikroangiopathischen hämolytischen Anämien und durch Kontakt des Blutes mit körperfremden Oberflächen im Rahmen von extrakorporalen Blutkreisläufen, wie zum Beispiel Hämodialyse, ECMO ("extracorporal membrane oxygenation"), LVAD ("left ventricular assist device") und ähnlichen Verfahren, AV-Fisteln, Gefäß- sowie Herzklappenprothesen.

Außerdem kommen die erfindungsgemäßen Verbindungen zur Behandlung und/oder Prophylaxe von Erkrankungen in Betracht, bei denen Mikrogerinnselbildungen beziehungweise Fibrinablagerungen in Gehirngefäßen auftreten, die zu Demenzerkrankungen, wie beispielsweise vaskulärer Demenz oder Morbus Alzheimer führen können. Hierbei kann das Gerinnsel sowohl über Okklusionen als auch über die Bindung weiterer krankheitsrelevanter Faktoren zur Erkrankung beitragen.

Außerdem kommen die erfindungsgemäßen Verbindungen insbesondere zur Behandlung und/oder Prophylaxe von Erkrankungen in Betracht, bei denen neben der prokoagulanten auch die proinflammatorische Komponente eine wesentliche Rolle spielt. Insbesondere die gegenseitige Verstärkung von Koagulation und Inflammation kann durch die erfindungsgemäßen Verbindungen unterbunden und daher die Wahrscheinlichkeit für eine thrombotische Komplikation entscheidend gesenkt werden. Dabei können sowohl die Faktor XIa-inhibitorische Komponente (über Hemmung der Thrombinproduktion) als auch die PK-inhibitorische Komponente zur antikoagulanten und antiinflammatorischen Wirkung (z.B. via Bradikinin) beitragen. Daher kommen unter anderem die Behandlung und/oder Prophylaxe im Rahmen von atherosklerotischen Gefäßerkrankungen,

Entzündungen im Rahmen von rheumatischen Erkrankungen des Bewegungsapparates, entzündlichen Erkrankungen der Lunge, wie beispielsweise Lungenfibrosen, entzündliche Erkrankungen der Niere, wie beispielsweise Glomerulonephritiden, entzündliche Erkrankungen des Darms, wie beispielsweise Morbus Crohn oder Colitis ulcerosa, oder Erkrankungen, die im Rahmen einer diabetischen Grunderkrankung vorliegen können, wie beispielsweise diabetische Retinopathie beziehungsweise Nephropathie in Betracht.

Bei der Progression von chronisch-entzündlichen Darmerkrankungen (CED) haben unter anderem mittels Plasmakallikrein generierten Kinine eine tragende Rolle. Deren pro-inflammatorische Wirkung über Aktivierung von Bradykinin-Rezeptoren induziert und potenziert den Krankheitverlauf. Studien an Morbus Crohn-Patienten zeigen eine Korrelation zwischen der Kallikrein-Konzentration im Darmepithel und dem Grad der Darmentzündung. Eine Aktivierung des Kallikrein-Kinin-Systems wurde ebenfalls in tierexperimentellen Studien beobachtet. Eine Hemmung der Bradykinin-Synthese durch Kallikrein-Inhibitoren könnte demnach auch zur Prophylaxe und/oder Therapie von chronisch-entzündlichen Darmerkrankungen eingesetzt werden.

Außerdem können die erfindungsgemäßen Verbindungen zur Inhibition des Tumorwachstums und der Metastasenbildung, sowie zur Prophylaxe und/oder Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Außerdem kommen die erfindungsgemäßen Verbindungen auch für die Prophylaxe und/oder Behandlung von pulmonaler Hypertonie in Betracht.

Der Begriff "pulmonale Hypertonie" umfasst im Rahmen der vorliegenden Erfindung die pulmonale arterielle Hypertonie, die pulmonale Hypertonie bei Erkrankungen des linken Herzens, die pulmonale Hypertonie bei Lungenerkrankung und/oder Hypoxie und die Pulmonale Hypertonie aufgrund chronischer Thrombembolien (CTEPH).

Die "pulmonale arterielle Hypertonie" beinhaltet die Idiopathische Pulmonale Arterielle Hypertonie (IPAH, früher auch als primäre pulmonale Hypertonie bezeichnet), die Familiär bedingte Pulmonale Arterielle Hypertonie (FPAH) und die Assoziierte Pulmonal-Arterielle Hypertonie (APAH), die assoziiert ist mit Kollagenosen, kongenitalen systemisch-pulmonalen Shuntvitien, portaler Hypertension, HIV-Infektionen, der Einnahme bestimmter Drogen und Medikamente, mit anderen Erkrankungen (Schilddrüsenerkrankungen, Glykogenspeicherkrankheiten, Morbus Gaucher, hereditäre Teleangiektasie, Hämoglobinopathien, myeloproliferative Erkrankungen, Splenektomie), mit Erkrankungen mit einer signifikanten venösen/kapillären Beteiligung wie der pulmonal-venookklusiven Erkrankung und der pulmonal-kapillären Hämangiomatose, sowie die persistierende pulmonale Hypertonie der Neugeborenen.

Die pulmonale Hypertonie bei Erkrankungen des linken Herzens beinhaltet die Erkrankung des linken Vorhofes oder Ventrikels und Mitral- oder Aortenklappenfehler.

Die pulmonale Hypertonie bei Lungenerkrankung und/oder Hypoxie beinhaltet chronisch obstruktive Lungenerkrankungen, interstitielle Lungenerkrankung, Schlafapnoe-Syndrom, alveolärer Hypoventilation, chronische Höhenkrankheit und anlagebedingte Fehlbildungen.

Die Pulmonale Hypertonie aufgrund chronischer Thrombembolien (CTEPH) beinhaltet den thrombembolischen Verschluss proximaler Lungenarterien, den thrombembolischen Verschluss distaler Lungenarterien und nicht-thrombotische Lungenembolien (Tumor, Parasiten, Fremdkörper).

Weiterer Gegenstand der vorliegenden Erfindung ist die Verwendung der erfindungsgemäßen Verbindungen zur Herstellung von Arzneimitteln zur Behandlung und/oder Prophylaxe von pulmonaler Hypertonie bei Sarkoidose, Histiozytose X und Lymphangiomatosis.

Außerdem kommen die erfindungsgemäßen Substanzen auch zur Behandlung von pulmonalen und hepatischen Fibrosen in Betracht.

Außerdem kommen die erfindungsgemäßen Verbindungen auch für die Behandlung und/oder Prophylaxe einer Disseminierten intravasalen Koagulation im Rahmen einer Infektionserkrankung und/oder von Systemic Inflammatory Syndrome (SIRS), septischer Organdysfunktion, septischem Organversagen und Multiorganversagen, Acute Respiratory Distress Syndrome (ARDS), Acute Lung Injury (ALI), Septischer Schock und/oder des septischen Organversagens in Betracht.

Im Verlauf einer Infektion kann es zur generalisierten Aktivierung des Gerinnungssystems kommen ("Disseminated Intravascular coagulation", oder "Verbrauchskoagulopathie", nachfolgend als "DIC" bezeichnet) mit Mikrothrombosierung in verschiedenen Organen und sekundärer Blutungskomplikationen. Außerdem kann es es zur endothelialen Schädigung mit Erhöhung der Gefäßpermeabilität und Austritt von Flüssigkeit und Proteinen in den Extravasalraum kommen. Im weiteren Verlauf kann ein Versagen eines Organs (z.B. Nierenversagen, Leberversagen, Atemversagen, zentralnervöse Defizite und Herz-/Kreislaufversagen) oder zum Multiorganversagen kommen.

Bei der DIC kommt es an der Oberfäche von geschädigten Endothelzellen, Fremdkörperoberflächen oder veretztem extravaskulärem Gewebe zur massiven Aktivierung des Gerinnungssystems. Als Folge kommt es zur Gerinnung in kleinen Gefäßen verschiedener Organe mit Hypoxie und anschließender Organdysfunktion. Sekundär kommt es zum Verbrauch von Gerinnungsfaktoren (z.B. Faktor X, Prothrombin und Fibrinogen) und Plättchen, wodurch die Gerinnungsfähigkeit des Blutes herabgesetzt wird und schwere Blutungen auftreten können.

Erfindungsgemäße Verbindungen, die Plasmakallikrein alleine oder in Kombination mit Faktor XIa hemmen, kommen zusätzlich zur Behandlung und/oder Prophylaxe von Erkrankungen in Betracht, in deren Verlauf Plasmakallikrein involviert ist. Zusätzlich zur antikoagulanten Aktivität stellt Plasmakallikrein eine wichtige Bradikinin-freisetzende Protease dar, die somit unter anderem zum Anstieg der endothelialen Permeabilität führt. Die Verbindungen können somit zur Behandlung und/oder Prophylaxe von Erkrankungen eingesetzt werden, die mit Ödembildungen einhergehen, wie zum Beispiel ophthalmologische Erkrankungen, insbesondere die diabetische Retinopathie oder das Makulaödem, oder das hereditäre Angioödem.

Zu den "ophthalmologischen Erkrankungen" im Sinne der vorliegenden Erfindung zählen insbesondere Erkrankungen wie diabetische Retinopathie, diabetisches Makulaödem (diabetic macular edema, DME), Makulaödem, Makulaödem assoziiert mit retinalem Venenverschluss, altersbedingte Makula-Degeneration (AMD), choroidale Neovaskularisierung (CNV), choroidale neovaskuläre Membranen (CNVM), zystoides Makulaödem (cystoid macula edema, CME), epiretinale Membranen (ERM) und Makula-Perforationen, Myopie-assoziierte choroidale Neovaskularisierung, angioide beziehungsweise vaskuläre Streifen (angioid streaks, vascular streaks), Retina-Ablösung, atrophische Veränderungen des retinalen Pigmentepithels, hypertrophische Veränderungen des retinalen Pigmentepithels, retinaler Venenverschluss, choroidaler retinaler Venenverschluss, Retinitis pigmentosa, Stargardt'sche Erkrankung, Frühgeborenen-Retinopathie, Glaukom, entzündliche Erkrankungen des Auges wie z.B. Uveitis, Skleritis oder Endophthalmitis, Katarakt, Refraktionsanomalien wie z.B. Myopie, Hyperopie oder Astigmatismus und Keratokonus, Erkrankungen des vorderen Auges wie z.B. korneale Angiogenese als Folge von z.B. Keratitis, Hornhaut-Transplantation oder Keratoplastie, korneale Angiogenese als Folge von Hypoxie (z.B. durch zu langes Tragen von Kontaktlinsen), Pterygium conjunctivae, subkorneales Ödem und intrakorneales Ödem.

Weiterhin kommen die erfindungsgemäßen Verbindungen zur Primärprophylaxe thrombotischer oder thromboembolischer Erkrankungen und/oder inflammatorischer Erkrankungen und/oder Erkrankungen mit erhöhter Gefäßpermeabilität in Patienten in Frage, in denen Genmutationen zu verstärkter Aktivität der Enzyme oder erhöhten Spiegeln der Zymogene führen und diese durch entsprechende Tests/Messungen der Enzymaktivität bzw. Zymogenkonzentrationen festgestellt werden.

Beschrieben wird die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Beschrieben wird ein Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind die erfindungsgemäßen Verbindungen zur Verwendung in einem Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind Arzneimittel, enthaltend eine erfindungsgemäße Verbindung und einen oder mehrere weitere Wirkstoffe.

Die erfindungsgemäßen Verbindungen können darüber hinaus auch zur Verhinderung von Koagulation *ex vivo* eingesetzt werden, z.B. zum Schutz von zu transplantierenden Organen vor durch Gerinnselbildung verursachten Organschäden und zum Schutz des Organ-Empfängers vor Thromboemboli aus dem transplantierten Organ, zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor XIa oder Plasmakallikrein enthalten könnten.

Weiterer Gegenstand der vorliegenden Erfindung ist ein Verfahren zur Verhinderung der Blutkoagulation *in vitro,* insbesondere bei Blutkonserven oder biologischen Proben, die Faktor XIa oder Plasmakallikrein oder beide Enzyme enthalten könnten, das dadurch gekennzeichnet ist, dass eine antikoagulatorisch wirksame Menge der erfindungsgemäßen Verbindung zugegeben wird. Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren wie beispielsweise Lovastatin (Mevacor), Simvastatin (Zocor), Pravastatin (Pravachol), Fluvastatin (Lescol) und Atorvastatin (Lipitor);
- Koronartherapeutika/Vasodilatatoren, insbesondere ACE-(Angiotensin-Converting-Enzyme)-Inhibitoren wie beispielsweise Captopril, Lisinopril, Enalapril, Ramipril, Cilazapril, Benazepril, Fosinopril, Quinapril und Perindopril, oder AII-(Angiotensin II)-Rezeptor-Antagonisten wie beispielsweise Embusartan, Losartan, Valsartan, Irbesartan, Candesartan, Eprosartan und Temisarta, oder β-Adrenozeptor-Antagonisten wie beispielsweise Carvedilol, Alprenolol, Bisoprolol, Acebutolol, Atenolol, Betaxolol, Carteolol, Metoprolol, Nadolol, Penbutolol, Pindolol, Propanolol und Timolol, oder alpha-1-Adrenozeptor-Antagonisten wie beispielsweise Prazosin, Bunazosin, Doxazosin und Terazosin, oder Diuretika wie beispielsweise Hydrochlorothiazid, Furosemid, Bumetanid, Piretanid, Torasemid, Amilorid und Dihydralazin, oder Calciumkanal-Blocker wie beispielsweise Verapamil und Diltiazem, oder Dihydropyridin-Derivate wie beispielsweise Nifedipin (Adalat) und Nitrendipin (Bayotensin), oder Nitropräparate wie beispielsweise Isosorbid-5-mononitrat, Isosorbiddinitrat und Glyceroltrinitrat, oder Substanzen, die eine Erhöhung von cyclischem Guanosinmonophosphat (cGMP) bewirken, wie beispielsweise Stimulatoren der löslichen Guanylatcyclase, wie beispielsweise Riociguat;
- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren) wie beispielsweise Gewebsplasminogen-Aktivator (t-PA, beispielsweise Actilyse^{®}), Streptokinase, Reteplase und Urokinase oder Plasminogen-modulierende Substanzen, die zu verstärkter Plasmin-Bildung führen;
- antikoagulatorisch wirksame Substanzen (Antikoagulantien) wie beispielsweise Heparin (UFH), niedermolekulare Heparine (NMH) wie beispielsweise Tinzaparin, Certoparin, Parnaparin, Nadroparin, Ardeparin, Enoxaparin, Reviparin, Dalteparin, Danaparoid, Semuloparin (AVE 5026), Adomiparin (M118) und EP-42675/ORG42675;
- direkte Thrombin Inhibitoren (DTI) wie beispielsweise Pradaxa (Dabigatran), Atecegatran (AZD-0837), DP-4088, SSR-182289A, Argatroban, Bivalirudin und Tanogitran (BIBT-986 und prodrug BIBT-1011), Hirudin;
- direkte Faktor Xa-Inhibitoren wie beispielsweise Rivaroxaban, Apixaban, Edoxaban (DU-176b), Betrixaban (PRT-54021), R-1663, Darexaban (YM-150), Otamixaban (FXV-673/RPR-130673), Letaxaban (TAK-442), Razaxaban (DPC-906), DX-9065a, LY-517717, Tanogitran (BIBT-986, prodrug: BIBT-1011), Idraparinux und Fondaparinux,
- plättchenaggregationshemmende Substanzen (Plättchenaggregationshemmer, Thrombozytenaggregationshemmer) wie beispielsweise Acetylsalicylsäure (wie beispielsweise Aspirin), P2Y12-Antagonisten wie beispielsweise Ticlopidin (Ticlid), Clopidogrel (Plavix), Prasugrel, Ticagrelor, Cangrelor, Elinogrel, PAR-1-Antagonisten wie beispielsweise Vorapaxar, PAR-4 Antagonisten, EP3-Antagonisten wie beispielsweise DG041;
- Plättchenadhäsionshemmern wie GPVI- und/oder GPIb-Antagonisten wie beispielsweise Revacept oder Caplacizumab;
- Fibrinogen-Rezeptor-Antagonisten (Glycoprotein-IIb/IIIa-Antagonisten) wie beispielsweise Abciximab, Eptifibatide, Tirofiban, Lamifiban, Lefradafiban und Fradafiban;
- rekombinantes humanes aktiviertes Protein C wie beispielsweise Xigris oder rekombinantes Thrombomodulin;
- sowie Antiarrhythmika;
- Inhibitoren der VEGF- und/oder PDGF Signalwege wie beispielsweise Aflibercept, Ranibizumab, Bevacizumab, KH-902, Pegaptanib, Ramucirumab, Squalamin oder Bevasiranib, Apatinib, Axitinib, Brivanib, Cediranib, Dovitinib, Lenvatinib, Linifanib, Motesanib, Pazopanib, Regorafenib, Sorafenib, Sunitinib, Tivozanib, Vandetanib, Vatalanib, Vargatef und E-10030;
- Hemmer der Angiopoietin-Tie Signalwege wie beispielsweise AMG386;
- Inhibitoren der Tie2 Rezeptortyrosinkinase;
- Hemmer der Integrin-Signalwege wie beispielsweise Volociximab, Cilengitid und ALG1001;
- Hemmer der PI3K-Akt-mTor Signalwege wie beispielsweise XL-147, Perifosin, MK2206, Sirolimus, Temsirolimus und Everolimus;
- Corticosteroid wie beispielsweise Anecortave, Betamethason, Dexamethason, Triamcinolon, Fluocinolon und Fluocinolonacetonid;
- Inhibitoren des ALK1-Smad1/5 Signalweges wie beispielsweise ACE041;
- Cyclooxygenaseinhibitoren wie beispielsweise Bromfenac und Nepafenac;
- Hemmer des Kallikrein-Kinin-Systems wie beispielsweise Safotibant und Ecallantid;
- Inhibitoren der Sphingosin-1-phosphat-Signalwege wie beispielsweise Sonepcizumab;
- Inhibitoren des Komplement-C5a Rezeptors wie beispielsweise Eculizumab;
- Inhibitoren des 5HT1a Rezeptors wie beispielsweise Tandospiron;
- Hemmer des Ras-Raf-Mek-Erk Signalwegs; Hemmer der MAPK Signalwege; Hemmer der FGF-Signalwege; Hemmer der Endothelzellproliferation; Apoptose-induzierende Wirkstoffe;
- Photodynamische Therapie, bestehend aus einem Wirkstoff und der Einwirkung von Licht, wobei der Wirkstoff beispielsweise Verteporfin ist.

Unter "Kombinationen" im Sinne der Erfindung werden nicht nur Darreichungsformen, die alle Komponenten enthalten (sog. Fixkombinationen) und Kombinationspackungen, die die Komponenten voneinander getrennt enthalten, verstanden, sondern auch gleichzeitig oder zeitlich versetzt applizierte Komponenten, sofern sie zur Prophylaxe und/oder Behandlung derselben Krankheit eingesetzt werden. Ebenso ist es möglich, zwei oder mehr Wirkstoffe miteinander zu kombinieren, es handelt sich dabei also jeweils um zwei- oder mehrfach-Kombinationen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat beziehungsweise Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die extraoculare (topische) Applikation eignen sich nach dem Stand der Technik funktionierende schnell und/oder modifiziert beziehungsweise kontrolliert den Wirkstoff abgebende Applikationsformen, die den Wirkstoff in kristalliner und/oder amorphisierter und/oder gelöster Form enthalten, wie z.B. Augentropfen, Sprays und Lotionen (z.B. Lösungen, Suspensionen, vesikuläre/kolloidale Systeme, Emulsionen, Aerosole), Pulver für Augentropfen, Sprays und Lotionen (z.B. gemahlener Wirkstoff, Mischungen, Lyophilisate, gefällter Wirkstoff), halbfeste Augenzubereitungen (z.B. Hydrogele, in-situ Hydrogele, Cremes und Salben), Augeninserte (feste und halbfeste Zubereitungen, z.B. Bioadhesives, Filme/Wafer, Tabletten, Kontaktlinsen).

Die intraoculare Applikation umfasst z.B. die intravitreale subretinale, subsclerale, intrachoroidale, subconjunctivale, retrobulbare und subtenone Applikation. Für die intraoculare Applikation eignen sich nach dem Stand der Technik funktionierende schnell und/oder modifiziert beziehungsweise kontrolliert den Wirkstoff abgebende Applikationsformen, die den Wirkstoff in kristalliner und/oder amorphisierter und/oder gelöster Form enthalten, wie z.B. Injektionen und Konzentrate für Injektionen (z.B. Lösungen, Suspensionen, vesikuläre/kolloidale Systeme, Emulsionen, Pulver für Injektionen (z.B. gemahlener Wirkstoff, Mischungen, Lyophilisate, gefällter Wirkstoff), Gele für Injektionen (halbfeste Zubereitungen, z.B. Hydrogele, in-situ Hydrogele) und Implantate (feste Zubereitungen, z.B. bioabbaubare und nicht-bioabbaubare Implantate, implantierbare Pumpen).

Bevorzugt ist die orale Applikation beziehungsweise bei ophthalmologischen Erkrankungen die extraokulare und die intraokulare Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 250 mg je 24 Stunden zur Erzielung wirksamer Ergebnisse zu verabreichen. Bei oraler Applikation beträgt die Menge etwa 5 bis 500 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt beziehungsweise Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### A) Beispiele

### Abkürzungen:

- Boc: *tert.* -Butyloxycarbonyl
- ca.: circa
- CDI: Carbonyldiimidazol
- d: Tag(e), Dublett (bei NMR)
- DC: Dünnschicht-Chromatographie
- DCM: Dichlormethan
- DCI: direkte chemische Ionisation (bei MS)
- dd: Doppeltes Dublett (bei NMR)
- DMAP: 4-Dimethylaminopyridin
- DMF: *N,N*-Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- h: Stunde(n)
- HATU: *O*-(7-Azabenzotriazol-1-yl)-*N,N,N',N'*-tetramethyluronium-Hexafluorphosphat
- HPLC: Hochdruck-, Hochleistungsflüssigchromatographie
- HV: Hochvakuum
- LC-MS: Flüssigchromatographie-gekoppelte Massenspektroskopie
- LDA: Lithiumdiisopropylamid
- m: Multiplett (bei NMR)
- min: Minute(n)
- MS: Massenspektroskopie
- NMR: Kernresonanzspektroskopie
- quant.: quantitativ
- RP: reverse phase (bei HPLC)
- RT: Raumtemperatur
- Rt: Retentionszeit (bei HPLC)
- s: Singulett (bei NMR)
- THF: Tetrahydrofuran
- TFA: Trifluoressigsäure
- T3P: 2,4,6-Tripropyl-1,3,5,2,4,6-trioxatriphosphinan-2,4,6-trioxid

### HPLC-, LC/MS- und GC-Methoden:

Methode 1: Instrument: Waters ACQUITY SQD UPLC System; Säule: Waters Acquity UPLC HSS T3 1.8 µ 50 mm x 1 mm; Eluent A: 11 Wasser + 0.25 ml 99%ige Ameisensäure, Eluent B: 11 Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 208-400 nm.
Methode 2: Instrument: Waters ACQUITY SQD UPLC System; Säule: Waters Acquity UPLC HSS T3 1.8 µ 50 mm x 1 mm; Eluent A: 1 1 Wasser + 0.25 ml 99%ige Ameisensäure, Eluent B: 11 Acetonitril + 0.25 ml 99%ige Ameisensäure; Gradient: 0.0 min 95% A → 6.0 min 5% A → 7.5 min 5% A; Ofen: 50°C; Fluss: 0.35 ml/min; UV-Detektion: 210-400 nm.
Methode 3: Instrument: Micromass Quattro Premier mit Waters UPLC Acquity; Säule: Thermo Hypersil GOLD 1.9 µ 50 mm x 1 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 97% A → 0.5 min 97% A → 3.2 min 5% A → 4.0 min 5% A; Ofen: 50°C; Fluss: 0.3 ml/min; UV-Detektion: 210 nm.
Methode 4: Instrument: Micromass Quattro Premier mit Waters UPLC Acquity; Säule: Thermo Hypersil GOLD 1.9 µ 50 mm x 1 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 1.5 min 10% A → 2.2 min 10% A; Ofen: 50°C; Fluss: 0.33 ml/min; UV-Detektion: 210 nm.
Methode 5: Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).
Methode 6: Instrument MS: Waters (Micromass) Quattro Micro; Instrument HPLC: Agilent 1100 Serie; Säule: YMC-Triart C18 3 µ 50 mm x 3 mm; Eluent A: 1 1 Wasser + 0.01 mol Ammoniumcarbonat, Eluent B: 1 1 Acetonitril; Gradient: 0.0 min 100% A → 2.75 min 5% A → 4.5 min 5% A; Ofen: 40°C; Fluss: 1.25 ml/min; UV-Detektion: 210 nm.
Methode 7: Instrument MS: Waters (Micromass) ZQ; Instrument HPLC: Agilent 1100 Serie; Säule: Agient ZORBAX Extend-C18 3.0 mm x 50 mm 3.5-Micron; Eluent A: 1 1 Wasser + 0.01 mol Ammoniumcarbonat, Eluent B: 1 1 Acetonitril; Gradient: 0.0 min 98% A → 0.2min 98% A → 3.0 min 5% A → 4.5 min 5% A; Ofen: 40°C; Fluss: 1.75 ml/min; UV-Detektion: 210 nm.

Mikrowelle: Als Mikrowellenreaktor wurde ein "single mode" Gerät vom Typ Emrys^{™} Optimizer verwendet.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische beziehungsweise saure Funktionalität enthalten. Ein solches Salz kann durch verschiedene dem Fachmann bekannte Methoden in die entsprechende freie Base beziehungsweise Säure überführt werden.

Wenn bei den im Folgenden beschriebenen Synthese-Intermediaten und Ausführungsbeispielen der Erfindung eine Verbindung in der Form eines Salzes der korrespondierenden Base beziehungseise Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃COOH", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

### Ausgangsverbindungen

### Allgemeine Methode 1A: Amid-Kupplung mit HATU/Diisopropylethylamin

Eine Lösung der entsprechenden Carbonsäure (1.0 eq.) in Dimethylformamid (7-15 ml/mmol) wurde unter Argon bei RT mit dem entsprechenden Amin (1.1 eq.), mit *N,N*-Diisopropylethylamin (2.2 eq.) und einer Lösung von HATU (1.2 eq.) in etwas DMF versetzt. Das Reaktionsgemisch wurde bei RT gerührt. Nach Zugabe von Wasser/Essigsäureethylester und Phasentrennung wurde die organische Phase mit Wasser und mit gesättigter, wässriger Natriumchlorid-Lösung gewaschen, getrocknet (Natriumsulfat), filtriert und im Vakuum eingeengt. Das Rohprodukt wurde anschließend entweder mittels Flash-Chromatographie (Kieselgel-60, Eluent: Cyclohexan-Essigsäureethylester-Gemische oder Dichlormethan-Methanol-Gemische) oder präparativer HPLC (Reprosil C18, Wasser-Acetonitril-Gradient oder Wasser-Methanol-Gradient) aufgereinigt.

### Allgemeine Methode 2A: Verseifung eines tert.-Butylesters mittels TFA

Eine Lösung von 1.0 eq. des entsprechenden *tert*.-Butylesterderivates in Dichlormethan (ca. 7 ml/mmol) wurde bei RT mit 20 eq. TFA versetzt und bei RT für 1 bis 8 h gerührt. Anschließend wurde das Reaktionsgemisch im Vakuum eingeengt, der Rückstand dreimal mit Dichlormethan coevaporiert und im Vakuum getrocknet. Das Rohprodukt wurde gegebenenfalls anschließend entweder mittels Flash-Chromatographie (Kieselgel-60, Eluent: Cyclohexan-Essigsäureethylester-Gemische oder Dichlormethan-Methanol-Gemische) oder präparativer HPLC (Reprosil C18, Wasser-Acetonitril-Gradient oder Wasser-Methanol-Gradient) aufgereinigt.

### Allgemeine Methode 3B: Verseifung mittels Lithiumhydroxid

Eine Lösung von 1.0 eq. des entsprechenden Methyl- oder Ethylesters in Tetrahydrofuran/Wasser (3:1, ca. 10 ml/mmol) wurde bei RT mit 3.0 eq. Lithiumhydroxid versetzt. Das Reaktionsgemisch wurde bei RT bis 60°C gerührt und anschließend mit wässriger, 1N Salzsäure-Lösung auf pH 1 gestellt. Nach Zugabe von Wasser/Essigsäureethylester und Phasentrennung wurde die wässrige Phase dreimal mit Essigsäureethylester extrahiert. Die vereinigten, organischen Phasen wurden getrocknet (Natriumsulfat), filtriert und im Vakuum eingeengt. Das Rohprodukt wurde anschließend entweder mittels Flash-Chromatographie (Kieselgel-60, Eluent: Cyclohexan-Essigsäureethylester-Gemische oder Dichlormethan-Methanol-Gemische) oder präparativer HPLC (Reprosil C18, Wasser-Acetonitril-Gradient oder Wasser-Methanol-Gradient) aufgereinigt.

### Beispiel 1.1A

### 3-(4-Aminophenyl)-1,2,4-oxadiazol-5(4H)-on

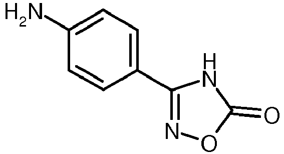

Eine Lösung von 1.5 g (7.2 mmol) 3-(4-Nitrophenyl)-1,2,4-oxadiazol-5(4*H*)-on in 75 ml Ethanol wurde mit 6.5 g (29 mmol, 4 eq.) Zinn(II)chlorid-dihydrat versetzt und 1 h bei 70°C gerührt. Nach Abkühlen auf RT wurde das Reaktionsgemisch auf Eiswasser gegossen und vorsichtig mit Natriumhydrogencarbonat bis pH 8 versetzt. Das Gemisch wurde über eine Filterschicht filtriert und der Rückstand mit Essigsäureethylester nachgewaschen. Die vereinigten Filtrate wurden im Vakuum eingeengt. Der Rückstand wurde mit Dichlormethan und Methanol verrührt, 10 min im Ultraschallbad behandelt und anschließend filtriert. Das Filtrat wurde im Vakuum eingeengt und getrocknet. Ausbeute: 1.4 g (quant.)
LC/MS [Methode 1]: Rₜ = 0.44 min; MS (ESIpos): m/z = 178 (M+H)⁺
¹H-NMR (400 MHz, DMSO-d₆): δ [ppm] = 7.42 (d, 2H), 6.51 (d, 2H), 5.23 (s, 2H), 4.13 (br. s, 1H).

### Beispiel 1.2A

### 4-Nitrobenzolcarboximidohydrazid

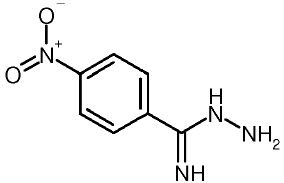

Eine Lösung von 2.0 g (9.9 mmol) 4-Nitrobenzolcarboximidamid-Monohydrochlorid in 20 ml Methanol wurde bei 0°C mit 5.2 ml (29.8 mmol, 3 eq.) *N,N*-Diisopropylethylamin und 0.62 g (80%ig, 9.9 mmol, 1.0 eq.) Hydrazin-Monohydrat versetzt und 64 h bei RT gerührt. Anschließend wurde das Reaktionsgemisch auf 10%ige wässriger Natriumchlorid-Lösung gegeben und nach Zugabe von Essigsäureethylester und Phasentrennung zweimal mit Essigsäureethylester extrahiert. Die vereinigten, organischen Phasen wurden über Natriumsulfat getrocknet und im Vakuum eingeengt. Ausbeute: 1.7 g (93% d. Th.)
LC/MS [Methode 6]: Rₜ = 1.77 min; MS (ESIpos): m/z = 181 (M+H)⁺.

### Beispiel 1.2B

### 5-(4-Nitrophenyl)-3-(trifluormethyl)-1H-1,2,4-triazol

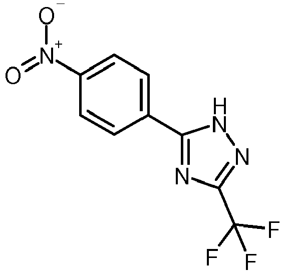

Eine Lösung von 1.7 g (9.3 mmol) 4-Nitrobenzolcarboximidohydrazid in 50 ml Dichlormethan wurde bei 0°C mit 1.95 g (9.3 mmol, 1 eq.) Trifluoressigsäureanhydrid versetzt und anschließend bei RT gerührt, wobei nach 20 min 50 ml Acetonitril für eine bessere Löslichkeit des Reaktionsgemisches zugegeben wurden. Das Reaktionsgemisch wurde 3 h bei 50°C gerührt und anschließend im Vakuum eingeengt. Der Rückstand wurde dreimal mit Dichlormethan coevaporiert und im Vakuum getrocknet. Ausbeute: 2.7 g (quant.)
LC/MS [Methode 1]: Rₜ = 0.94 min; MS (ESIpos): m/z = 259 (M+H)⁺.

### Beispiel 1.2C

### 4-[3-(Trifluormethyl)-1H-1,2,4-triazol-5-yl]anilin

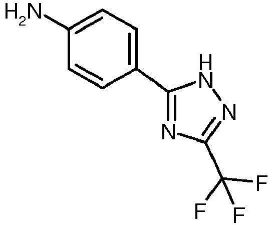

Eine Lösung von 2.7 g (9.9 mmol) 5-(4-Nitrophenyl)-3-(trifluormethyl)-1*H*-1,2,4-triazol in 110 ml Ethanol wurde mit 8.9 g (39.7 mmol, 4 eq.) Zinn(II)chlorid-dihydrat versetzt und 1 h bei 70°C gerührt. Nach Abkühlen auf RT wurde das Reaktionsgemisch auf Eiswasser gegossen und vorsichtig mit Natriumhydrogencarbonat bis pH 8 versetzt. Das Gemisch wurde über eine Filterschicht filtriert und der Rückstand mit Essigsäureethylester nachgewaschen. Nach Phasentrennung wurde die wässrige Phase zweimal mit Essigsäureethylester gewaschen. Die vereinigten, organischen Phasen wurden mit wässriger Natriumchlorid-Lösung gewaschen, getrocknet (Magnesiumsulfat), filtriert und im Vakuum eingeengt. Ausbeute: 1.9 g (79% d. Th.)
LC/MS [Methode 6]: Rₜ = 1.66 min; MS (ESIpos): m/z = 229 (M+H)⁺.

### Beispiel 1.3A

### 5-(4-Nitrophenyl)-3-oxo-2,3-dihydro-1H-pyrazol-1-carbonsäure-tert.-butylester

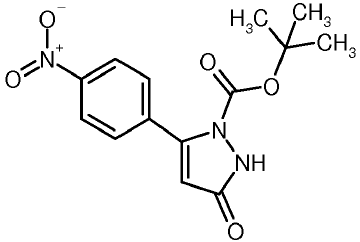

Eine Lösung von 2.5 g (12.2 mmol) 5-(4-Nitrophenyl)-1,2-dihydro-3*H*-pyrazol-3-on in 50 ml Dichlormethan wurde bei RT mit 2.7 g (12.2 mmol, 1.0 eq.) Di-*tert*.-butyldicarbonat und 1.7 ml (12.2 mmol, 1.0 eq.) Triethylamin versetzt und 4 h bei RT gerührt. Das Reaktionsgemisch wurde mit Dichlormethan und Wasser verdünnt. Nach Phasentrennung wurde die organische Phase getrocknet (Natriumsulfat), filtriert und im Vakuum eingeengt. Das Rohprodukt wurde mittels Flash-Chromatographie (Kieselgel-60, Eluent: Dichlormethan-Methanol-Gemische) aufgereinigt. Ausbeute: 2.2 g (58% d. Th.).
LC/MS [Methode 1]: Rₜ = 1.07 min; MS (ESIpos): m/z = 306 (M+H)⁺.

### Beispiel 1.3B

### 5-(4-Aminophenyl)-3-oxo-2,3-dihydro-1H-pyrazol-1-carbonsäure-tert.-butylester

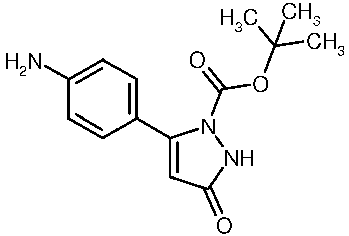

Eine Lösung von 2.2 g (7.1 mmol) 5-(4-Nitrophenyl)-3-oxo-2,3-dihydro-1*H*-pyrazol-1-carbonsäure-*tert*.-butylester in 100 ml Ethanol wurde in Gegenwart von 253 mg Palladium (10%ig auf Aktivkohle) bei RT unter Normaldruck hydriert. Anschließend wurde das Reaktionsgemisch über Celite filtriert und das Filtrat im Vakuum eingeengt und getrocknet. Ausbeute: 1.99 g (92% d. Th., 90% Reinheit)
LC/MS [Methode 7]: Rₜ = 2.06 min; MS (ESIpos): m/z = 276 (M+H)⁺.

### Beispiel 2.1A

### 3-Aminocyclopent-2-en-1-on

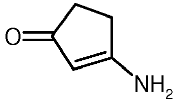

Eine Lösung von 5.5 g (43.6 mmol) 3-Ethoxy-2-cyclopenten-1-on in 55 ml Ethanol wurde mit 27.5 ml wässriger Ammoniak-Lösung (28%ig) versetzt und 16 h bei 85°C gerührt. Nach Abkühlen auf RT wurde das Reaktionsgemisch im Vakuum eingeengt und getrocknet. Ausbeute: 4.2 g (quant.)
GC/MS [Methode 5]: Rₜ = 4.78 min; MS (EI): m/z = 97.

### Beispiel 2.2A

### N-(3-Oxocyclopent-1-en-1-yl)glycinsäure-tert. -butylester

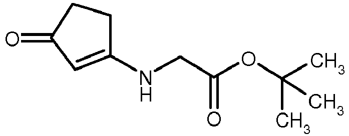

Eine Lösung von 6.7 g (68.6 mmol) Cyclopentan-1,3-dion, 9.0 g (68.6 mmol, 1.0 eq.) Glycinsäure-*tert*.-butylester und 1.3 g (6.8 mmol, 0.1 eq.) 4-Toluolsulfonsäure-Monohydrat in 350 ml Toluol wurde 3 h am Wasserabscheider unter Rückfluß gerührt. Nach Abkühlen auf RT wurde das Toluol im Vakuum entfernt. Nach Zugabe von Wasser/Dichlormethan und Phasentrennung wurde die organische Phase mit Wasser und mit gesättigter, wässriger Natriumchlorid-Lösung gewaschen, getrocknet (Natriumsulfat), filtriert und im Vakuum eingeengt. Der Rückstand wurde mit Cyclohexan verrührt, filtriert und im Vakuum getrocknet. Ausbeute: 11.9 g (82% d. Th.)
LC/MS [Methode 1]: Rₜ = 0.58 min; MS (ESIpos): m/z = 212 (M+H)⁺.

### Beispiel 2.3A

### N-(3-Oxocyclohex-1-en-1-yl)glycinsäure-tert.-butylester

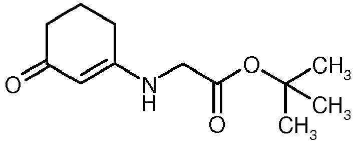

Eine Lösung von 3.0 g (27.4 mmol) Cyclohexan-1,3-dion, 3.6 g (27.4 mmol, 1.0 eq.) Glycinsäure-*tert*.-butylester und 522 mg (2.74 mmol, 0.1 eq.) 4-Toluolsulfonsäure-Monohydrat in 150 ml Toluol wurde 3 h am Wasserabscheider unter Rückfluß gerührt. Nach Abkühlen auf RT wurde das Toluol im Vakuum entfernt. Nach Zugabe von Wasser/Dichlormethan und Phasentrennung wurde die organische Phase mit Wasser und mit gesättigter, wässriger Natriumchlorid-Lösung gewaschen, getrocknet (Natriumsulfat), filtriert und im Vakuum eingeengt. Der Rückstand wurde mit Cyclohexan verrührt, filtriert und im Vakuum getrocknet. Ausbeute: 5.1 g (74% d. Th., 90% Reinheit)
LC/MS [Methode 1]: Rₜ = 0.61 min; MS (ESIpos): m/z = 226 (M+H)⁺
¹H-NMR (400 MHz, DMSO-d₆): δ [ppm] = 7.19 (br. s, 1H), 4.66 (br. s, 1H), 3.73 (d, 2H), 2.36-2.31 (m, 2H), 2.10-2.05 (m, 2H), 1.84-1.77 (m, 2H), 1.42 (s, 9H).

### Beispiel 3.1A

### 4-(3-Chlorphenyl)-2-methyl-6-oxo-1,4,5,6-tetrahydropyridin-3-carbonsäuremethylester (Racemat)

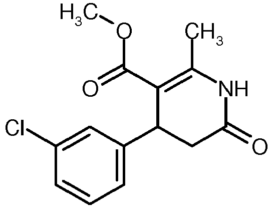

Eine Lösung von 7.5 g (53.4 mmol) 3-Chlorbenzaldehyd, 6.2 g (53.4 mmol, 1.0 eq.) Acetessigsäuremethylester, 7.7 g (53.4 mmol, 1.0 eq.) 2,2-Dimethyl-1,3-dioxan-4,6-dion und 4.3 g (55.8 mmol, 1.05 eq.) Ammoniumacetat in 53 ml Eisessig wurde 5 h unter Rückfluß gerührt. Nach Abkühlen des Reaktionsgemisches wurde der anfallende Niederschlag filtriert, mit Diethylether gewaschen und im Vakuum getrocknet. Ausbeute: 5.7 g (38% d. Th.)
LC/MS [Methode 1]: Rₜ = 0.91 min; MS (ESIpos): m/z = 280 (M+H)⁺.

### Beispiel 3.1B

### 1-(2-tert.-Butoxy-2-oxoethyl)-4-(3-chlorphenyl)-2-methyl-6-oxo-1,4,5,6-tetrahydropyridin-3-carbonsäuremethylester (Racemat)

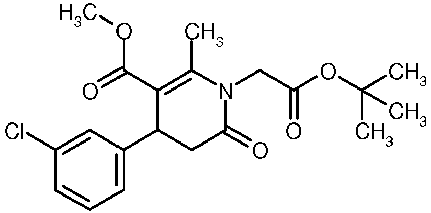

Eine Lösung von 7.2 g (25.8 mmol) 4-(3-Chlorphenyl)-2-methyl-6-oxo-1,4,5,6-tetrahydropyridin-3-carbonsäuremethylester (Racemat) in 253 ml Dimethylformamid wurde unter Argon bei RT mit 6.0 g (31.0 mmol, 1.2 eq.) Bromessigsäure-*tert*.-butylester und 7.1 g (51.6 mmol, 2.0 eq.) Kaliumcarbonat versetzt. Das Reaktionsgemisch wurde über Nacht bei 120°C gerührt, nochmals mit 2.5 g (12.9 mmol, 0.5 eq.) Bromessigsäure-*tert*.-butylester versetzt, weitere 2 h bei 120°C gerührt, nochmals mit 2.5 g (12.9 mmol, 0.5 eq.) Bromessigsäure-*tert*.-butylester versetzt und eine weitere Nacht bei 120°C gerührt. Nach Abkühlen auf RT wurde das Dimethylformamid im Vakuum entfernt. Nach Zugabe von Wasser/Essigsäureethylester und Phasentrennung wurde die organische Phase mit Wasser und mit gesättigter, wässriger Natriumchlorid-Lösung gewaschen, getrocknet (Natriumsulfat), filtriert und im Vakuum eingeengt. Die Aufreinigung des Rohproduktes erfolgte anschließend mittels Flash-Chromatographie (Kieselgel-60, Eluent: Dichlormethan-Methanol-Gemische). Ausbeute: 3.9 g (34% d. Th., 88% Reinheit)
LC/MS [Methode 1]: Rₜ = 1.20 min; MS (ESIpos): m/z = 394 (M+H)⁺.

### Beispiel 3.1C

### [6-(Brommethyl)-4-(3-chlorphenyl)-5-(methoxycarbonyl)-2-oxo-3,4-dihydropyridin-l(2H)-yl]essigsäure (Racemat)

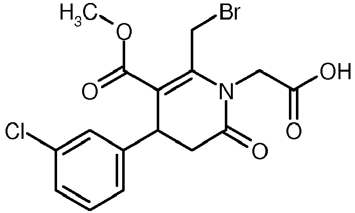

Eine Lösung von 3.9 g (9.9 mmol) 1-(2-*tert*.-Butoxy-2-oxoethyl)-4-(3-chlorphenyl)-2-methyl-6-oxo-1,4,5,6-tetrahydropyridin-3-carbonsäuremethylester (Racemat) in 79 ml Dichlormethan wurde unter Eiskühlung tropfenweise mit 1.6 g (9.9 mmol, 1.0 eq.) Brom versetzt und 60 min bei RT gerührt. Nach Zugabe von weiterem Dichlormethan wurde das Reaktionsgemisch mit gesättigter, wässriger Natriumthiosulfat-Lösung gewaschen und nach Phasentrennung die organische Phase getrocknet (Natriumsulfat), filtriert und im Vakuum eingeengt. Ausbeute: 3.4 g (66% d. Th., 79% Reinheit)
LC/MS [Methode 1]: Rₜ = 0.96 min; MS (ESIpos): m/z = 416 (M+H)⁺.

### Beispiel 3.1D

### [4-(3-Chlorphenyl)-2,5-dioxo-2,3,4,5,6,7-hexahydro-1H-pyrrolo[3,4-b]pyridin-1-yl]essigsäure (Racemat)

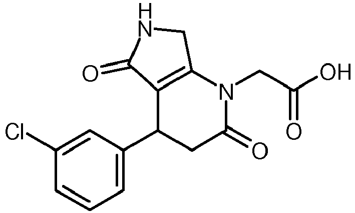

Eine Lösung von 1.4 g (1.7 mmol, 50% Reinheit) [6-(Brommethyl)-4-(3-chlorphenyl)-5-(methoxycarbonyl)-2-oxo-3,4-dihydropyridin-1(2*H*)-yl]essigsäure (Racemat) in 8 ml Acetonitril wurde mit 12 ml einer Ammoniaklösung (7 molar in Methanol) versetzt und 30 min bei RT gerührt. Das Reaktionsgemisch wurde im Vakuum eingeengt und der Rückstand mit 60 ml einer 0.5 molaren wässrigen Salzsäurelösung verrührt. Der Niederschlag wurde filtriert, mit Wasser gewaschen und im Vakuum getrocknet. Ausbeute: 680 mg (93% d. Th., 74% Reinheit)
LC/MS [Methode 1]: Rₜ = 0.58 min; MS (ESIpos): m/z = 321 (M+H)⁺.

### Beispiel 3.1E

### 2-[4-(3-Chlorphenyl)-2,5-dioxo-2,3,4,5,6,7-hexahydro-1H-pyrrolo[3,4-b]pyridin-1-yl]-N-[4-(1H-tetrazol-5-yl)phenyl]acetamid (Racemat)

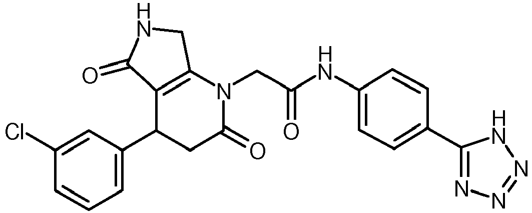

Nach der allgemeinen Methode 1A wurden 500 mg (1.1 mmol, 70% Reinheit) [4-(3-Chlorphenyl)-2,5-dioxo-2,3,4,5,6,7-hexahydro-1*H*-pyrrolo[3,4-b]pyridin-1-yl]essigsäure (Racemat) mit 211 mg (1.3 mmol, 1.2 eq.) 4-(1*H*-Tetrazol-5-yl)anilin umgesetzt. Die Aufreinigung des Rohproduktes erfolgte anschließend mittels präparativer HPLC (Reprosil C18, Wasser-Acetonitril-Gradient). Ausbeute: 24 mg (5% d. Th.)
LC/MS [Methode 1]: Rₜ = 0.75 min; MS (ESIpos): m/z = 464 (M+H)⁺
¹H-NMR (400 MHz, DMSO-d₆): δ [ppm] = 10.64 (s, 1H), 8.01 (d, 2H), 7.84-7.80 (m, 3H), 7.43 (s, 1H), 7.37-7.28 (m, 3H), 4.59 (d, 1H), 4.36 (d, 1H), 4.12 (dd, 2H), 3.98 (d, 1H), 3.23 (dd, 1H), 2.63 (d, 1H).

### Beispiel 3.2A

### 4-(2,5-Dichlorphenyl)-2-methyl-6-oxo-1,4,5,6-tetrahydropyridin-3-carbonsäuremethylester (Racemat)

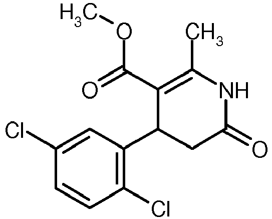

Eine Lösung von 11.5 g (36.3 mmol) 2,5-Dichlorbenzaldehyd, 4.2 g (36.3 mmol, 1.0 eq.) Acetessigsäuremethylester, 5.2 g (36.3 mmol, 1.0 eq.) 2,2-Dimethyl-1,3-dioxan-4,6-dion und 2.9 g (38.1 mmol, 1.05 eq.) Ammoniumacetat in 35 ml Eisessig wurde 5 h unter Rückfluß gerührt. Nach Abkühlen des Reaktionsgemisches wurde der anfallende Niederschlag filtriert, mit Wasser gewaschen und im Vakuum getrocknet. Ausbeute: 4.0 g (33% d. Th., 92% Reinheit)
LC/MS [Methode 1]: Rₜ = 0.95 min; MS (ESIpos): m/z = 314 (M+H)⁺.

### Beispiel 3.2B

### 1-(2-tert.-Butoxy-2-oxoethyl)-4-(2,5-dichlorphenyl)-2-methyl-6-oxo-1,4,5,6-tetrahydropyridin-3-carbonsäuremethylester (Racemat)

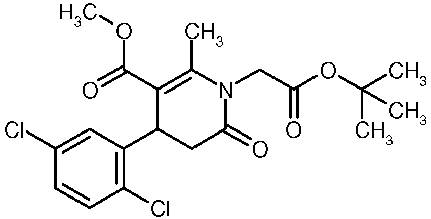

Eine Lösung von 1.6 g (4.8 mmol) 4-(2,5-Dichlorphenyl)-2-methyl-6-oxo-1,4,5,6-tetrahydropyridin-3-carbonsäuremethylester (Racemat) in 30 ml Dimethylformamid wurde bei RT mit 1.3 g (6.7 mmol, 1.4 eq.) Bromessigsäure-*tert*.-butylester und 1.3 g (9.5 mmol, 2.0 eq.) Kaliumcarbonat versetzt und 2 h bei 120°C gerührt. Nach Abkühlen auf RT wurde das Reaktionsgemisch im Vakuum eingeengt. Nach Zugabe von Wasser/Essigsäureethylester und Phasentrennung wurde die organische Phase mit Wasser und mit gesättigter, wässriger Natriumchlorid-Lösung gewaschen, getrocknet (Natriumsulfat), filtriert und im Vakuum eingeengt. Ausbeute: 2.1 g (quant.)
LC/MS [Methode 2]: Rₜ = 1.31 min; MS (ESIpos): m/z = 371 (M+H-*tert*.-Bu)⁺.

### Beispiel 3.2C

### 2-(Brommethyl)-1-(2-tert.-butoxy-2-oxoethyl)-4-(2,5-dichlorphenyl)-6-oxo-1,4,5,6-tetrahydropyridin-3-carbonsäuremethylester (Racemat)

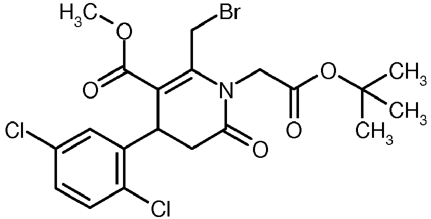

Eine Lösung von 2.1 g (4.9 mmol) 1-(2-*tert*.-Butoxy-2-oxoethyl)-4-(2,5-dichlorphenyl)-2-methyl-6-oxo-1,4,5,6-tetrahydropyridin-3-carbonsäuremethylester (Racemat) in 40 ml Dichlormethan wurde unter Eiskühlung tropfenweise mit 0.8 g (4.9 mmol, 1.0 eq.) Brom versetzt und 60 min bei RT gerührt. Nach Zugabe von weiterem Dichlormethan wurde das Reaktionsgemisch mit gesättigter, wässriger Natriumthiosulfat-Lösung gewaschen, die organische Phase getrocknet (Natriumsulfat), filtriert und im Vakuum eingeengt. Ausbeute: 2.2 g (88% d. Th., 82% Reinheit)
LC/MS [Methode 2]: Rₜ = 1.01 min; MS (ESIpos): m/z = 450 (M+H-*tert*.-Bu)⁺.

### Beispiel 3.2D

### [4-(2,5-Dichlorphenyl)-6-methyl-2,5-dioxo-2,3,4,5,6,7-hexahydro-1H-pyrrolo[3,4-b]pyridin-1-yl]essigsäure-tert.-butylester (Racemat)

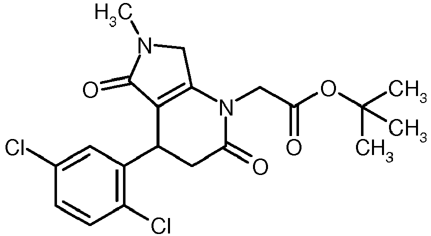

Eine Lösung von 1.8 g (2.9 mmol, 82% Reinheit) 2-(Brommethyl)-1-(2-*tert*.-butoxy-2-oxoethyl)-4-(2,5-dichlorphenyl)-6-oxo-1,4,5,6-tetrahydropyridin-3-carbonsäuremethylester (Racemat) in 70 ml Tetrahydrofuran wurde mit 2.2 ml (4.4 mmol, 1.5 eq.) einer Methylaminlösung (2 molar in Tetrahydrofuran) versetzt. Das Reaktionsgemisch wurde 60 min bei RT gerührt und im Vakuum eingeengt. Der Rückstand wurde im Eisbad mit Acetonitril verrührt, der Niederschlag filtiert und die Mutterlauge im Vakuum eingeengt. Die Aufreinigung des Rohproduktes erfolgte anschließend mittels Flash-Chromatographie (Kieselgel-60, Eluent: Dichlormethan-Methanol-Gemische). Ausbeute: 0.42 g (28% d. Th., 83% Reinheit)
LC/MS [Methode 2]: Rₜ = 1.06 min; MS (ESIpos): m/z = 425 (M+H)⁺.

### Beispiel 3.2E

### [4-(2,5-Dichlorphenyl)-6-methyl-2,5-dioxo-2,5,6,7-tetrahydro-1H-pyrrolo[3,4-b]pyridin-1-yl]essigsäure-tert.-butylester

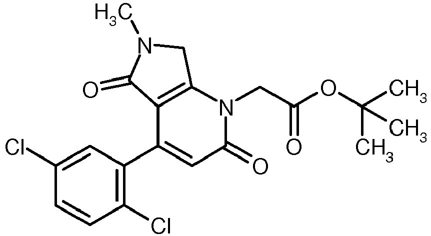

Eine Lösung von 333 mg (0.65 mmol, 83% Reinheit) [4-(2,5-Dichlorphenyl)-6-methyl-2,5-dioxo-2,3,4,5,6,7-hexahydro-1*H*-pyrrolo[3,4-b]pyridin-1-yl]essigsäure-*tert*.-butylester (Racemat) in 10 ml Dioxan wurde mit einer Lösung von 1781 mg (3.25 mmol, 5.0 eq.) Ammoniumcer(IV)nitrat in 2.5 ml Wasser versetzt und 7 h bei 50°C gerührt. Nach Abkühlen auf RT wurde das Reaktionsgemisch im Vakuum eingeengt. Nach Zugabe von Wasser/Essigsäureethylester und Phasentrennung wurde die organische Phase mit Wasser und mit gesättigter, wässriger Natriumchlorid-Lösung gewaschen, getrocknet (Natriumsulfat), filtriert und im Vakuum eingeengt. Ausbeute: 301 mg (24% d. Th., 22% Reinheit)
LC/MS [Methode 2]: Rₜ = 1.02 min; MS (ESIpos): m/z = 423 (M+H)⁺.

### Beispiel 3.2F

### [4-(2,5-Dichlorphenyl)-6-methyl-2,5-dioxo-2,5,6,7-tetrahydro-1H-pyrrolo[3,4-b]pyridin-1-yl]essigsäure

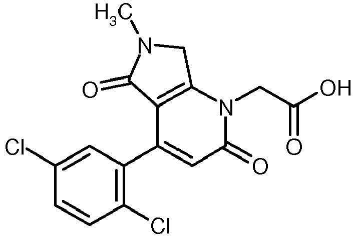

Nach der allgemeinen Methode 2A wurden 280 mg (0.66 mmol, 22% Reinheit) [4-(2,5-Dichlorphenyl)-6-methyl-2,5-dioxo-2,5,6,7-tetrahydro-1*H*-pyrrolo[3,4-b]pyridin-1-yl]essigsäure-*tert*.-butylester mit TFA verseift. Ausbeute: 328 mg (76% d. Th., 56% Reinheit)
LC/MS [Methode 1]: Rₜ = 0.65 min; MS (ESIpos): m/z = 367 (M+H)⁺.

### Beispiel 4.1A

### 5-(2,5-Dichlorbenzyliden)-2,2-dimethyl-1,3-dioxan-4,6-dion

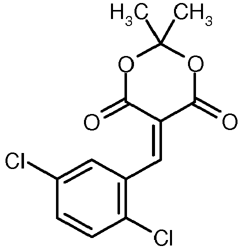

Eine Lösung von 2.75 g (19.0 mmol) 2,2-Dimethyl-1,3-dioxan-4,6-dion und 5.0 g (28.6 mmol, 1.5 eq.) 2,5-Dichlorbenzaldehyd in 250 ml Toluol wurde mit 358 µl (3.6 mmol, 0.2 eq.) Piperidin und 1.2 ml (21.0 mmol, 1.1 eq.) Essigsäure versetzt und 5 h am Wasserabscheider unter Rückfluß gerührt. Nach Abkühlen auf RT wurde das Reaktionsgemisch im Vakuum eingeengt, der Rückstand mit Cyclohexan verrührt, filtriert und im Vakuum getrocknet. Ausbeute: 4.94 g (82% d. Th.)
¹H-NMR (400 MHz, DMSO-d₆): δ [ppm] = 8.40 (s, 1H), 7.78 (d, 1H), 7.64 (d, 1H), 7.59 (dd, 1H), 1.79 (s, 6H).

### Beispiel 4.1B

### [4-(2,5-Dichlorphenyl)-2,5-dioxo-2,3,4,5,6,7-hexahydro-1H-cyclopenta[b]pyridin-1-yl]essigsäure-tert.-butylester (Racemat)

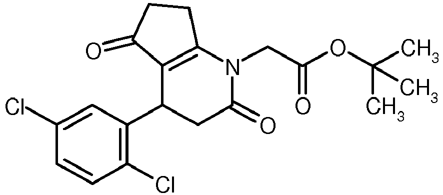

Eine Lösung von 5.0 g (23.7 mmol) 5-(2,5-Dichlorbenzyliden)-2,2-dimethyl-1,3-dioxan-4,6-dion und 7.1 g (23.7 mmol, 1.0 eq.) *N*-(3-Oxocyclopent-1-en-1-yl)glycinsäure-*tert*.-butylester in 80 ml Ethanol wurde 30 min unter Rückfluß gerührt. Nach Abkühlen auf RT wurde das Reaktionsgemisch im Vakuum eingeengt. Die Aufreinigung des Rohproduktes erfolgte mittels Flash-Chromatographie (Kieselgel-60, Eluent: Cyclohexan-Essigsäureethylester-Gemische). Ausbeute: 7.4 g (72% d. Th., 94% Reinheit)
LC/MS [Methode 1]: Rₜ = 1.12 min; MS (ESIpos): m/z = 410 (M+H)⁺.

### Beispiel 4.1C

### [4-(2,5-Dichlorphenyl)-2,5-dioxo-2,3,4,5,6,7-hexahydro-1H-cyclopenta[b]pyridin-1-yl]essigsäure (Racemat)

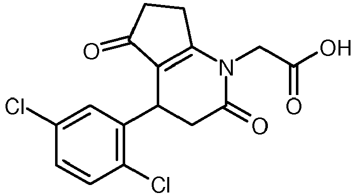

Eine Lösung von 274 mg (0.67 mmol) [4-(2,5-Dichlorphenyl)-2,5-dioxo-2,3,4,5,6,7-hexahydro-1*H*-cyclopenta[b]pyridin-1-yl]essigsäure-tert.-butylester (Racemat) in 10 ml Aceton wurde mit einer Lösung von 1464 mg (2.67 mmol, 4.0 eq.) Ammoniumcer(IV)nitrat in 2.7 ml Wasser versetzt und über Nacht bei RT gerührt. Nach Abkühlen auf RT wurde das Reaktionsgemisch anschließend im Vakuum eingeengt. Nach Zugabe von Wasser/Dichlormethan und Phasentrennung wurde die organische Phase mit Wasser und mit gesättigter, wässriger Natriumchlorid-Lösung gewaschen, getrocknet (Natriumsulfat), filtriert und im Vakuum eingeengt. Ausbeute: 211 mg (63% d. Th., 71 % Reinheit)
LC/MS [Methode 1]: Rₜ = 1.08 min; MS (ESIpos): m/z = 354 (M+H)⁺

### Beispiel 4.1D

### 2-[4-(2,5-Dichlorphenyl)-2,5-dioxo-2,3,4,5,6,7-hexahydro-1H-cyclopenta[b]pyridin-1-yl]-N-[4-(1H-tetrazol-5-yl)phenyl]acetamid (Racemat)

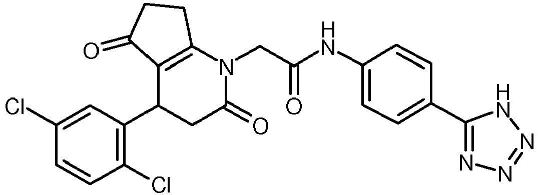

Nach der allgemeinen Methode 1A wurden 211 mg (0.42 mmol, 71% Reinheit) [4-(2,5-Dichlorphenyl)-2,5-dioxo-2,3,4,5,6,7-hexahydro-1*H*-cyclopenta[b]pyridin-1-yl]essigsäure (Racemat) mit 82 mg (0.51 mmol, 1.2 eq.) 4-(1*H*-Tetrazol-5-yl)anilin umgesetzt. Ausbeute: 138 mg (58% d. Th., 89% Reinheit)
LC/MS [Methode 1]: Rₜ = 0.87 min; MS (ESIpos): m/z = 497 (M+H)⁺.

### Beispiel 4.2A

### [4-(2,5-Dichlorphenyl)-2,5-dioxo-2,5,6,7-tetrahydro-1H-cyclopenta[b]pyridin-1-yl]essigsäure-tert.-butylester

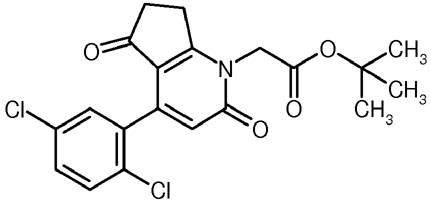

Eine Lösung von 4.7 g (10.7 mmol, 94% Reinheit) [4-(2,5-Dichlorphenyl)-2,5-dioxo-2,3,4,5,6,7-hexahydro-1*H*-cyclopenta[b]pyridin-1-yl]essigsäure-*tert*.-butylester (Racemat) in 186 ml Dioxan wurde mit einer Lösung von 29.2 g (53.3 mmol, 5.0 eq.) Ammoniumcer(IV)nitrat in 46 ml Wasser versetzt und 5 h bei 50°C gerührt. Nach Abkühlen auf RT wurde das Reaktionsgemisch im Vakuum eingeengt. Nach Zugabe von Wasser/Dichlormethan und Phasentrennung wurde die organische Phase mit Wasser und mit gesättigter, wässriger Natriumchlorid-Lösung gewaschen, getrocknet (Natriumsulfat), filtriert und im Vakuum eingeengt. Ausbeute: 5.5 g (quant.)
LC/MS [Methode 1]: Rₜ = 1.10 min; MS (ESIpos): m/z = 408 (M+H)⁺.

### Beispiel 4.2B

### [4-(2,5-Dichlorphenyl)-2,5-dioxo-2,5,6,7-tetrahydro-1H-cyclopenta[b]pyridin-1-yl]essigsäure

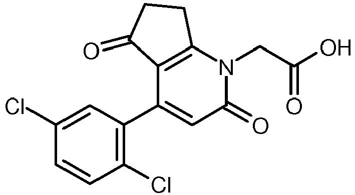

Nach der allgemeinen Methode 2A wurden 2.0 g (5.0 mmol) [4-(2,5-Dichlorphenyl)-2,5-dioxo-2,5,6,7-tetrahydro-1*H*-cyclopenta[b]pyridin-1-yl]essigsäure-*tert*.-butylester mit TFA verseift. Ausbeute: 1.9 g (95% d. Th., 89% Reinheit)
LC/MS [Methode 1]: Rₜ = 0.72 min; MS (ESIpos): m/z = 352 (M+H)⁺.

### Beispiel 4.3A

### 4-({[4-(2,5-Dichlorphenyl)-2,5-dioxo-2,5,6,7-tetrahydro-1H-cyclopenta[b]pyridin-1-yl]acetyl}-amino)benzoesäure-tert.-butylester

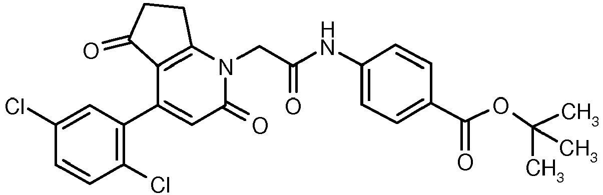

Nach der allgemeinen Methode 1A wurden 119 mg (0.30 mmol, 89% Reinheit) [4-(2,5-Dichlorphenyl)-2,5-dioxo-2,5,6,7-tetrahydro-1*H*-cyclopenta[b]pyridin-1-yl]essigsäure mit 64 mg (0.33 mmol, 1.1 eq.) 4-Aminobenzoesäure-*tert*.-butylester umgesetzt. Ausbeute: 48 mg (29% d. Th., 94% Reinheit)
LC/MS [Methode 1]: Rₜ = 1.14 min; MS (ESIpos): m/z = 527 (M+H)⁺.

### Beispiel 4.4A

### 5-[4-({[4-(2,5-Dichlorphenyl)-2,5-dioxo-2,5,6,7-tetrahydro-1H-cyclopenta[b]pyridin-1-yl]acetyl}amino)phenyl]-3-oxo-2,3-dihydro-1H-pyrazol-1-carbonsäure-tert. -butylester

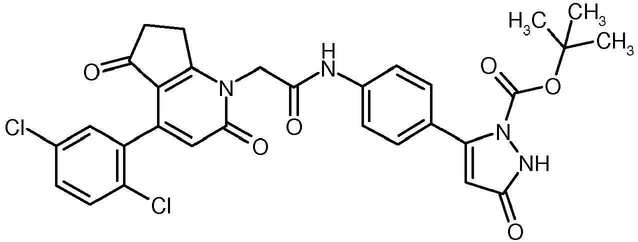

Nach der allgemeinen Methode 1A wurden 102 mg (0.25 mmol, 86% Reinheit) [4-(2,5-Dichlorphenyl)-2,5-dioxo-2,5,6,7-tetrahydro-1*H*-cyclopenta[b]pyridin-1-yl]essigsäure mit 95 mg (0.28 mmol, 1.1 eq.) 5-(4-Aminophenyl)-3-oxo-2,3-dihydro-1*H*-pyrazol-1-carbonsäure-*tert*.-butylester umgesetzt. Ausbeute: 53 mg (35% d. Th.)
LC/MS [Methode 1]: Rₜ = 1.12 min; MS (ESIpos): m/z = 609 (M+H)⁺.

### Beispiel 5.1A

### 5-(2-Brom-5-chlorbenzyliden)-2,2-dimethyl-1,3-dioxan-4,6-dion

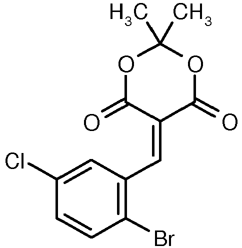

Eine Lösung von 4.6 g (31.9 mmol) 2,2-Dimethyl-1,3-dioxan-4,6-dion und 10.5 g (47.8 mmol, 1.5 eq.) 2-Brom-5-chlorbenzaldehyd in 450 ml Toluol wurde mit 0.6 ml (6.1 mmol, 0.2 eq.) Piperidin und 2 ml (35.1 mmol, 1.1 eq.) Essigsäure versetzt und 3 h am Wasserabscheider unter Rückfluß gerührt. Nach Abkühlen auf RT wurde das Reaktionsgemisch im Vakuum eingeengt, der Rückstand mit Diethylether verrührt, filtriert und im Vakuum getrocknet. Ausbeute: 9.3 g (84% d. Th.)
¹H-NMR (400 MHz, DMSO-d₆): δ [ppm] = 8.35 (s, 1H), 7.78 (d, 1H), 7.71 (d, 1H), 7.50 (dd, 1H), 1.79 (s, 6H).

### Beispiel 5.1B

### 4-(2-Brom-5-chlorphenyl)-3,4,6,7-tetrahydro-1H-cyclopenta[b]pyridin-2,5-dion (Racemat)

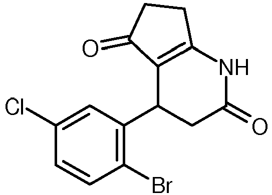

Einen Lösung von 2.6 g (26.9 mmol) 3-Aminocyclopent-2-en-1-on und 9.3 g (26.9 mmol, 1.0 eq.) 5-(2-Brom-5-chlorbenzyliden)-2,2-dimethyl-1,3-dioxan-4,6-dion in 100 ml Dioxan wurde bei 80°C gerührt. Nach 3 h wurde das Reaktionsgemisch auf RT abgekühlt, mit 3.1 g (8.1 mmol, 0.3 eq.) HATU und 0.9 ml (5.4 mmol, 0.2 eq.) *N*,*N*-Diisopropylethylamin versetzt, über Nacht bei RT gerührt und im Vakuum eingeengt. Nach Zugabe von Wasser/Essigsäureethylester und Phasentrennung wurde die organische Phase getrocknet (Natriumsulfat), filtriert und im Vakuum eingeengt. Die Aufreinigung des Rohproduktes erfolgte anschließend mittels Flash-Chromatographie (Kieselgel-60, Eluent: Dichlormethan-Methanol-Gemische). Ausbeute: 4.95 g (44% d. Th., 81% Reinheit)
LC/MS [Methode 1]: Rₜ = 0.84 min; MS (ESIpos): m/z = 340 (M+H)⁺.

### Beispiel 5.1C

### 4-(2-Brom-5-chlorphenyl)-6,7-dihydro-1H-cyclopenta[b]pyridin-2,5-dion

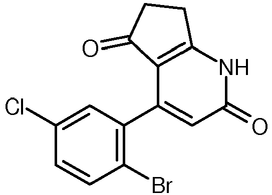

Eine Lösung von 4.95 g (11.8 mmol, 81% Reinheit) 4-(2-Brom-5-chlorphenyl)-3,4,6,7-tetrahydro-1*H*-cyclopenta[b]pyridin-2,5-dion (Racemat) in 180 ml Dioxan wurde mit einer Lösung von 32.3 g (58.9 mmol, 5.0 eq.) Ammoniumcer(IV)nitrat in 60 ml Wasser versetzt und 3 h bei 50°C gerührt. Nach Abkühlen auf RT wurde das Reaktionsgemisch im Vakuum eingeengt. Nach Zugabe von Wasser/Dichlormethan und Phasentrennung wurde die organische Phase mit Wasser und mit gesättigter, wässriger Natriumchlorid-Lösung gewaschen, getrocknet (Natriumsulfat), filtriert und im Vakuum eingeengt. Der Rückstand wurde mit Diethylether verrührt, filtriert und im Vakuum getrocknet. Ausbeute: 3.1 g (70% d. Th., 90% Reinheit)
LC/MS [Methode 3]: Rₜ = 1.78 min; MS (ESIpos): m/z = 338 (M+H)⁺
¹H-NMR (400 MHz, DMSO-d₆): δ [ppm] = 12.74 (s, 1H), 7.69 (d, 1H), 7.42 (dd, 1H), 7.36 (d, 1H), 6.10 (s, 1H), 3.03-2.86 (m, 2H), 2.56-2.46 (m, 2H).

### Beispiel 5.1D

### [4-(2-Brom-5-chlorphenyl)-2,5-dioxo-2,5,6,7-tetrahydro-1H-cyclopenta[b]pyridin-1-yl]essigsäure-tert.-butylester

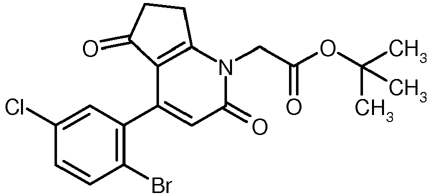

Eine Lösung von 300 mg (0.80 mmol, 90% Reinheit) 4-(2-Brom-5-chlorphenyl)-6,7-dihydro-1*H-*cyclopenta[b]pyridin-2,5-dion, 0.14 ml (0.96 mmol, 1.2 eq.) Bromessigsäeure-*tert*.-butylester und 165 mg (1.20 mmol) Kaliumcarbonat in 18 ml Dimethylformamid wurde 2 h bei 120°C gerührt. Nach Abkühlen auf RT wurde das Reaktionsgemisch im Vakuum eingeengt. Nach Zugabe von Wasser/Essigsäureethylester und Phasentrennung wurde die organische Phase mit Wasser und mit gesättigter, wässriger Natriumchlorid-Lösung gewaschen, getrocknet (Natriumsulfat), filtriert und im Vakuum eingeengt. Der Rückstand wurde mit Diethylether verrührt, filtriert und im Vakuum getrocknet. Ausbeute: 169 mg (47% d. Th.)
LC/MS [Methode 1]: Rₜ = 1.11 min; MS (ESIpos): m/z = 452 (M+H)⁺
¹H-NMR (400 MHz, DMSO-d₆): δ [ppm] = 7.70 (d, 1H), 7.43 (dd, 1H), 7.40 (d, 1H), 6.29 (s, 1H), 4.79 (s, 2H), 3.07-3.01 (m, 2H), 2.61-2.55 (m, 2H), 1.45 (s, 9H).

### Beispiel 5.1E

### [4-(2-Brom-5-chlorphenyl)-2,5-dioxo-2,5,6,7-tetrahydro-1H-cyclopenta[b]pyridin-1-yl]essigsäure

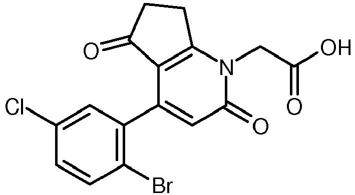

Nach der allgemeinen Methode 2A wurden 140 mg (0.31 mmol) [4-(2-Brom-5-chlorphenyl)-2,5-dioxo-2,5,6,7-tetrahydro-1*H*-cyclopenta[b]pyridin-1-yl]essigsäure-*tert*.-butylester mit TFA verseift. Ausbeute: 90 mg (69% d. Th., 94% Reinheit)
LC/MS [Methode 1]: Rₜ = 0.76 min; MS (ESIpos): m/z = 396 (M+H)⁺.

### Beispiel 6.1A

### 5-[5-Chlor-2-(trifluormethyl)benzyliden]-2,2-dimethyl-1,3-dioxan-4,6-dion

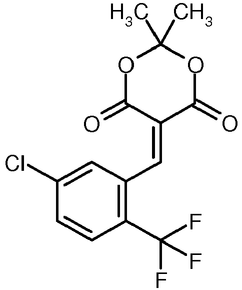

Eine Lösung von 3.0 g (20.8 mmol) 2,2-Dimethyl-1,3-dioxan-4,6-dion und 5.0 g (24.0 mmol, 1.2 eq.) 5-Chlor-2-(trifluormethyl)benzaldehyd in 295 ml Toluol wurde mit 0.4 ml (4.0 mmol, 0.2 eq.) Piperidin und 1.3 ml (23.0 mmol, 1.1 eq.) Essigsäure versetzt und 3 h am Wasserabscheider unter Rückfluß gerührt. Nach Abkühlen auf RT wurde das Reaktionsgemisch im Vakuum eingeengt, der Rückstand mit Diethylether verrührt, filtriert und im Vakuum getrocknet. Ausbeute: 5.4 g (69% d. Th., 89% Reinheit)
¹H-NMR (400 MHz, DMSO-d₆): δ [ppm] = 8.57 (s, 1H), 7.87 (d, 1H), 7.75 (d, 1H), 7.70 (s, 1H), 1.78 (s, 6H).

### Beispiel 6.1B

### 4-[5-Chlor-2-(trifluormethyl)phenyl]-3,4,6,7-tetrahydro-1H-cyclopenta[b]pyridin-2,5-dion (Racemat)

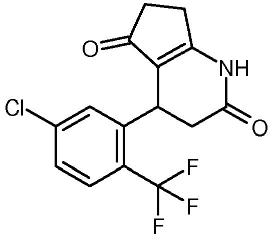

Eine Lösung von 1.4 g (14.4 mmol) 3-Aminocyclopent-2-en-1-on und 5.4 g (14.4 mmol) 5-[5-Chlor-2-(trifluormethyl)benzyliden]-2,2-dimethyl-1,3-dioxan-4,6-dion in 60 ml Dioxan wurde bei 80°C gerührt. Nach 3 h wurde das Reaktionsgemisch auf RT abgekühlt, mit 2.2 g (5.7 mmol) HATU und 1.0 ml (5.7 mmol) *N,N*-Diisopropylethylamin versetzt, über Nacht bei RT gerührt und im Vakuum eingeengt. Nach Zugabe von Wasser/Diethylether und Phasentrennung wurde der anfallende Niederschlag filtriert, mit Diethylether gewaschen und im Vakuum getrocknet. Die Mutterlauge wurde mit Wasser/Essigsäureethylester versetzt, nach Phasentrennung wurde die organische Phase getrocknet (Natriumsulfat), filtriert und im Vakuum eingeengt. Die Aufreinigung des Rohproduktes erfolgte anschließend mittels Flash-Chromatographie (Kieselgel-60, Eluent: Dichlormethan-Methanol-Gemische). Ausbeute: 1.46 g (28% d. Th., 92% Reinheit) und 1.30 g (26% d. Th., 93% Reinheit)
LC/MS [Methode 1]: Rₜ = 0.85 min; MS (ESIpos): m/z = 330 (M+H)⁺
¹H-NMR (400 MHz, DMSO-d₆): δ [ppm] = 10.85 (s, 1H), 7.74 (d, 1H), 7.52 (dd, 1H), 7.24 (d, 1H), 4.18 (t, 1H), 2.98 (dd, 1H), 2.78 (dd, 1H), 2.67-2.60 (m, 1H), 2.47-2.33 (m, 3H).

### Beispiel 6.1C

### 4-[5-Chlor-2-(trifluormethyl)phenyl]-6,7-dihydro-1H-cyclopenta[b]pyridin-2,5-dion

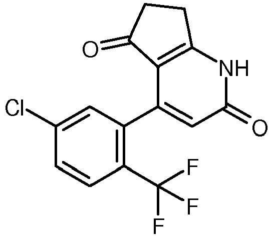

Eine Lösung von 2.8 g (7.8 mmol, 93% Reinheit) 4-[5-Chlor-2-(trifluormethyl)phenyl]-3,4,6,7-tetrahydro-1*H*-cyclopenta[b]pyridin-2,5-dion (Racemat) in 130 ml Dioxan wurde mit einer Lösung von 21.3 g (38.9 mmol, 5.0 eq.) Ammoniumcer(IV)nitrat in 45 ml Wasser versetzt und 5 h bei 50°C gerührt. Nach Abkühlen auf RT wurde das Reaktionsgemisch im Vakuum eingeengt. Nach Zugabe von Wasser/Essigsäureethylester und Phasentrennung wurde die organische Phase mit Wasser und mit gesättigter, wässriger Natriumchlorid-Lösung gewaschen, getrocknet (Natriumsulfat), filtriert und im Vakuum eingeengt. Der Rückstand wurde mit Diethylether verrührt, filtriert und im Vakuum getrocknet. Ausbeute: 1.7 g (63% d. Th.)
LC/MS [Methode 1]: Rₜ = 0.83 min; MS (ESIpos): m/z = 328 (M+H)⁺
¹H-NMR (400 MHz, DMSO-d₆): δ [ppm] = 12.74 (s, 1H), 7.82 (d, 1H), 7.70 (d, 1H), 7.44 (s, 1H), 6.10 (s, 1H), 2.98-2.92 (m, 2H), 2.40-2.44 (m, 2H).

### Beispiel 6.1D

### {4-[5-Chlor-2-(trifluormethyl)phenyl]-2,5-dioxo-2,5,6,7-tetrahydro-1H-cyclopenta[b]pyridin-1-yl}essigsäure-tert.-butylester

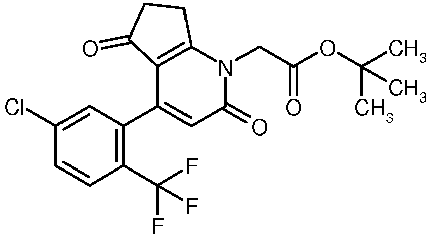

Eine Lösung von 700 mg (2.1 mmol) 4-[5-Chlor-2-(trifluormethyl)phenyl]-6,7-dihydro-1*H-*cyclopenta[b]pyridin-2,5-dion, 0.36 ml (2.5 mmol, 1.2 eq.) Bromessigsäeure-*tert*.-butylester und 425 mg (1.5 mmol) Kaliumcarbonat in 15 ml Dimethylformamid wurde 2 h bei 120°C gerührt. Nach Abkühlen auf RT wurde das Reaktionsgemisch im Vakuum eingeengt. Nach Zugabe von Wasser/Essigsäureethylester und Phasentrennung wurde die organische Phase mit Wasser und mit gesättigter, wässriger Natriumchlorid-Lösung gewaschen, getrocknet (Natriumsulfat), filtriert und im Vakuum eingeengt. Die Aufreinigung des Rohproduktes erfolgte anschließend mittels Flash-Chromatographie (Kieselgel-60, Eluent: Cyclohexan-Essigsäureethylester-Gemische). Ausbeute: 625 mg (67% d. Th.)
LC/MS [Methode 1]: Rₜ = 1.11 min; MS (ESIpos): m/z = 442 (M+H)⁺
¹H-NMR (400 MHz, DMSO-d₆): δ [ppm] = 7.84 (d, 1H), 7.72 (dd, 1H), 7.48 (d, 1H), 6.30 (s, 1H), 4.79 (dd, 2H), 3.07-3.02 (m, 2H), 2.59-2.55 (m, 2H), 1.44 (s, 9H).

### Beispiel 6.1E

### {4-[5-Chlor-2-(trifluormethyl)phenyl]-2,5-dioxo-2,5,6,7-tetrahydro-1H-cyclopenta[b]pyridin-1-yl}essigsäure

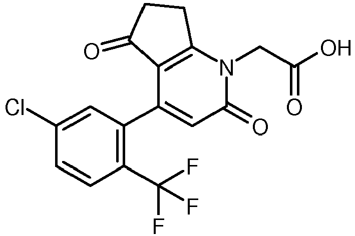

Nach der allgemeinen Methode 2A wurden 605 mg (1.3 mmol) {4-[5-Chlor-2-(trifluormethyl)phenyl]-2,5-dioxo-2,5,6,7-tetrahydro-1*H*-cyclopenta[b]pyridin-1-yl}essigsäure-*tert*.-butylester mit TFA verseift. Ausbeute: 690 mg (quant.)
LC/MS [Methode 1]: Rₜ = 0.79 min; MS (ESIpos): m/z = 386 (M+H)⁺.

### Beispiel 6.2A

5-{4-[({4-[5-Chlor-2-(trifluormethyl)phenyl]-2,5-dioxo-2,5,6,7-tetrahydro-1H-cyclopenta[b]pyridin-1-yl}acetyl)amino]phenyl}-3-oxo-2,3-dihydro-1*H*-pyrazol-1-carbonsäure-*tert*.-butylester

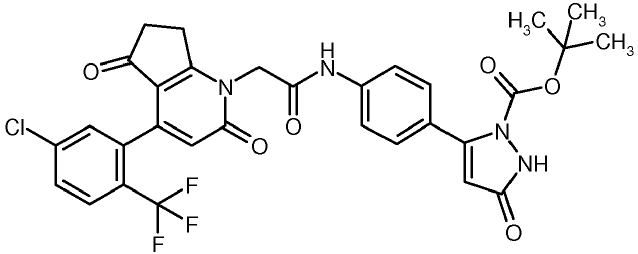

Nach der allgemeinen Methode 1A wurden 100 mg (0.4 mmol) {4-[5-Chlor-2-(trifluormethyl)phenyl]-2,5-dioxo-2,5,6,7-tetrahydro-1*H*-cyclopenta[b]pyridin-1-yl}essigsäure mit 83 mg (0.27 mmol, 1.1 eq.) 5-(4-Aminophenyl)-3-oxo-2,3-dihydro-1*H*-pyrazol-1-carbonsäure-*tert*.-butylester umgesetzt. Ausbeute: 36 mg (15% d. Th., 64% Reinheit)
LC/MS [Methode 1]: Rₜ = 1.14 min; MS (ESIpos): m/z = 643 (M+H)⁺.

### Beispiel 7.1A

### (4-Hydroxy-2,5-dioxo-2,5,6,7-tetrahydro-1H-cyclopenta[b]pyridin-1-yl)-essigsäure-tert.-butylester

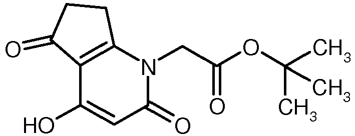

Eine Lösung von 1.5 g (7.1 mmol) *N*-(3-Oxocyclopent-1-en-1-yl)glycinsäure-*tert*.-butylester und 3.62 g (7.81 mmol) Bis(2,4,6-trichlorphenylmalonat) in 20 ml Diethylenglycoldimethylether wurde für 3 h bei 100°C gerührt. Die Reaktionsmischung wurde auf Raumtemperatur abgekühlt und das Lösungsmittel unter reduziertem Druck entfernt. Der Rückstand wurde mit 50 ml Diethylether verrührt und der Niederschlag abgesaugt, mit Diethylether gewaschen und im Vakuum getrocknet. Ausbeute: 1.0 g (49% d. Th., 89% Reinheit)
LC/MS [Methode 1]: Rₜ = 0.86 min; MS (ESIpos): m/z = 280 (M+H)⁺.
¹H-NMR (400 MHz, DMSO-d₆): δ [ppm] = 11.17 (br. s, 1H), 5.54 (s, 1H), 4.63 (s, 2H), 2.92-2.88 (m, 2H), 2.55-2.49 (m, 2H), 1.42 (s, 9H).

### Beispiel 7.1B

### (2,5-Dioxo-4-{[(trifluormethyl)sulfonyl]oxy}-2,5,6,7-tetrahydro-1H-cyclopenta[b]pyridin-1-yl)essigsäure-tert. -butylester

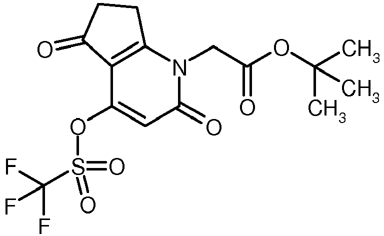

Eine Lösung von 1.06 g (3.80 mmol) (4-Hydroxy-2,5-dioxo-2,5,6,7-tetrahydro-1*H-*cyclopenta[*b*]pyridin-1-yl)essigsäure-*tert*.-butylester in 21 ml Dichlormethan wurde bei 0°C mit 580 µl (4.18 mmol, 1.1 eq.) Triethylamin versetzt. Anschließend wurden 1.49 g (4.18 mmol, 1.1 eq.) *N,N*-Bis(trifluormethansulfonyl)anilin portionsweise hinzugegeben. Es wurde 2 d bei Raumtemperatur gerührt. Das Lösungsmittel wurde unter reduziertem Druck entfernt und der Rückstand mittels MPLC (120 g, 30 µm Kartusche, 50 ml/min, Cyclohexan/Essigsäureethylester-Gradient: 10 min 100% Cyclohexan, 15 min 75% Cyclohexan, 35 min 66% Cyclohexan, 1 min 50% Cyclohexan, dann isokratisch) aufgereinigt. Ausbeute: 950 mg (55% d. Th., 90% Reinheit)
LC/MS [Methode 1]: Rₜ = 1.03 min; MS (ESIpos): m/z = 412 (M+H)⁺
¹H-NMR (400 MHz, DMSO-d₆): δ [ppm] = 6.64 (s, 1H), 4.76 (s, 2H), 3.10-3.07 (m, 2H), 2.73-2.69 (m, 2H), 1.43 (s, 9H).

### Beispiel 7.1C

### [4-(5-Chlor-2-cyanophenyl)-2,5-dioxo-2,5,6,7-tetrahydro-1H-cyclopenta[b]pyridin-1-yl]essigsäure-tert.-butylester

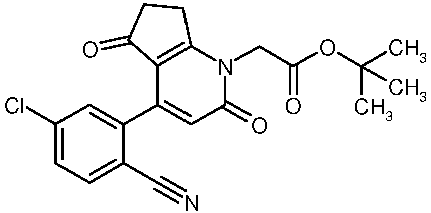

In einem ausgeheizten, mit Argon gespülten Kolben wurden 472 mg (1.15 mmol) (2,5-Dioxo-4-{[(trifluormethyl)sulfonyl]oxy}-2,5,6,7-tetrahydro-1*H*-cyclopenta[*b*]pyridin-1-yl)essigsäure-*tert*.-butylester, 239 mg (1.32 mmol, 1.15 eq.) 5-Chlor-2-cyanophenylboronsäure, 478 mg (3.44 mmol, 3.0 eq.) Kaliumcarbonat und 133 mg (0.115 mmol, 0.1 eq.) Tetrakis(triphenylphosphin)-palladium(0) vorgelegt, dreimal evakuiert und mit Argon belüftet. Es wurden 15 ml Dioxan zugegeben und das Reaktionsmisch 16 h bei 110°C gerührt. Nach Abkühlen auf RT wurde das Reaktionsgemisch anschließend über Celite filtriert und das Filtrat unter reduziertem Druck eingeengt. Das Rohprodukt wurde mittels MPLC (120 g, 30 µm Kartusche, 50 ml/min, Cyclohexan/Essigsäureethylester-Gradient: 10 min 100% Cyclohexan, 15 min 75% Cyclohexan, 35 min 66% Cyclohexan, 1 min 50% Cyclohexan, dann 25 min isokratisch) gereinigt. Ausbeute: 236 mg (50% d. Th.)
LC/MS [Methode 1]: Rₜ = 1.00 min.; MS (ESIneg): m/z = 397 (M+H)⁻
¹H-NMR (400 MHz, DMSO-d₆): δ [ppm] = 7.97 (d, 1H), 7.74 (dd, 1H), 7.68 (d, 1H), 6.49 (s, 1H), 4.81 (s, 2H), 3.10-3.05 (m, 2H), 2.64-2.60 (m, 2H), 1.45 (s, 9H).

### Beispiel 7.1D

### [4-(5-Chlor-2-cyanophenyl)2,5-dioxo-2,5,6,7-tetrahydro-1H-cyclopenta[b]pyridine-1-yl]essigsäure

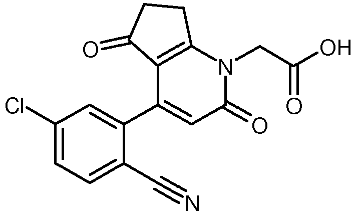

Nach der allgemeinen Methode 2A wurden 150 mg (380 µmol) [4-(5-Chlor-2-cyanophenyl)-2,5-dioxo-2,5,6,7-tetrahydro-1*H*-cyclopenta[*b*]pyridin-1-yl]essigsäure-*tert*.-butylester mit TFA verseift. Ausbeute: 120 mg (90% d. Th.)
LC/MS [Methode 1]: Rₜ = 0.65 min.; MS (ESIpos): m/z = 343 (M+H)⁺
¹H-NMR (400 MHz, DMSO-d₆): δ [ppm] = 13.4 (br. s, 1H), 7.97 (d, 1H), 7.74 (dd, 1H), 7.69 (d, 1H), 6.49 (s, 1H), 4.82 (s, 2H), 3.13-3.07 (m, 2H), 2.64-2.59 (m, 2H).

### Beispiel 7.2A

### 4-({[4-(5-Chlor-2-cyanophenyl)-2,5-dioxo-2,5,6,7-tetrahydro-1H-cyclopenta[b]pyridin-1-yl]acetyl}amino)benzoesäure-tert.-butylester

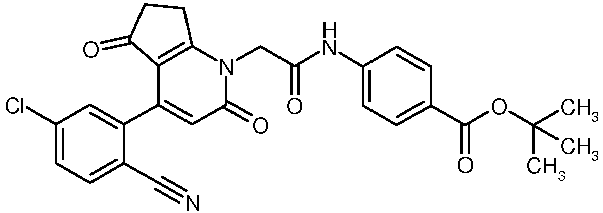

Nach der allgemeinen Methode 1A wurden 90 mg (0.26 mmol) [4-(5-Chlor-2-cyanophenyl)2,5-dioxo-2,5,6,7-tetrahydro-1*H*-cyclopenta[*b*]pyridine-1-yl]essigsäure mit 61 mg (0.32 mmol, 1.2 eq.) 4-Aminobenzoesäure-*tert*.-butylester umgesetzt. Ausbeute: 55 mg (40% d. Th.)
LC/MS [Methode 1]: Rₜ = 1.12 min.; MS (ESIneg): m/z = 516 (M+H)⁻
¹H-NMR (400 MHz, DMSO-d₆): δ [ppm] = 10.88 (s, 1H), 7.98 (d, 1H), 7.89 (d, 2H), 7.76-7.68 (m, 4H), 6.49 (s, 1H), 4.97 (s, 2H), 3.17-3.12 (m, 2H), 2.65-2.60 (m, 2H), 1.54 (s, 9H).

### Beispiel 7.3A

### 5-[4-({[4-(5-Chlor-2-cyanophenyl)-2,5-dioxo-2,5,6,7-tetrahydro-1H-cyclopenta[b]pyridin-1-yl]acetyl }amino)phenyl]-3-oxo-2,3-dihydro-1H-pyrazol-1-carbonsäure-tert. -butylester

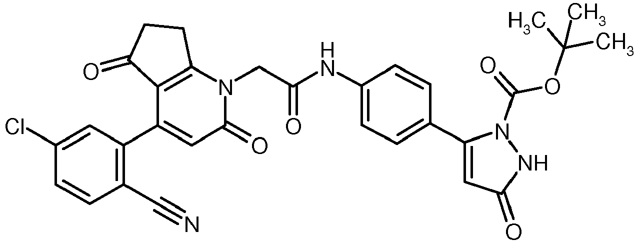

Nach der allgemeinen Methode 1A wurden 118 mg (0.296 mmol, 86% Reinheit) [4-(5-Chlor-2-cyanophenyl)2,5-dioxo-2,5,6,7-tetrahydro-1*H*-cyclopenta[*b*]pyridine-1-yl]essigsäure mit 112 mg (0.326 mmol, 1.2 eq., 80% Reinheit) 5-(4-Aminophenyl)-3-oxo-2,3-dihydro-1*H*-pyrazol-1-carbonsäure-*tert*.-butylester umgesetzt. Ausbeute: 100 mg (55% d. Th.)
LC/MS [Methode 1]: Rₜ = 1.04 min.; MS (ESIpos): m/z = 600 (M+H)⁺
¹H-NMR (400 MHz, DMSO-d₆): δ [ppm] = 12.95 (d, 1H), 10.72 (s, 1H), 7.98 (d, 1H), 7.76-7.67 (m, 6H), 6.51-6.49 (m, 2H), 4.97 (s, 2H), 3.17-3.13 (m, 2H), 2.65-2.61 (m, 2H), 1.50 (s, 9H).

### Beispiel 7.4A

### (4-Hydroxy-2,5-dioxo-5,6,7,8-tetrahydrochinolin-1(2H)-yl)essigsäure-tert.-butylester

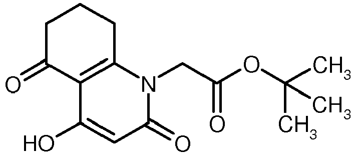

Eine Lösung von 2.00 g (8.88 mmol) *N*-(3-Oxocyclohex-1-en-1-yl)glycinsäure-*tert*.-butylester und 4.52 g (9.77 mmol, 1.1 eq.) Bis(2,4,6-trichlorphenylmalonat) in 25 ml Diethylenglycoldimethylether wurde 5 h bei 100°C gerührt. Die Reaktionsmischung wurde auf Raumtemperatur abgekühlt und das Lösungsmittel unter reduziertem Druck entfernt. Der Rückstand wurde in 8 ml Dichlormethan gelöst und mittels MPLC (120 g, 30 µm Kartusche, 50 ml/min Cyclohexan/Essigsäureethylester-Gradient: 10 min 100% Cyclohexan, 15 min 75% Cyclohexan, 35 min 66% Cyclohexan, 1 min 50% Cyclohexan, dann 25 min isokratisch) gereinigt. Ausbeute: 720 mg (27% d. Th.)
LC/MS [Methode 1]: Rₜ = 0.83 min; MS (ESIpos): m/z = 294 (M+H)⁺
¹H-NMR (400 MHz, DMSO-d₆): δ [ppm] = 12.75 (br. s, 1H), 5.60 (s, 1H), 4.81 (s, 2H), 2.92-2.87 (m, 2H), 2.61-2.56 (m, 2H), 2.07-1.99 (m, 2H), 1.43 (s, 9H).

### Beispiel 7.4B

### [2,5-Dioxo-4-{[(trifluormethyl)sulfonyl]oxy}-5,6,7,8-tetrahydrochinolin-1(2H)-yl]essigsäure-tert.-butylester

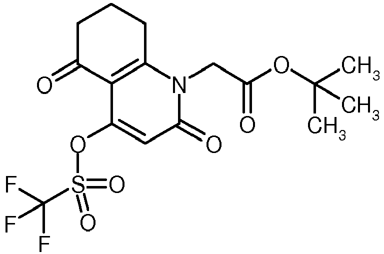

Eine Lösung von 717 mg (2.44 mmol) (4-Hydroxy-2,5-dioxo-5,6,7,8-tetrahydrochinolin-1(2*H*)-yl)essigsäure-*tert*.-butylester in 14 ml Dichlormethan wurde bei 0°C mit 375 µl (2.70 mmol, 1.1 eq.) Triethylamin versetzt. Anschließend wurden 961 mg (2.70 mmol, 1.1 eq.) *N,N-*Bis(trifluormethansulfonyl)anilin portionsweise hinzugegeben und 6 d bei 60°C gerührt. Nach Abkühlen auf RT wurde das Lösungsmittel unter reduziertem Druck entfernt und der Rückstand mittels MPLC (120 g, 30 µm Kartusche, 50 ml/min, Cyclohexan/Essigsäureethylester-Gradient: 10 min 100% Cyclohexan, 15 min 75% Cyclohexan, 35 min 66% Cyclohexan, 1 min 50% Cyclohexan, dann isokratisch) aufgereinigt. Ausbeute: 128 mg (12% d. Th.)
LC/MS [Methode 1]: Rₜ = 1.07 min; MS (ESIneg): m/z = 424 (M+H)⁻
¹H-NMR (400 MHz, DMSO-d₆): δ [ppm] = 6.55 (s, 1H), 4.86 (s, 2H), 2.99-2.94 (m, 2H), 2.54-2.45 (m, 2H), 2.06-1.98 (m, 2H), 1.43 (s, 9H).

### Beispiel 7.4C

### [4-(5-Chlor-2-cyanophenyl)-2,5-dioxo-5,6,7,8-tetrahydrochinolin-1(2H)-yl]essigsäure-tert.-butylester

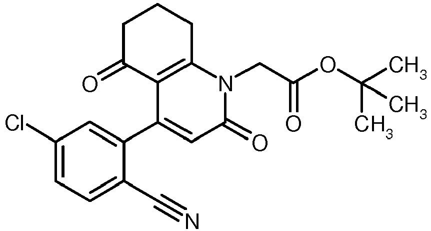

In einem ausgeheizten, mit Argon gespülten Kolben wurden 125 mg (0.29 mmol) [2,5-Dioxo-4-{[(trifluormethyl)sulfonyl]oxy}-5,6,7,8-tetrahydrochinolin-1(2*H*)-yl]essigsäure-*tert*.-butylester, 61.3 mg (0.34 mmol, 1.15 eq.) 5-Chlor-2-cyanophenylboronsäure, 122 mg (0.88 mmol, 3.0 eq.) Kaliumcarbonat und 33.9 mg (0.029 mmol, 0.1 eq.) Tetrakis(triphenylphosphin)palladium(0) vorgelegt, dreimal evakuiert und mit Argon belüftet. Es wurden 15 ml Dioxan zugegeben und das Reaktionsmisch für 16 h bei 110°C gerührt. Nach Abkühlen auf RT wurde das Reaktionsgemisch anschließend über Celite filtriert und das Filtrat unter reduziertem Druck eingeengt. Das Rohprodukt wurde mittels präp. HPLC (Säule: Chromatorex C18,10 µm, 125*30 mm, Acetonitril/Wasser+0.05% Ameisensäure-Gradient: 0-3 min 10% Acetonitril, bis 35 min 90% Acetonitril und weitere 3 min 90% Acetonitril) gereinigt. Ausbeute: 45 mg (37% d. Th.)
LC/MS [Methode 1]: Rₜ = 1.05 min.; MS (ESIneg): m/z = 411 (M+H)⁻
¹H-NMR (400 MHz, DMSO-d₆): δ [ppm] = 7.88 (d, 1H), 7.63 (dd, 1H), 7.55 (d, 1H), 6.36 (s, 1H), 4.91 (d, 2H), 3.06-2.88 (m, 2H), 2.44-2.37 (m, 2H), 2.12-1.96 (m, 2H), 1.45 (s, 9H).

### Beispiel 7.4D

### [4-(5-Chlor-2-cyanophenyl)-2,5-dioxo-5,6,7,8-tetrahydrochinolin-1(2H)-yl]essigsäure

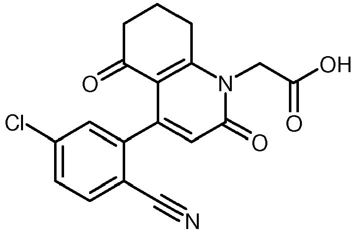

Nach der allgemeinen Methode 2A wurden 44.0 mg (107 µmol) [4-(5-Chlor-2-cyanophenyl)-2,5-dioxo-5,6,7,8-tetrahydrochinolin-1(2*H*)-yllessigsäure-*tert*.-butylester mit TFA verseift. Ausbeute: 38 mg (quant.)
LC/MS [Methode 3]: Rₜ = 1.67 min.; MS (ESIpos): m/z = 357 (M+H)⁺
¹H-NMR (400 MHz, DMSO-d₆): δ [ppm] = 13.39 (br. s, 1H), 7.88 (d, 1H), 7.63 (dd, 1H), 7.56 (d, 1H), 6.36 (s, 1H), 4.97-4.86 (m, 2H), 3.10-2.89 (m, 2H), 2.44-2.39 (m, 2H), 2.10-1.97 (m, 2H).

### Beispiel 7.5A

### 4-({[4-(5-Chlor-2-cyanophenyl)-2,5-dioxo-5,6,7,8-tetrahydrochinolin-1(2H)-yl]acetyl}amino)-benzoesäure-tert.-butylester

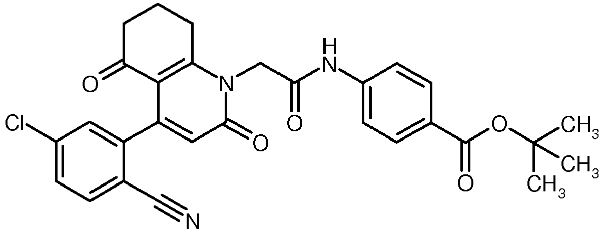

Nach der allgemeinen Methode 1A wurden 40.0 mg (0.112 mmol) [4-(5-Chlor-2-cyanophenyl)-2,5-dioxo-5,6,7,8-tetrahydrochinolin-1(2*H*)-yl]essigsäure mit 26.0 mg (0.135 mmol, 1.2 eq.) 4-Aminobenzoesäure-tert.-butylester-umgesetzt. Ausbeute: 16 mg (26% d. Th.)
LC/MS [Methode 3]: Rt = 2.46 min.; MS (ESIneg): m/z = 531 (M+H)⁻
¹H-NMR (400 MHz, DMSO-d₆): δ [ppm] = 10.86 (s, 1H), 7.89 (d, 1H), 7.88 (d, 2H), 7.72 (d, 2H), 7.64 (dd, 1H), 7.56 (d, 1H), 6.37 (s, 1H), 5.07 (q, 2H), 3.16-2.95 (m, 2H), 2.45-2.38 (m, 2H), 2.10-1.99 (m, 2H), 1.54 (s, 9H).

### Ausführungsbeispiele

### Allgemeine Methode 1: Amidkupplung mit Carbonsäuren

Eine Lösung der entsprechenden Carbonsäure (1.0 eq.) in Dimethylformamid (ca. 12 ml/mmol) wurde unter Argon bei RT mit dem entsprechenden Amin (1.1 eq.), *N,N*-Diisopropylethylamin (2.2 eq.) und einer Lösung aus HATU (1.2 eq.) in wenig DMF versetzt. Das Reaktionsgemisch wurde bei RT gerührt. Nach Zugabe von Wasser/Essigsäureethylester und Phasentrennung wurde die organische Phase mit Wasser und mit gesättigter, wässriger Natriumchlorid-Lösung gewaschen, getrocknet (Natriumsulfat), filtriert und im Vakuum eingeengt. Die Aufreinigung des Rohproduktes erfolgte anschließend mittels präparativer HPLC (Reprosil C18, Wasser-Acetonitril-Gradient oder Wasser-Methanol-Gradient).

### Allgemeine Methode 2: Verseifung eines tert.-Butylesters oder eines Boc-geschützten Amins mittels TFA

Eine Lösung des entsprechenden *tert*.-Butylestersderivates oder eines Boc-geschützten Amins (1.0 eq.) in Dichlormethan (ca. 25 ml/mmol) wurde bei RT mit TFA (20 eq.) versetzt und bei RT für 1-8 h gerührt. Anschließend wurde das Reaktionsgemisch im Vakuum eingeengt. Der Rückstand wurde dreimal mit Dichlormethan coevaporiert. Die Aufreinigung des Rohproduktes erfolgte anschließend mittels präparative RP-HPLC (Eluent: Acetonitril-Wasser-Gradient oder Wasser-Methanol-Gradient).

### Allgemeine Methode 3: Verseifung eines Methyl- oder Ethylesters

Eine Lösung des entsprechenden Methyl- oder Ethylesters (1.0 eq.) in einem Gemisch aus Tetrahydrofuran/Wasser (3:1, ca. 15 ml/mmol) wurde bei RT mit Lithiumhydroxid (2-4eq.) versetzt und bei RT gerührt. Anschließend wurde das Reaktionsgemisch mit wässriger SalzsäureLösung (1N) auf pH 1 gestellt. Nach Zugabe von Wasser/Essigsäureethylester wurde die wässrige Phase dreimal mit Essigsäureethylester extrahiert. Die vereinigten, organischen Phasen wurden getrocknet (Natriumsulfat), filtriert und im Vakuum eingeengt. Das Rohprodukt wurde anschließend entweder mittels Flash-Chromatographie (Kieselgel-60, Eluent: Cyclohexan-Essigsäureethylester-Gemische oder Dichlormethan-Methanol-Gemische) oder präparativer HPLC (Reprosil C18, Wasser-Acetonitril-Gradient oder Wasser-Methanol-Gradient) aufgereinigt.

### Beispiel 1

### 2-[4-(3-Chlorphenyl)-2,5-dioxo-2,5,6,7-tetrahydro-1H-pyrrolo[3,4-b]pyridin-1-yl]-N-[4-(1H-tetrazol-5-yl)phenyl]acetamid

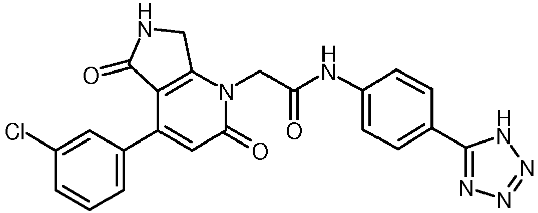

Eine Lösung von 78 mg (0.17 mmol) 2-[4-(3-Chlorphenyl)-2,5-dioxo-2,3,4,5,6,7-hexahydro-1*H-*pyrrolo[3,4-b]pyridin-1-yl]-*N*-[4-(1*H*-tetrazol-5-yl)phenyl]acetamid (Racemat) in 2.7 ml Aceton wurde mit einer Lösung von 369 mg (0.67 mmol, 4.0 eq.) Ammoniumcer(IV)nitrat in 0.7 ml Wasser versetzt und über Nacht bei RT gerührt. Anschließend wurde das Reaktionsgemisch auf Wasser gegeben, der Niederschlag filtriert und im Vakuum getrocknet. Die Aufreinigung des Rohproduktes erfolgte mittels präparativer HPLC (Sunfire C 18 5µm, Wasser-Methanol-Gradient). Ausbeute: 32 mg (41% d. Th.)
LC/MS [Methode 4]: Rₜ = 0.86 min; MS (ESIpos): m/z = 462 (M+H)⁺
¹H-NMR (400 MHz, DMSO-d₆): δ [ppm] = 10.81 (s, 1H), 8.32 (s, 1H), 8.00 (d, 2H), 7.79 (d, 2H), 7.65 (s, 1H), 7.56-7.44 (m, 3H), 6.42 (s, 1H), 4.86 (s, 2H), 4.39 (s, 2H).

### Beispiel 2

### 2-[4-(2,5-Dichlorphenyl)-6-methyl-2,5-dioxo-2,5,6,7-tetrahydro-1H-pyrrolo[3,4-b]pyridin-1-yl]-N-[4-(1H-tetrazol-5-yl)phenyl] acetamid

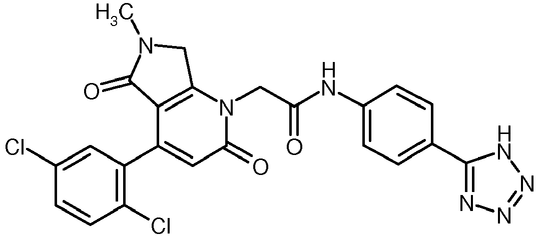

Nach der allgemeinen Methode 1 wurden 105 mg (0.16 mmol, 56% Reinheit) [4-(2,5-Dichlorphenyl)-6-methyl-2,5-dioxo-2,5,6,7-tetrahydro-1*H*-pyrrolo[3,4-b]pyridin-1-yl]essigsäure mit 28 mg (0.18 mmol, 1.1 eq.) 4-(1*H*-Tetrazol-5-yl)anilin umgesetzt. Ausbeute: 12 mg (14% d. Th.)
LC/MS [Methode 1]: Rₜ = 0.79 min; MS (ESIpos): m/z = 510 (M+H)⁺
¹H-NMR (400 MHz, DMSO-d₆): δ [ppm] = 10.87 (s, 1H), 8.02 (d, 2H), 7.82 (d, 2H), 7.57 (d, 1H), 7.53 (dd, 1H), 7.47 (d, 1H), 6.37 (s, 1H), 4.89 (s, 2H), 4.50 (s, 2H), 2.92 (s, 3H).

### Beispiel 3

### 2-[4-(2,5-Dichlorphenyl)-2,5-dioxo-2,5,6,7-tetrahydro-1H-cyclopenta[b]pyridin-1-yl]-N-[4-(1H-tetrazol-5-yl)phenyl]acetamid

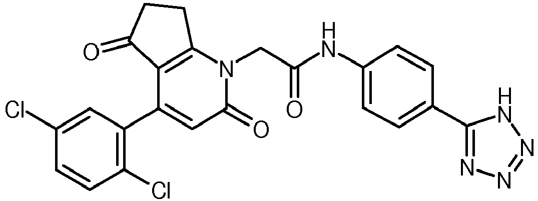

Eine Lösung von 113 mg (0.2 mmol, 89% Reinheit) 2-[4-(2,5-Dichlorphenyl)-2,5-dioxo-2,3,4,5,6,7-hexahydro-1*H*-cyclopenta[b]pyridin-1-yl]-*N*-[4-(1*H*-tetrazol-5-yl)phenyl] acetamid (Racemat) in 3 ml Aceton wurde bei RT mit einer Lösung von 443 mg (0.8 mmol) Ammoniumcer(IV)nitrat in 0.8 ml Wasser versetzt, über Nacht bei RT gerührt und anschließend im Vakuum eingeengt. Nach Zugabe von Wasser/Essigsäureethylester und Phasentrennung wurde die organische Phase mit Wasser und mit gesättigter, wässriger Natriumchlorid-Lösung gewaschen, getrocknet (Natriumsulfat), filtriert und im Vakuum eingeengt. Die Aufreinigung des Rohproduktes erfolgte mittels präparativer HPLC (Kromasil 100 C18, Acetonitril/Wasser + 2%ige Ameisensäure). Ausbeute: 8 mg (8% d. Th.)
LC/MS [Methode 1]: Rₜ = 0.87 min; MS (ESIpos): m/z = 495 (M+H)⁺
¹H-NMR (400 MHz, DMSO-d₆): δ [ppm] = 16.77 (br. s, 1H), 10.88 (s, 1H), 8.02 (d, 2H), 7.83 (d, 2H), 7.56 (s, 1H), 7.54 (d, 1H), 7.45 (d, 1H), 6.34 (s, 1H), 4.97 (s, 2H), 3.19-3.05 (m, 2H), 2.68-2.55 (m, 2H).

### Beispiel 4

### 4-({[4-(2,5-Dichlorphenyl)-2,5-dioxo-2,5,6,7-tetrahydro-1H-cyclopenta[b]pyridin-1-yl]acetyl }amino)benzoesäure

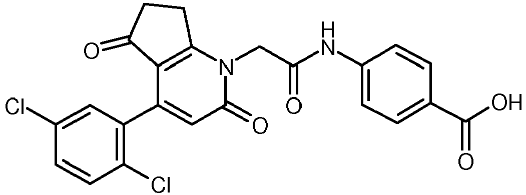

Nach der allgemeinen Methode 2 wurden 43 mg (0.08 mmol, 94% Reinheit) 4-({[4-(2,5-Dichlorphenyl)-2,5-dioxo-2,5,6,7-tetrahydro-1*H*-cyclopenta[b]pyridin-1-yl]acetyl}amino)-benzoesäure-*tert*.-butylester mit TFA verseift. Ausbeute: 39 mg (99% d. Th., 92% Reinheit)
LC/MS [Methode 1]: Rₜ = 0.86 min; MS (ESIpos): m/z = 471 (M+H)⁺
¹H-NMR (400 MHz, DMSO-d₆): δ [ppm] = 10.87 (s, 1H), 7.93 (d, 2H), 7.72 (d, 2H), 7.57 (d, 1H), 7.52 (dd, 1H), 7.45 (d, 1H), 6.33 (s, 1H), 4.96 (s, 2H), 3.15-3.07 (br. s, 2H), 2.63-2. (br. s, 2H).

### Beispiel 5

### 2-[4-(2,5-Dichlorphenyl)-2,5-dioxo-2,5,6,7-tetrahydro-1H-cyclopenta[b]pyridin-1-yl]-N-[4-(5-oxo-4,5-dihydro-1,2,4-oxadiazol-3-yl)phenyl]acetamid

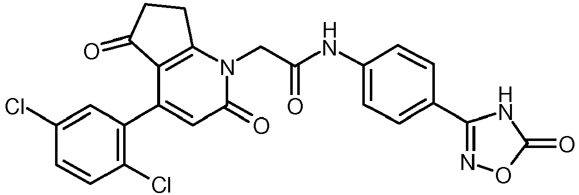

Nach der allgemeinen Methode 1 wurden 120 mg (0.29 mmol, 86% Reinheit) [4-(2,5-Dichlorphenyl)-2,5-dioxo-2,5,6,7-tetrahydro-1*H*-cyclopenta[b]pyridin-1-yl]essigsäure und 57 mg (0.32 mmol) 3-(4-Aminophenyl)-1,2,4-oxadiazol-5(4*H*)-on umgesetzt. Ausbeute: 14 mg (9% d. Th.)
LC/MS [Methode 1]: Rₜ = 0.91 min; MS (ESIpos): m/z = 511 (M+H)⁺
¹H-NMR (400 MHz, DMSO-d₆): δ [ppm] = 10.76 (s, 1H), 7.76 (d, 2H), 7.70 (d, 2H), 7.56 (d, 1H), 7.51 (dd, 1H), 7.44 (d, 1H), 6.33 (s, 1H), 4.95 (s, 2H), 3.15-3.07 (br. s, 2H), 2.63-2.55 (br. s, 2H).

### Beispiel 6

### 2-[4-(2,5-Dichlorphenyl)-2,5-dioxo-2,5,6,7-tetrahydro-1H-cyclopenta[b]pyridin-1-yl]-N-{4-[3-(trifluormethyl)-1H-1,2,4-triazol-5-yl]phenyl}acetamid

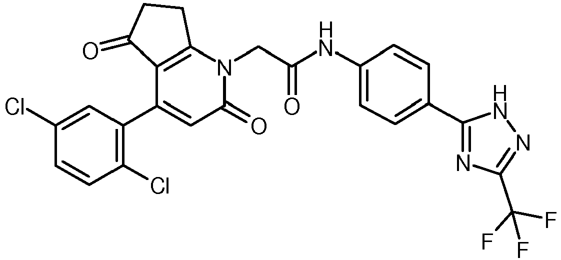

Nach der allgemeinen Methode 1 wurden 120 mg (0.29 mmol, 86% Reinheit) [4-(2,5-Dichlorphenyl)-2,5-dioxo-2,5,6,7-tetrahydro-1*H*-cyclopenta[b]pyridin-1-yl]essigsäure und 77 mg (0.32 mmol, 1.1 eq.) 4-[3-(Trifluormethyl)-1*H*-1,2,4-triazol-5-yl]anilin umgesetzt. Ausbeute: 35 mg (21% d. Th.)
LC/MS [Methode 1]: Rₜ = 1.05 min; MS (ESIpos): m/z = 562 (M+H)⁺
¹H-NMR (400 MHz, DMSO-d₆): δ [ppm] = 15.18 (s, 1H), 10.85 (s, 1H), 8.01 (d, 2H), 7.80 (d, 2H), 7.55 (d, 1H), 7.53 (dd, 1H), 7.44 (d, 1H), 6.33 (s, 1H), 4.97 (s, 2H), 3.16-3.08 (br. s, 2H), 2.64-2.56 (br. s, 2H).

### Beispiel 7

### 2-[4-(2,5-Dichlorphenyl)-2,5-dioxo-2,5,6,7-tetrahydro-1H-cyclopenta[b]pyridin-1-yl]-N-[4-(5-oxo-2,5-dihydro-1H-pyrazol-3-yl)phenyl]acetamid

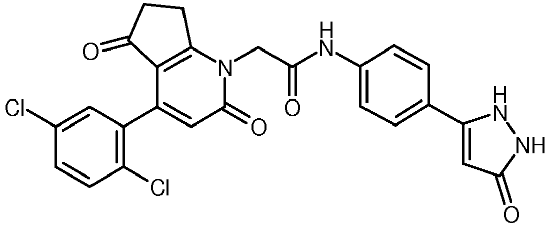

Nach der allgemeinen Methode 2 wurden 53 mg (0.09 mmol) 5-[4-({[4-(2,5-Dichlorphenyl)-2,5-dioxo-2,5,6,7-tetrahydro-1*H*-cyclopenta[b]pyridin-1-yl]acetyl}amino)phenyl]-3-oxo-2,3-dihydro-1*H*-pyrazol-1-carbonsäure-*tert*.-butylester mit TFA verseift. Ausbeute: 5 mg (11% d. Th.)
LC/MS [Methode 1]: Rₜ = 0.86 min; MS (ESIpos): m/z = 509 (M+H)⁺
¹H-NMR (400 MHz, DMSO-d₆): δ [ppm] = 11.98 (s, 1H), 10.65 (s, 1H), 9.57 (s, 1H), 7.63 (br. s, 4H), 7.57 (d, 1H), 7.52 (dd, 1H), 7.45 (d, 1H), 6.33 (s, 1H), 5.85 (br. s, 1H), 4.96 (s, 2H), 3.16-3.04 (br. s, 2H), 2.63-2.55 (br. s, 2H).

### Beispiel 8

### 2-[4-(2,5-Dichlorphenyl)-2,5-dioxo-2,5,6,7-tetrahydro-1H-cyclopenta[b]pyridin-1-yl]-N-[4-(1H-imidazol-5-yl)phenyl] acetamid

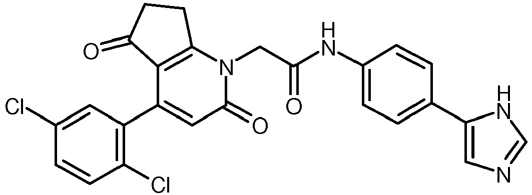

Nach der allgemeinen Methode 1 wurden 102 mg (0.25 mmol, 86% Reinheit) [4-(2,5-Dichlorphenyl)-2,5-dioxo-2,5,6,7-tetrahydro-1*H*-cyclopenta[b]pyridin-1-yl]essigsäure und 44 mg (0.28 mmol, 1.1 eq.) 4-(1*H*-Imidazol-5-yl)anilin umgesetzt. Ausbeute: 23 mg (18% d. Th.)
LC/MS [Methode 1]: Rₜ = 0.75 min; MS (ESIpos): m/z = 493 (M+H)⁺
¹H-NMR (400 MHz, DMSO-d₆): δ [ppm] = 10.58 (s, 1H), 7.90 (br. s, 1H), 7.73 (d, 2H), 7.62 (d, 2H), 7.59-7.55 (m, 2H), 7.53 (dd, 1H), 7.44 (d, 1H), 6.33 (s, 1H), 4.94 (s, 2H), 3.12 (br. s, 2H), 2.60 (br. s, 2H).

### Beispiel 9

### 2-[4-(2-Brom-5-chlorphenyl)-2,5-dioxo-2,5,6,7-tetrahydro-1H-cyclopenta[b]pyridin-1-yl]-N-[4-(1H-tetrazol-5-yl)phenyl]acetamid

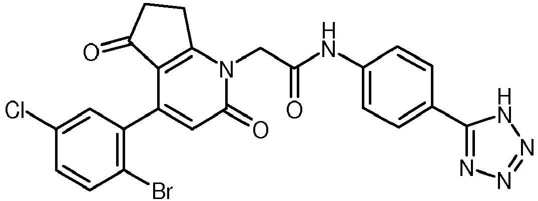

Nach der allgemeinen Methode 1 wurden 89 mg (0.21 mmol, 93% Reinheit) [4-(2-Brom-5-chlorphenyl)-2,5-dioxo-2,5,6,7-tetrahydro-1*H*-cyclopenta[b]pyridin-1-yl]essigsäure mit 37 mg (0.23 mmol, 1.1 eq.) 4-(1*H*-Tetrazol-5-yl)anilin umgesetzt. Ausbeute: 28 mg (25% d. Th.)
LC/MS [Methode 1]: Rₜ = 0.83 min; MS (ESIpos): m/z = 539 (M+H)⁺
¹H-NMR (400 MHz, DMSO-d₆): δ [ppm] = 10.88 (s, 1H), 8.02 (d, 2H), 7.83 (d, 2H), 7.72 (d, 1H), 7.44 (dd, 1H), 7.41 (d, 1H), 6.30 (s, 1H), 4.97 (s, 2H), 3.17-3.09 (br. s, 2H), 2.63-2.56 (br. s, 2H).

### Beispiel 10

### 2-[4-(2-Brom-5-chlorphenyl)-2,5-dioxo-2,5,6,7-tetrahydro-1H-cyclopenta[b]pyridin-1-yl]-N-[4-(5-oxo-4,5-dihydro-1,2,4-oxadiazol-3-yl)phenyl] acetamid

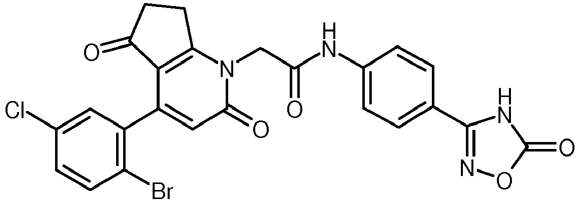

Nach der allgemeinen Methode 1 wurden 90 mg (0.21 mmol, 94% Reinheit) [4-(2-Brom-5-chlorphenyl)-2,5-dioxo-2,5,6,7-tetrahydro-1*H*-cyclopenta[b]pyridin-1-yl]essigsäure mit 45 mg (0.26 mmol, 1.2 eq.) 3-(4-Aminophenyl)-1,2,4-oxadiazol-5(4*H*)-on umgesetzt. Ausbeute: 43 mg (36% d. Th.)
LC/MS [Methode 1]: Rₜ = 0.92 min; MS (ESIpos): m/z = 555 (M+H)⁺
¹H-NMR (400 MHz, DMSO-d₆): δ [ppm] = 12.86 (s, 1H), 10.90 (s, 1H), 7.79 (s, 4H), 7.72 (d, 1H), 7.44 (dd, 1H),7.40 (d, 1H), 6.29 (s, 1H), 4.97 (s, 2H), 3.15-3.08 (br. s, 2H), 2.62-2.56 (br. s, 2H).

### Beispiel 11

### 2-{4-[5-Chlor-2-(trifluormethyl)phenyl]-2,5-dioxo-2,5,6,7-tetrahydro-1H-cyclopenta[b]pyridin-1-yl}-N-[4-(5-oxo-2,5-dihydro-1H-pyrazol-3-yl)phenyl]acetamid

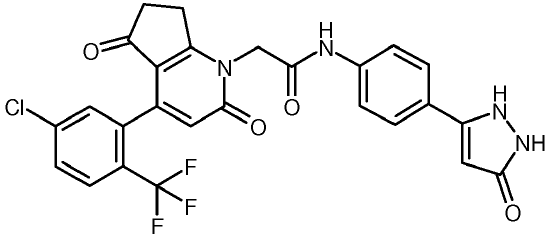

Nach der allgemeinen Methode 2 wurden 35 mg (0.04 mmol, 64% Reinheit) 5-{4-[({4-[5-Chlor-2-(trifluormethyl)phenyl]-2,5-dioxo-2,5,6,7-tetrahydro-1*H*-cyclopenta[b]pyridin-1-yl}acetyl)amino]-phenyl}-3-oxo-2,3-dihydro-1*H*-pyrazol-1-carbonsäure-*tert*.-butylester mit TFA verseift. Ausbeute: 34 mg (quant.)
LC/MS [Methode 1]: Rₜ = 0.89 min; MS (ESIpos): m/z = 543 (M+H)⁺
¹H-NMR (400 MHz, DMSO-d₆): δ [ppm] = 11.98 (s, 1H), 10.65 (s, 1H), 9.57 (s, 1H), 7.85 (d, 1H), 7.74 (dd, 1H), 7.64 (br. s, 4H), 7.49 (d, 1H), 6.30 (s, 1H), 5.85 (br. s, 1H), 4.96 (q, 2H), 3.15-3.08 (m, 2H), 2.60-2.56 (m, 2H).

### Beispiel 12

### 4-({[4-(5-Chlor-2-cyanophenyl)-2,5-dioxo-2,5,6,7-tetrahydro-1H-cyclopenta[b]pyridin-1-yl]acetyl}amino)benzoesäure

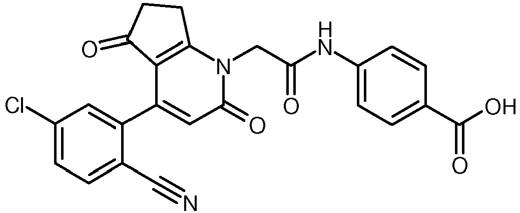

Nach der allgemeinen Methode 2 wurden 54 mg (0.10 mmol) 4-({[4-(5-Chlor-2-cyanophenyl)-2,5-dioxo-2,5,6,7-tetrahydro-1*H*-cyclopenta[*b*]pyridin-1-yl]acetyl}amino)benzoesäure-*tert*.-butylester mit TFA verseift und mittels präparativer HPLC (Säule: Chromatorex C18, 10 µm, 125 mm x 30 mm, Acetonitril/Wasser-Gradient: 0-3 min 10% Acetonitril, bis 35 min 90% Acetonitril und weitere 3 min 90% Acetonitril). Ausbeute: 27 mg (55% d. Th.)
LC/MS [Methode 1]: Rₜ = 0.80 min; MS (ESIpos): m/z = 461 (M+H)⁺
¹H-NMR (400 MHz, DMSO-d₆): δ [ppm] = 12.77 (br. s, 1H), 10.87 (s, 1H), 7.98 (d, 1H), 7.92 (d, 2H), 7.77-7.68 (m, 4H), 6.50 (s, 1H), 4.98 (s, 2H), 3.17-3.12 (m, 2H), 2.66-2.60 (m, 2H).

### Beispiel 13

### 2-[4-(5-Chlor-2-cyanophenyl)-2,5-dioxo-2,5,6,7-tetrahydro-1H-cyclopenta[b]pyridin-1-yl]-N-[4-(5-oxo-4,5-dihydro-1,2,4-oxadiazol-3-yl)phenyl]acetamid

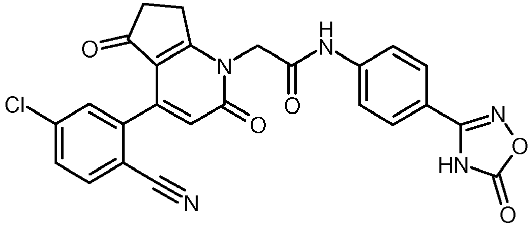

Nach der allgemeinen Methode 1 wurden 90 mg (0.26 mmol) [4-(5-Chlor-2-cyanophenyl)2,5-dioxo-2,5,6,7-tetrahydro-1*H*-cyclopenta[*b*]pyridine-1-yl]essigsäure und 56 mg (0.32 mmol, 1.2 eq.) 3-(4-Aminophenyl)-1,2,4-oxadiazol-5(4*H*)-on umgesetzt und mittels präparativer HPLC (Säule: Chromatorex C18, 10µm, 125 mm x 30 mm, Acetonitril/Wasser-Gradient: 0-3 min 10% Acetonitril, bis 35 min 90% Acetonitril und weitere 3 min 90% Acetonitril). Ausbeute: 34 mg (25% d. Th.)
LC/MS [Methode 1]: Rₜ = 0.82 min; MS (ESIpos): m/z = 502 (M+H)⁺
¹H-NMR (400 MHz, DMSO-d₆): δ [ppm] = 12.87 (br. s, 1H), 10.92 (s, 1H), 7.98 (d, 1H), 7.80 (br. s, 4H), 7.74 (dd, 1H), 7.68 (d, 1H), 6.49 (s, 1H), 4.98 (br. s, 2H), 3.17-3.12 (m, 2H), 2.65-2.61 (m, 2H).

### Beispiel 14

### 2-[4-(5-Chlor-2-cyanophenyl)-2,5-dioxo-2,5,6,7-tetrahydro-1H-cyclopenta[b]pyridin-1-yl]-N-[4-(5-oxo-2,5-dihydro-1H-pyrazol-3-yl)phenyl]acetamid

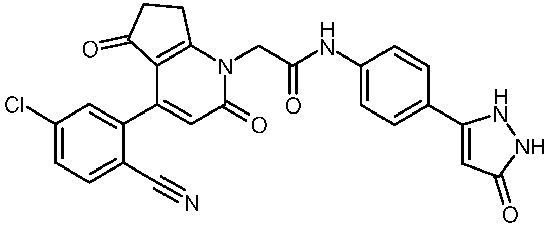

Nach der allgemeinen Methode 2 wurden 93 mg (0.16 mmol) 5-[4-({[4-(5-Chlor-2-cyanophenyl)-2,5-dioxo-2,5,6,7-tetrahydro-1*H*-cyclopenta[*b*]pyridin-1-yl]acetyl}amino)phenyl]-3-oxo-2,3-dihydro-1*H*-pyrazol-1-carbonsäure-*tert*.-butylester mit TFA verseift und mittels präparativer HPLC (Säule: Chromatorex C18, 10 µm, 125 mm x 30 mm, Acetonitril/Wasser-Gradient: 0-3 min 10% Acetonitril, bis 35 min 90% Acetonitril und weitere 3 min 90% Acetonitril). Ausbeute: 71 mg (90% d. Th.)
LC/MS [Methode 1]: Rₜ = 0.73 min; MS (ESIpos): m/z = 500 (M+H)⁺
¹H-NMR (400 MHz, DMSO-d₆): δ [ppm] = 10.57 (br. s, 1H), 7.98 (d, 1H), 7.74 (dd, 1H), 7.70 (d, 1H), 7.52 (br. s, 4H), 6.49 (s, 1H), 4.96 (br. s, 3H), 3.18-3.12 (m, 2H), 2.65-2.60 (m, 2H). 2 N*H-*Resonanzen nicht sichtbar.

### Beispiel 15

### 4-({[4-(5-Chlor-2-cyanophenyl)-2,5-dioxo-5,6,7,8-tetrahydrochinolin-1(2H)-yl]acetyl}amino)-benzoesäure

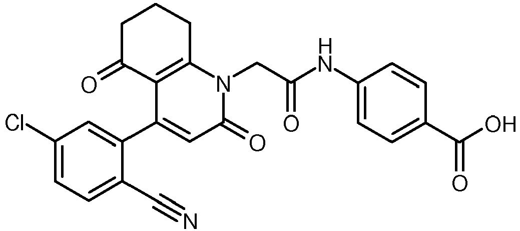

Nach der allgemeinen Methode 2 wurden 15 mg (0.03 mmol) 4-({[4-(5-Chlor-2-cyanophenyl)-2,5-dioxo-5,6,7,8-tetrahydrochinolin-1(2*H*)-yl]acetyl}amino)benzoesäure-*tert*.-butylester mit TFA verseift und mittels präparativer HPLC (Säule: Kromasil, C18, 5 µm, 250 mm x 20 mm, Acetonitril/Wasser+0.05% Ameisensäure-Gradient: 0-3 min 10% Acetonitril, bis 33 min 90% Acetonitril und weitere 8 min 90% Acetonitril) gereinigt. Ausbeute: 5.6 mg (41% d. Th.)
LC/MS [Methode 1]: Rₜ = 0.83 min; MS (ESIneg): m/z = 474 (M+H)⁻
¹H-NMR (400 MHz, DMSO-d₆): δ [ppm] = 12.78 (br. s, 1H), 10.83 (s, 1H), 7.92 (d, 2H), 7.89 (d, 1H), 7.71 (d, 2H), 7.63 (dd, 1H), 7.56 (d, 1H), 6.37 (s, 1H), 5.08 (q, 2H), 3.15-2.94 (m, 2H), 2.45-2.35 (m, 2H), 2.05-2.00 (m, 2H).

### Beispiel 16

### 2-[4-(5-Chlor-2-cyanophenyl)-2,5-dioxo-5,6,7,8-tetrahydrochinolin-1(2H)-yl]-N-[4-(5-oxo-4,5-dihydro-1,2,4-oxadiazol-3-yl)phenyl] acetamid

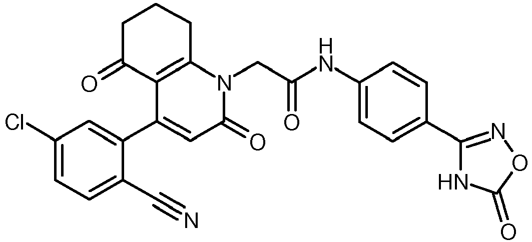

Nach der allgemeinen Methode 1 wurden 28 mg (0.08 mmol) [4-(5-Chlor-2-cyanophenyl)-2,5-dioxo-5,6,7,8-tetrahydrochinolin-1(2*H*)-yl]essigsäure und 17 mg (0.09 mmol, 1.2 eq.) 3-(4-Aminophenyl)-1,2,4-oxadiazol-5(4*H*)-on umgesetzt und mittels präparativer HPLC (Säule: Chromatorex C18, 10 µm, 125 mm x 30 mm, Acetonitril/Wasser-Gradient: 0-3 min 10% Acetonitril, bis 35 min 90% Acetonitril und weitere 3 min 90% Acetonitril). Ausbeute: 18 mg (45% d. Th.)
LC/MS [Methode 1]: Rₜ = 0.88 min; MS (ESIpos): m/z = 516 (M+H)⁺
¹H-NMR (400 MHz, DMSO-d₆): δ [ppm] = 12.88 (br. s, 1H), 10.91 (s, 1H), 7.89 (d, 1H), 7.79 (br. s, 4H), 7.64 (dd, 1H), 7.56 (d, 1H), 6.37 (s, 1H), 5.09 (q, 2H), 3.15-2.95 (m, 2H), 2.46-2.38 (m, 2H), 2.11-1.99 (m, 2H).

### B) Bewertung der physiologischen Wirksamkeit

Die Eignung der erfindungsgemäßen Verbindungen zur Behandlung von thromboembolischen Erkrankungen kann in folgenden Assaysystemen gezeigt werden:

### a) Testbeschreibungen (in vitro)

### a.1) Messung der FXIa-Hemmung

Zur Bestimmung der Faktor XIa-Hemmung der erfindungsgemäßen Substanzen wird ein biochemisches Testsystem verwendet, in dem die Umsetzung eines peptidischen Faktor XIa-Substrates zur Ermittlung der enzymatischen Aktivität von humanem Faktor XIa benutzt wird. Dabei spaltet Faktor XIa von dem peptischen Faktor XIa-Substrat das C-terminale Aminomethylcoumarin (AMC) ab, dessen Fluoreszenz gemessen wird. Die Bestimmungen werden in Mikrotiterplatten durchgeführt.

Prüfsubstanzen werden in Dimethylsulfoxid aufgelöst und seriell in Dimethylsulfoxid verdünnt (3000 µM bis 0.0078 µM; resultierende Endkonzentrationen im Test: 50 µM bis 0.00013 µM). Jeweils 1 µl der verdünnten Substanzlösungen werden in die Vertiefungen von weißen Mikrotiterplatten der Firma Greiner (384 Vertiefungen) vorgelegt. Anschließend werden nacheinander 20 µl Assaypuffer (50 mM Tris/HCl pH 7.4; 100 mM Natriumchlorid-Lösung; 5 mM Calciumchlorid-Lösung; 0,1% bovines Serumalbumin) und 20 µl Faktor XIa der Firma Kordia (0.45 nM in Assaypuffer) hinzugefügt. Nach 15 min Inkubation wird die Enzymreaktion durch Zugabe von 20 µl des in Assaypuffer gelösten Faktor XIa Substrates Boc-Glu(OBzl)-Ala-Arg-AMC (10 µM in Assaypuffer) der Firma Bachem gestartet, für 30 min bei Raumtemperatur (22°C) inkubiert und anschließend eine Fluoreszensmessung durchgeführt (Anregung: 360 nM, Emission: 460 nM). Die gemessenen Emissionen der Testansätze mit Prüfsubstanz werden mit denen von Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet. Wirkdaten aus diesem Test sind in der folgenden Tabelle A aufgeführt:

**Tabelle A**

| **Beispiel-Nr.** | **IC₅₀ [nM]** | | **Beispiel**-**Nr**. | **IC₅₀ [nM]** |
|---|---|---|---|---|
| 1 | 930 | | 2 | 340 |
| 3 | 230 | | 4 | 420 |
| 5 | 330 | | 6 | 550 |
| 7 | 130 | | 8 | 470 |
| 9 | 170 | | 10 | 190 |
| 11 | 750 | | 12 | 60 |
| 13 | 34 | | 14 | 140 |
| 15 | 98 | | 16 | 77 |

### a.2) Bestimmung der Selektivität

Zum Nachweis der Selektivität der Substanzen bezüglich FXIa-Hemmung werden die Prüfsubstanzen auf ihre Hemmung anderer humaner Serinproteasen wie Faktor Xa, Trypsin und Plasmin hin untersucht. Zur Bestimmung der enzymatischen Aktivität von Faktor Xa (1.3 nmol/l von Kordia), Trypsin (83 mU/ml von Sigma) und Plasmin (0.1 µg/ml von Kordia) werden diese Enzyme gelöst (50 mmol/l Tris-Puffer [C,C,C-Tris(hydroxymethyl)-aminomethan], 100 mmol/l Natriumchlorid, 0.1% BSA [bovines Serumalbumin], 5 mmol/l Calciumchlorid, pH 7.4) und für 15 min mit Prüfsubstanz in verschiedenen Konzentrationen in Dimethylsulfoxid sowie mit Dimethylsulfoxid ohne Prüfsubstanz inkubiert. Anschließend wird die enzymatische Reaktion durch Zugabe der entsprechenden Substrate gestartet (5 µmol/l Boc-Ile-Glu-Gly-Arg-AMC von Bachem für Faktor Xa und Trypsin, 5 50 µmol/l MeOSuc-Ala-Phe-Lys-AMC von Bachem für Plasmin). Nach einer Inkubationszeit von 30 min bei 22°C wird die Fluoreszenz gemessen (Anregung: 360 nm, Emission: 460 nm). Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.3) Thrombin Generation Assay (Thrombogram)

Die Wirkung der Prüfsubstanzen auf das Thrombogram (Thrombin Generation Assay nach Hemker) wird in vitro in Humanplasma (Octaplas® von der Firma Octapharma) bestimmt.

Im Thrombin Generation Assay nach Hemker wird die Aktivität von Thrombin in gerinnendem Plasma durch die Messung der fluoreszenten Spaltprodukte des Substrats I-1140 (Z-Gly-Gly-Arg-AMC, Bachem) bestimmt. Die Reaktionen werden in Gegenwart variierender Konzentrationen an Prüfsubstanz oder dem entsprechenden Lösungsmittel durchgeführt. Um die Reaktion zu starten werden Reagenzien der Firma Thrombinoscope verwendet (30 pM or 0.1 pM recombinant tissue factor, 24 µM phospholipids in HEPES). Außerdem wird ein Thrombin Calibrator der Firma Thrombinoscope verwendet, dessen amidolytische Aktivität zur Berechnung der Thrombinaktivität in einer Probe mit unbekannter Menge an Thrombin benötigt wird. Die Testdurchführung erfolgt nach Herstellerangaben (Thrombionsocpe BV): 4 µl der Prüfsubstanz oder des Lösungsmittels, 76 µl Plasma und 20 µl PPP-Reagenz oder Thrombin Calibrator werden 5 min bei 37°C inkubiert. Nach Zugabe von 20 µl 2.5 mM Thrombinsubstrat in 20 mM Hepes, 60 mg/ml BSA, 102 mM Calciumchlorid wird die Thrombin Generierung 120 min alle 20 s gemessen. Die Messung wird mit einem Fluorometer (Fluoroskan Ascent) der Firma Thermo Electron durchgeführt, der mit einem 390/460 nM Filterpaar und einem Dispenser ausgestattet ist.

Durch die Verwendung der "thrombinoscope software" wird das Thrombogramm berechnet und graphisch dargestellt. Die folgenden Parameter werden berechnet: lag time, time to Peak, Peak, ETP (endogenous thrombin potential) und start tail.

### a.4) Bestimmung der antikoagulatorischen Wirkung

Die antikoagulatorische Wirkung der Prüfsubstanzen wird in vitro in Human- und Rattenplasma bestimmt. Dazu wird Blut unter Verwendung einer 0.11 molaren Natriumcitrat-Lösung als Vorlage in einem Mischungsverhältnis Natriumcitrat/Blut 1/9 abgenommen. Das Blut wird unmittelbar nach der Abnahme gut gemischt und 15 Minuten bei ca. 4000 g zentrifugiert. Der Überstand wird abpipettiert.

Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Neoplastin® von der Firma Boehringer Mannheim oder Hemoliance® RecombiPlastin von der Firma Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

Die aktivierte partielle Thromboplastinzeit (APTT) wird in Gegenwart variierender Konzentrationen an Prüfsubstanz oder dem entsprechenden Lösungsmittel mit einem handelsüblichen Testkit (PTT Reagent von der Firma Roche) bestimmt. Die Testverbindungen werden 3 Minuten bei 37°C mit dem Plasma und dem PTT Reagenz (Cephalin, Kaolin) inkubiert. Anschließend wird durch Zugabe von 25 mM Calciumchlorid die Gerinnung ausgelöst und der Zeitpunkt des Gerinnungseintritts bestimmt. Es wird die Konzentration an Prüfsubstanz ermittelt, die eine 50%ige Verlängerung beziehungsweise ein Verdoppelung der APTT bewirkt.

### a.5) Bestimmung der Plasma-Kallikrein Aktivität

Zur Bestimmung der Plasma Kallikrein-Hemmung der erfindungsgemäßen Substanzen wird ein biochemisches Testsystem verwendet, in dem die Umsetzung eines peptidischen Plasma Kallikrein-Substrates zur Ermittlung der enzymatischen Aktivität von humanem Plasma Kallikrein benutzt wird. Dabei spaltet Plasma Kallikrein von dem peptischen Plasma Kallikrein-Substrat das C-terminale Aminomethylcoumarin (AMC) ab, dessen Fluoreszenz gemessen wird. Die Bestimmungen werden in Mikrotiterplatten durchgeführt.

Prüfsubstanzen werden in Dimethylsulfoxid aufgelöst und seriell in Dimethylsulfoxid verdünnt (3000 µM bis 0.0078 µM; resultierende Endkonzentrationen im Test: 50 µM bis 0.00013 µM). Jeweils 1 µl der verdünnten Substanzlösungen werden in die Vertiefungen von weißen Mikrotiterplatten der Firma Greiner (384 Vertiefungen) vorgelegt. Anschließend werden nacheinander 20 µl Assaypuffer (50 mM Tris/HCl pH 7,4; 100 mM Natriumchlorid-Lösung; 5 mM Calciumchlorid-Lösung; 0.1% bovines Serumalbumin) und 20 µl Plasma-Kallikrein der Firma Kordia (0.6 nM in Assaypuffer) hinzugefügt. Nach 15 min Inkubation wird die Enzymreaktion durch Zugabe von 20 µl des in Assaypuffer gelösten Substrates H-Pro-Phe-Arg-AMC (10 µM in Assaypuffer) der Firma Bachem gestartet, für 30 min bei Raumtemperatur (22°C) inkubiert und anschließend eine Fluoreszensmessung durchgeführt (Anregung: 360 nM, Emission: 460 nM). Die gemessenen Emissionen der Testansätze mit Prüfsubstanz werden mit denen von Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen IC₅₀-Werte berechnet.

### a.6) Bestimmung der Endothel Integrität

Die Aktivität der erfindungsgemäßen Verbindungen werden mittels eines in-vitro Permeabilitäts-Assays auf "human umbilical venous cells" (HUVEC) charakterisiert. Mittels des EOS Apparatus (EC IS: Electric Cell-substrate Impedance Sensing; Applied Biophysics Inc; Troy, NY) können Unterschiede des transendothelialen elektrischen Widerstandes (TEER) über einer endothelialen Zell-Monoschicht, die über Gold-Elektroden plattiert wurde, kontinuierlich gemessen werden. HUVECs werden auf einer 96-well sensor Elektrodenplatte (96W1 E, Ibidi GmbH, Martinsried) ausgesäht. Eine Hyperpermeabilität der enstandenen konfluenten Zell-Monoschicht wird mittels Stimulation mit Kininogen, Prekallikrein und Faktor XII (je 100 nM) induziert. Die erfindungsgemäßen Verbindungen werden vor der Zugabe der oben angegebenen Substanzen zugegeben. Die üblichen Konzentationen der Verbindungen sind 1 x 10⁻¹⁰ bis 1 x 10⁻⁶ M.

### a.7) Bestimmung der in-vitro Permeabilität von Endothelzellen

In einem weiteren Hyperpermeabilitäts-Modell wird die Aktivität der Substanzen auf die Modulation der makromolekularen Permeabilität bestimmt. HUVECs werden auf einer Fibronektin-überzogenen Transwell Filter Membran (24-well plates, 6.5 mm-Einsatz mit 0.4 µM Polykarbonat Membran; Costar #3413) ausgesäht. Die Filtermembran trennt den oberen von dem unteren Zellkulturraum mit der konfluenten Endothelzellschicht auf dem Boden des oberen Zellkulturraumes. Dem Medium des oberen Raumes wird 250 g/ml 40 kDa FITC-Dextan (Invitrogen, D1844) zugesetzt. Die Hyperpermeabilität der Monolayer-Schicht wird mittels Stimulation mit Kininogen, Prekallikrein und Faktor XII (je 100 nM) induziert. Medium Proben werden alle 30 min aus der unteren Kammer entnommen und die relative Fluoreszenz, als Parameter für die Veränderungen der makromolekularen Permeabilität in Abhängigkeit von der Zeit, mit einem Fluorimeter bestimmt. Die erfindungsgemäßen Verbindungen werden vor der Zugabe der oben angegebenen Substanzen zugegeben. Die üblichen Konzentationen der Verbindungen sind 1 x 10⁻¹⁰ bis 1 x 10⁻⁶ M.

### b) Bestimmung der antithrombotischen Wirkung (in vivo)

### b.1) Arterielles Thrombose-Modell (Eisen(II)chlorid-induzierte Thrombose) in Kombination mit Ohrblutungszeit im Kaninchen

Die antithrombotische Aktivität der FXIa-Inhibitoren wird in einem arteriellen Thrombose-Modell getestet. Dabei wird die Thrombusbildung durch chemische Beschädigung eines Bereichs der Arteria carotis im Kaninchen ausgelöst. Simultan wird die Ohrblutungszeit bestimmt.

Männliche Kaninchen (Crl:KBL (NZW)BR, Charles River) unter normaler Diät mit einem Gewicht von 2.2 - 2.5 kg Körpergewicht werden durch intramuskuläre Applikation von Xylazin und Ketamin (Rompun, Bayer, 5 mg/kg und Ketavet, Pharmacia & Upjohn GmbH, 40 mg/kg Körpergewicht) anästhesiert. Die Anästhesie wird weiterhin durch intravenöse Gabe derselben Präparate (bolus: Dauerinfusion) über die rechte Ohrvene unterstützt.

Nach Freipräparation der rechten Arteria carotis wird der Gefäßschaden dadurch erzeugt, dass ein Stück Filterpapier (10 mm x 10 mm) auf einem Streifen Parafilm® (25 mm x 12 mm) um die A. carotis gewickelt wird, ohne den Blutfluß dadurch zu beeinträchtigen. Das Filterpapier enthält 100 µL einer 13%igen Lösung von Eisen(II)chlorid (Sigma) in Wasser. Nach 5 min wird das Filterpapier entfernt und das Gefäß zweimal mit wässriger 0.9%iger Natriumchlorid-Lösung gespült. 30 min nach der Verletzung wird die Arteria carotis im Bereich der Schädigung herauspräpariert und eventuell vorhandenes thrombotisches Material entnommen und gewogen.

Die Prüfsubstanzen werden entweder intravenös über die Vena femoralis den anästhesierten oder oral mittels Schlundsonde den wachen Tieren jeweils 5 min beziehungsweise 2 h vor Schädigung verabreicht.

Die Ohrblutungszeit wird 2 min nach der Schädigung der Arteria carotis bestimmt. Hierzu wird das linke Ohr rasiert und ein definierter Schnitt von 3 mm Länge (Klinge Art.Nummer 10-150-10, Martin, Tuttlingen, Germany) parallel zur Ohrlängsachse gesetzt. Dabei wird darauf geachtet, kein sichtbares Gefäß zu verletzen. Eventuell austretendes Blut wird in 15 Sekunden-Intervallen mit genau gewogenen Filterpapierstücken aufgenommen, ohne die Wunde direkt zu berühren. Die Blutungszeit wird berechnet als die Zeitspanne vom Setzen des Schnitts bis zu dem Zeitpunkt, an dem am Filterpapier kein Blut mehr nachweisbar ist. Das ausgetretene Blutvolumen wird nach Wiegen der Filterpapierstücke berechnet.

### c) Bestimmung der Wirkung auf die Extravasation/Ödembildung und/oder Neovaskularisierung im Auge (in vivo)

### c.1) Testung der Wirksamkeit von Substanzen im Laser-induzierten choroidalen Neovaskularisierungs-Modell

Diese Studie dient dem Zweck, die Wirksamkeit einer Testsubstanz auf die Reduktion der Extravasation/Ödembildung und/oder der choroidalen Neovaskularisierung im Rattenmodell der Laser-induzierten choroidalen Neovaskularisierung zu untersuchen.

Dafür werden pigmentierte Ratten vom Stamm Brown-Norway, die keine Anzeichen ophthalmologischer Erkrankungen aufweisen, ausgewählt und nach dem Zufallsprinzip Behandlungsgruppen zugeordnet. Am Tag 0 werden die Tiere mittels intraperitonealer Injektion narkotisiert (15 mg/kg Xylazin und 80 mg/kg Ketamin). Nach Instillation eines Tropfens einer 0.5%igen Tropicamid-Lösung zur Weitstellung der Pupillen wird die choroidale Neovaskularisierung mittels eines 532 nm Argon Laser Photokoagulators an sechs definierten Stellen um den Nervus opticus herum ausgelöst (50-75 µm Durchmesser, 150 mW Intensität, 100 ms Dauer). Die Testsubstanz und das entsprechende Vehikel (z.B. PBS, isotone Kochsalzlösung) werden entweder systemisch oral beziehungsweise intraperitonal appliziert oder lokal am Auge durch mehrfache Gabe als Augentropfen beziehungsweise intravitreale Injektion verabreicht. Das Körpergewicht aller Tiere wird vor Beginn der Studie, und anschließend täglich während der Studie bestimmt.

An Tag 21 wird eine Angiographie mittels einer Fluoreszenz-Funduskammera (z.B. Kowe, HRA) durchgeführt. In Narkose und nach erneuter Pupillenerweiterung wird ein 10%iger Natrium-Fluorescein-Farbstoff subkutan (s.c.) injiziert. 2-10 min später werden Bilder des Augenhintergrundes aufgenommen. Die Stärke der Extravasation/des Ödems, dargestellt durch die Leckage von Fluorescein, wird von zwei bis drei verblindeten Beobachtern beurteilt und in Schweregrade von 0 (keine Extravasation) bis 3 (starke Einfärbung über die eigentliche Läsion hinaus) eingeteilt.

Nach Abtöten der Tiere an Tag 23 werden die Augen entnommen und in 4%iger Paraformaldehyd-Lösung für eine Stunde bei Raumtemperatur fixiert. Nach einem Waschdurchgang wird die Retina vorsichtig herausgeschält, und der Sklera-Choroidea-Komplex wird mit einem FITC-Isolektin B4 Antikörper angefärbt und anschließend flach auf einen Objetträger aufgebracht. Die so erhaltenen Präparate werden mittels eines Fluoreszenz-Mikroskops (Apotom, Zeiss) bei einer Anregungs-Wellenlänge von 488 nm ausgewertet. Die Fläche beziehungsweise das Volumen der choroidalen Neovaskularisierung (in µm² bzw. µm³) wird durch morphometrische Analyse mittels Axiovision 4.6 Software berechnet.

### c.2) Testung der Wirksamkeit von Substanzen im Sauerstoff-induzierten Retinopathie Modell

Es wurde gezeigt, dass eine durch Sauerstoff induzierte Retinopathie ein wertvolles Tiermodell für die Untersuchung der pathologischen retinalen Angiogenese darstellt. Dieses Modell basiert auf der Beobachtung, dass eine Hyperoxie während der frühen postnatalen Entwicklung in der Retina zum Anhalten oder zur Verlangsamung des Wachstums von normalen retinalen Blutgefäßen führt. Sobald die Tiere nach einer 7-tägigen Hyperoxiephase zur normoxischen Raumluft zurückkehren, ist dieses gleichbedeutend einer relativen Hypoxie, da in der Retina die normalen Gefäße fehlen, welche erforderlich sind, um eine ausreichende Versorgung des neuralen Gewebes unter normoxischen Bedingungen zu gewährleisten. Die dadurch erzeugte ischämische Situation führt zur abnormen Neovaskularisation, die einige Ähnlichkeiten mit der pathophysiologischen Neovaskularisation in Augenerkrankungen wie der feuchten AMD aufzeigt. Darüber hinaus ist die hervorgerufene Neovaskularisierung sehr reproduzierbar, quantifizierbar und ein wichtiger Parameter für die Untersuchung der Krankheitsmechanismen und möglichen Behandlungen für verschiedenste Formen von Netzhauterkrankungen.

Das Ziel dieser Studie ist es, die Wirksamkeit von täglich systemisch verabreichten Dosen der Testverbindung auf das Wachstum der retinalen Gefäße im Sauerstoff-induzierten Retinopathie-Modell zu untersuchen. Neugeborene von C57B1 / 6 Mäusen und ihre Mütter werden am postnatalen Tag 7 (PD7) einer Hyperoxie (70% Sauerstoff) für 5 Tage ausgesetzt. Ab PD12, werden die Mäuse unter normoxischen Bedingungen (Raumluft, 21% Sauerstoff) bis zum PD17 gehalten. Von Tag 12 bis Tag 17 werden die Mäuse täglich mit der Testsubstanz oder dem entsprechenden Vehikel behandelt. Am Tag 17 werden alle Mäuse mit Isofluran narkotisiert und anschließend durch Genickbruch abgetötet. Die Augen werden entnommen und in 4% Formalin fixiert. Nach dem Waschen in Phosphat-gepufferter Kochsalzlösung wird die Netzhaut präpariert, ein Flachpräperat davon erzeugt und dieses mit Isolectin B4 Antikörper gefärbt. Die Quantifizierung der neugewachsenen Gefäße wird unter Verwendung eines Zeiss ApoTome durchgeführt.

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6 h gerührt.

### Lösung oder Suspension zur topischen Anwendung am Auge (Augentropfen):

Eine sterile pharmazeutische Zubereitung zur topischen Anwendung am Auge kann durch Rekonstitution eines Lyophilisats der erfindungsgemäßen Verbindung in steriler Kochsalz-Lösung hergestellt werden. Als Konservierungsmittel für eine solche Lösung oder Suspension ist beispielsweise Benzalkoniumchlorid, Thiomersal oder Phenylquecksilbernitrat in einem Konzentrationsbereich von 0.001 bis 1 Gewichtsprozent geeignet.

## Patentansprüche

1. Verbindung der Formel 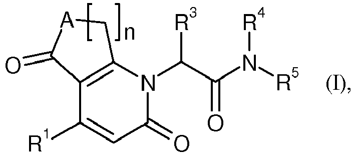 in welcher
n für die Zahl 1 oder 2 steht,
A für -N(R²)- oder -CH₂- steht, wobei
R² für Wasserstoff oder C₁-C₄-Alkyl steht,
R¹ für eine Gruppe der Formel 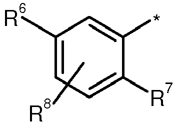 steht,
wobei * die Anknüpfstelle an den Oxopyridinring ist,
R⁶ für Brom, Chlor, Fluor, Methyl, Difluormethyl, Trifluormethyl, Methoxy, Difluormethoxy oder Trifluormethoxy steht,
R⁷ für Wasserstoff, Brom, Chlor, Fluor, Cyano, Nitro, Hydroxy, Methyl, Difluormethyl, Trifluormethyl, Methoxy, Ethoxy, Difluormethoxy, Trifluormethoxy, Ethinyl, 3,3,3-Trifluorprop-1-in-1-yl oder Cyclopropyl steht,
R⁸ für Wasserstoff, Chlor oder Fluor steht,
R³ für Wasserstoff steht,
R⁴ für Wasserstoff steht,
R⁵ für eine Gruppe der Formel 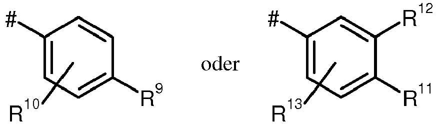 steht,
wobei # die Anknüpfstelle an das Stickstoffatom ist,
R⁹ für Hydroxycarbonyl oder 5-gliedriges Heterocyclyl steht, wobei Heterocyclyl substituiert sein kann mit 1 bis 2 Substituenten unabhängig voneinander ausgewählt aus der Gruppe bestehend aus Oxo, Hydroxy, Thioxo, Sulfanyl, Methyl, Difluormethyl, Trifluormethyl, 2-Hydroxycarbonyl-1,1,2,2-tetrafluorethyl und 2-Methoxycarbonyl-1,1,2,2-tetrafluorethyl,
worin Methyl substituiert sein kann mit einem Substituenten Methoxy,
R¹⁰ für Wasserstoff, Chlor, Fluor oder Methyl steht,
R¹¹ und R¹² zusammen mit den Kohlenstoffatomen an die sie gebunden sind einen 5-gliedrigen Heterocyclus bilden,
wobei der Heterocyclus substituiert sein kann mit 1 bis 2 Substituenten unabhängig voneinander ausgewählt aus der Gruppe bestehend aus Oxo, Chlor, Hydroxy, Hydroxycarbonyl, Methyl, Difluormethyl, Trifluormethyl, 1,1,2,2,2-Pentafluorethyl, 2-Hydroxycarbonyl-1,1,2,2-tetrafluorethyl und 2-Methoxy-carbonyl-1,1,2,2-tetrafluorethyl,
R¹³ für Wasserstoff, Chlor, Fluor, Methyl oder Methoxy steht,
oder eines ihrer Salze, ihrer Solvate oder der Solvate ihrer Salze.

2. Verbindung nach Anspruch 1, **dadurch gekennzeichnet, dass**
n für die Zahl 1 oder 2 steht,
A für -N(R²)- oder -CH₂- steht,
wobei
R² für Wasserstoff oder Methyl steht,
R¹ für eine Gruppe der Formel 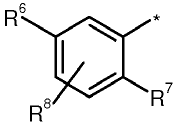 steht,
wobei * die Anknüpfstelle an den Oxopyridinring ist,
R⁶ für Chlor steht,
R⁷ für Wasserstoff, Brom, Chlor, Cyano, Methyl, Difluormethyl, Trifluormethyl, Difluormethoxy oder Trifluormethoxy steht,
R⁸ für Wasserstoff oder Fluor steht,
R³ für Wasserstoff steht,
R⁴ für Wasserstoff steht,
R⁵ für eine Gruppe der Formel 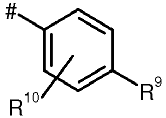 steht,
wobei # die Anknüpfstelle an das Stickstoffatom ist,
R⁹ für Hydroxycarbonyl, Oxadiazolyl, Pyrazolyl, Imidazolyl, Triazolyl oder Tetrazolyl steht,
wobei Oxadiazolyl, Pyrazolyl, Imidazolyl und Triazolyl substituiert sein können mit 1 bis 2 Substituenten unabhängig voneinander ausgewählt aus der Gruppe bestehend aus Oxo, Hydroxy, Methyl und Trifluormethyl,
R¹⁰ für Wasserstoff steht,
oder eines ihrer Salze, ihrer Solvate oder der Solvate ihrer Salze.

3. Verbindung nach einem der Ansprüche 1 oder 2, **dadurch gekennzeichnet, dass**
n für die Zahl 1 oder 2 steht,
A für -CH₂- steht,
R¹ für eine Gruppe der Formel 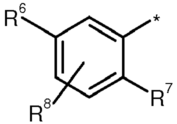 steht,
wobei * die Anknüpfstelle an den Oxopyridinring ist,
R⁶ für Chlor steht,
R⁷ für Brom oder Cyano steht,
R⁸ für Wasserstoff steht,
R³ für Wasserstoff steht,
R⁴ für Wasserstoff steht,
R⁵ für eine Gruppe der Formel 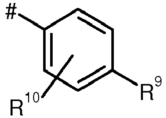 steht,
wobei # die Anknüpfstelle an das Stickstoffatom ist,
R⁹ für Hydroxycarbonyl, Oxadiazolyl, Pyrazolyl oder Tetrazolyl steht, wobei Oxadiazolyl und Pyrazolyl substituiert sein können mit 1 bis 2 Substituenten unabhängig voneinander ausgewählt aus der Gruppe bestehend aus Oxo, Hydroxy und Trifluormethyl,
R¹⁰ für Wasserstoff steht,
oder eines ihrer Salze, ihrer Solvate oder der Solvate ihrer Salze.

4. Verfahren zur Herstellung einer Verbindung der Formel (I) oder eines ihrer Salze, ihrer Solvate oder der Solvate ihrer Salze nach Anspruch 1, **dadurch gekennzeichnet, dass** entweder
[A] eine Verbindung der Formel 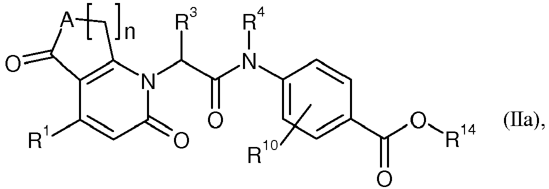 in welcher
n, A, R¹, R³, R⁴ und R¹⁰ die in Anspruch 1 angegebene Bedeutung haben, und
R¹⁴ für tert-Butyl steht,
mit einer Säure zu einer Verbindung der Formel 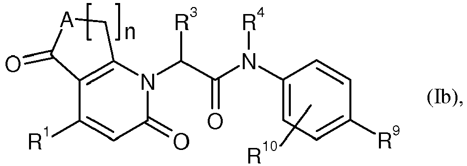 in welcher
n, A, R¹, R³, R⁴ und R¹⁰ die in Anspruch 1 angegebene Bedeutung haben, und
R⁹ für Hydroxycarbonyl steht,
umgesetzt wird,
oder
[B] eine Verbindung der Formel 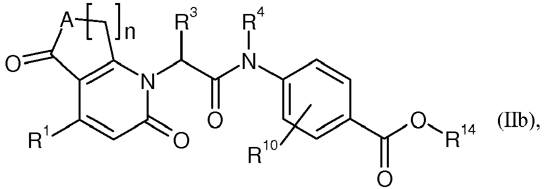 in welcher
n, A, R¹, R³, R⁴ und R¹⁰ die in Anspruch 1 angegebene Bedeutung haben, und
R¹⁴ für Methyl oder Ethyl steht,
mit einer Base zu einer Verbindung der Formel 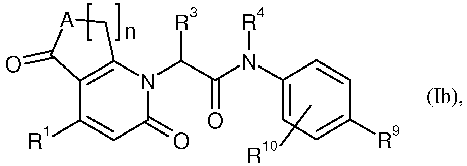 in welcher
n, A, R¹, R³, R⁴ und R¹⁰ die in Anspruch 1 angegebene Bedeutung haben, und
R⁹ für Hydroxycarbonyl steht,
umgesetzt wird,
oder
[C] eine Verbindung der Formel 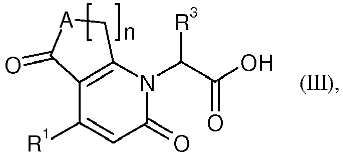 in welcher
n, A, R¹ und R³ die in Anspruch 1 angegebene Bedeutung haben,
mit einer Verbindung der Formel 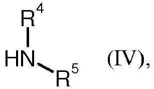 in welcher
R⁴ und R⁵ die in Anspruch 1 angegebene Bedeutung haben,
in Gegenwart eines Dehydratisierungsreagenzes zu einer Verbindung der Formel (I) umgesetzt wird,
oder
[D] eine Verbindung der Formel 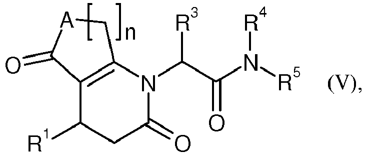 in welcher
n, A, R¹, R³, R⁴ und R⁵ die in Anspruch 1 angegebene Bedeutung haben,
mit einem Oxidationsmittel umgesetzt wird.

5. Verbindung nach einem der Ansprüche 1 bis 3 zur Behandlung und/oder Prophylaxe von Krankheiten.

6. Verwendung einer Verbindung nach einem der Ansprüche 1 bis 3 zur Herstellung eines Arzneimittels zur Behandlung und/oder Prophylaxe von Krankheiten.

7. Verwendung einer Verbindung nach einem der Ansprüche 1 bis 3 zur Herstellung eines Arzneimittels zur Behandlung und/oder Prophylaxe von thrombotischen beziehungsweise thromboembolischen Erkrankungen.

8. Verwendung einer Verbindung nach einem der Ansprüche 1 bis 3 zur Herstellung eines Arzneimittels zur Behandlung und/oder Prophylaxe von ophthalmologischen Erkrankungen.

9. Verwendung einer Verbindung nach einem der Ansprüche 1 bis 3 zur Herstellung eines Arzneimittels zur Behandlung und/oder Prophylaxe von hereditärem Angioödem oder entzündlichen Erkrankungen des Darms, wie Morbus Crohn oder Colitis ulcerosa.

10. Arzneimittel enthaltend eine Verbindung nach einem der Ansprüche 1 bis 3 in Kombination mit einem inerten, nichttoxischen, pharmazeutisch geeigneten Hilfsstoff.

11. Arzneimittel nach Anspruch 10 zur Behandlung und/oder Prophylaxe von thrombotischen beziehungsweise thromboembolischen Erkrankungen.

12. Arzneimittel nach Anspruch 10 zur Behandlung und/oder Prophylaxe von ophthalmologischen Erkrankungen.

13. Arzneimittel nach Anspruch 10 zur Behandlung und/oder Prophylaxe von hereditärem Angioödem oder entzündlichen Erkrankungen des Darms, wie Morbus Crohn oder Colitis ulcerosa.

## Claims

1. Compound of the formula 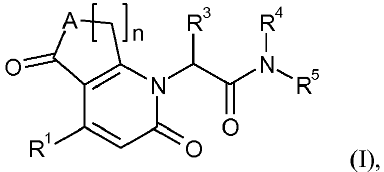 in which
n represents the number 1 or 2,
A represents -N(R²)- or -CH₂-, wherein
R² represents hydrogen or C₁-C₄-alkyl,
R¹ represents a group of the formula 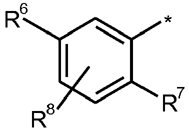 where * is the point of attachment to the oxopyridine ring,
R⁶ represents bromine, chlorine, fluorine, methyl, difluoromethyl, trifluoromethyl, methoxy, difluoromethoxy or trifluoromethoxy,
R⁷ represents hydrogen, bromine, chlorine, fluorine, cyano, nitro, hydroxy, methyl, difluoromethyl, trifluoromethyl, methoxy, ethoxy, difluoromethoxy, trifluoromethoxy, ethynyl, 3,3,3-trifluoroprop-1-yn-1-yl or cyclopropyl,
R⁸ represents hydrogen, chlorine or fluorine,
R³ represents hydrogen,
R⁴ represents hydrogen,
R⁵ represents a group of the formula 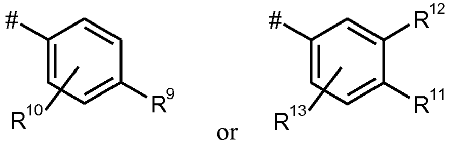 where # is the attachment site to the nitrogen atom,
R⁹ represents hydroxycarbonyl or 5-membered heterocyclyl, where heterocyclyl may be substituted by 1 to 2 substituents independently of one another selected from the group consisting of oxo, hydroxy, thioxo, sulphanyl, methyl, difluoromethyl, trifluoromethyl, 2-hydroxycarbonyl-1,1,2,2-tetrafluoroethyl and 2-methoxycarbonyl-1,1,2,2-tetrafluoroethyl,
where methyl may be substituted by a methoxy substituent,
R¹⁰ represents hydrogen, chlorine, fluorine or methyl,
R¹¹ and R¹² together with the carbon atoms to which they are bonded form a 5-membered heterocycle, where the heterocycle may be substituted by 1 to 2 substituents independently of one another selected from the group consisting of oxo, chlorine, hydroxy, hydroxycarbonyl, methyl, difluoromethyl, trifluoromethyl, 1,1,2,2,2-pentafluoroethyl, 2-hydroxycarbonyl-1,1,2,2-tetrafluoroethyl and 2-methoxycarbonyl-1,1,2,2-tetrafluoroethyl,
R¹³ represents hydrogen, chlorine, fluorine, methyl or methoxy,
or one of the salts thereof, solvates thereof or solvates of the salts thereof.

2. Compound according to Claim 1, **characterized in that**
n represents the number 1 or 2,
A represents -N(R²)- or -CH₂-, wherein
R² represents hydrogen or methyl,
R¹ represents a group of the formula 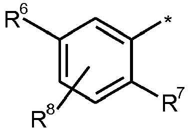 where * is the point of attachment to the oxopyridine ring,
R⁶ represents chlorine,
R⁷ represents hydrogen, bromine, chlorine, cyano, methyl, difluoromethyl, trifluoromethyl, difluoromethoxy or trifluoromethoxy,
R⁸ represents hydrogen or fluorine,
R³ represents hydrogen,
R⁴ represents hydrogen,
R⁵ represents a group of the formula 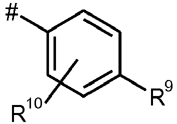 where # is the attachment site to the nitrogen atom,
R⁹ represents hydroxycarbonyl, oxadiazolyl, pyrazolyl, imidazolyl, triazolyl or tetrazolyl, where oxadiazolyl, pyrazolyl, imidazolyl and triazolyl may be substituted by 1 to 2 substituents independently of one another selected from the group consisting of oxo, hydroxy, methyl and trifluoromethyl,
R¹⁰ represents hydrogen,
or one of the salts thereof, solvates thereof or solvates of the salts thereof.

3. Compound according to either of Claims 1 and 2, **characterized in that**
n represents the number 1 or 2,
A represents -CH₂-,
R¹ represents a group of the formula 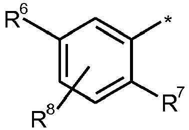 where * is the point of attachment to the oxopyridine ring,
R⁶ represents chlorine,
R⁷ is bromine or cyano,
R⁸ represents hydrogen,
R³ represents hydrogen,
R⁴ represents hydrogen,
R⁵ represents a group of the formula 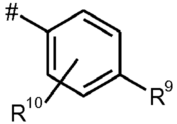 where # is the attachment site to the nitrogen atom,
R⁹ represents hydroxycarbonyl, oxadiazolyl, pyrazolyl or tetrazolyl,
where oxadiazolyl and pyrazolyl may be substituted by 1 to 2 substituents independently of one another selected from the group consisting of oxo, hydroxy and trifluoromethyl,
R¹⁰ represents hydrogen,
or one of the salts thereof, solvates thereof or solvates of the salts thereof.

4. Process for preparing a compound of the formula (I) or one of the salts thereof, solvates thereof or solvates of the salts thereof according to Claim 1, **characterized in that** either
[A] a compound of the formula 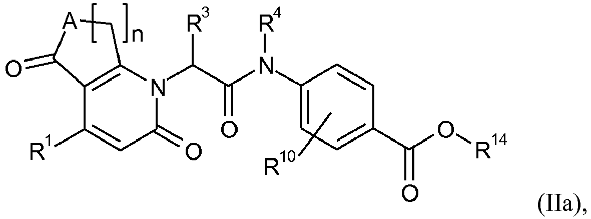 in which
n, A, R¹, R³, R⁴ and R¹⁰ are each as defined in Claim 1, and
R¹⁴ represents tert-butyl,
is reacted with an acid to give a compound of the formula 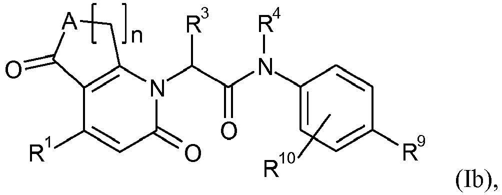 in which
n, A, R¹, R³, R⁴ and R¹⁰ are each as defined in Claim 1, and R⁹ represents hydroxycarbonyl,
or
[B] a compound of the formula 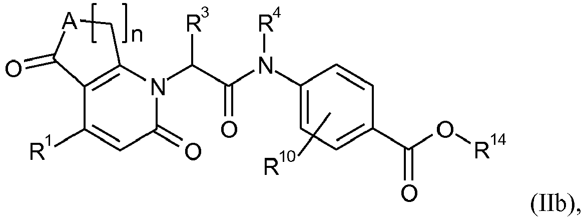 in which
n, A, R¹, R³, R⁴ and R¹⁰ are each as defined in Claim 1, and
R¹⁴ represents methyl or ethyl,
is reacted with a base to give a compound of the formula 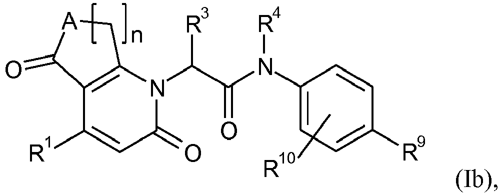 in which
n, A, R¹, R³, R⁴ and R¹⁰ are each as defined in Claim 1, and
R⁹ represents hydroxycarbonyl,
or
[C] a compound of the formula 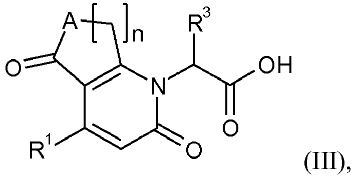 in which
n, A, R¹ and R³ are each as defined in Claim 1,
is reacted with a compound of the formula 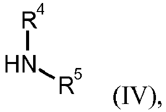 in which
R⁴ and R⁵ are each as defined in Claim 1,
in the presence of a dehydrating reagent to give a compound of the formula (I),
or
[D] a compound of the formula 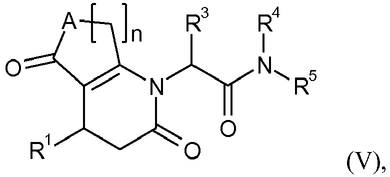 in which
n, A, R¹, R³, R⁴ and R⁵ are each as defined in Claim 1,
is reacted with an oxidizing agent.

5. Compound according to any of Claims 1 to 3 for the treatment and/or prophylaxis of diseases.

6. Use of a compound according to any of Claims 1 to 3 for producing a medicament for the treatment and/or prophylaxis of diseases.

7. Use of a compound according to any of Claims 1 to 3 for producing a medicament for the treatment and/or prophylaxis of thrombotic or thromboembolic disorders.

8. Use of a compound according to any of Claims 1 to 3 for producing a medicament for the treatment and/or prophylaxis of ophthalmic disorders.

9. Use of a compound according to any of Claims 1 to 3 for producing a medicament for the treatment and/or prophylaxis of hereditary angiooedema or inflammatory disorders of the intestine, such as Crohn's disease or ulcerative colitis.

10. Medicament comprising a compound according to any of Claims 1 to 3 in combination with an inert, nontoxic, pharmaceutically suitable excipient.

11. Medicament according to Claim 10 for treatment and/or prophylaxis of thrombotic or thromboembolic disorders.

12. Medicament according to Claim 10 for the treatment and/or prophylaxis of ophthalmic disorders.

13. Medicament according to Claim 10 for the treatment and/or prophylaxis of hereditary angiooedema or inflammatory disorders of the intestine, such as Crohn's disease or ulcerative colitis.

## Revendications

1. Composé de formule 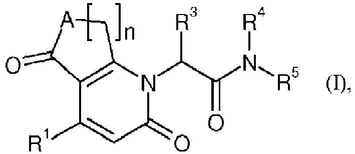 dans laquelle
n représente le nombre 1 ou 2,
A représente -N(R²)- ou -CH₂-,
où
R² représente hydrogène ou C₁-C₄-alkyle,
R¹ représente un groupe de formule 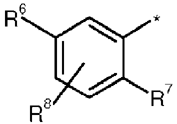 où * est le site de liaison au cycle oxopyridine,
R⁶ représente brome, chlore, fluor, méthyle, difluorométhyle, trifluorométhyle, méthoxy, difluorométhoxy ou trifluorométhoxy,
R⁷ représente hydrogène, brome, chlore, fluor, cyano, nitro, hydroxy, méthyle, difluorométhyle, trifluorométhyle, méthoxy, éthoxy, difluorométhoxy, trifluorométhoxy, éthynyle, 3,3,3-trifluoroprop-1-yn-1-yle ou cyclopropyle,
R⁸ représente hydrogène, chlore ou fluor,
R³ représente hydrogène,
R⁴ représente hydrogène,
R⁵ représente un groupe de formule 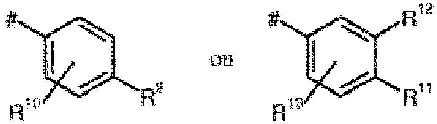 où # est le site de liaison à l'atome d'azote,
R⁹ représente hydroxycarbonyle ou hétérocyclyle à 5 chaînons, hétérocyclyle pouvant être substitué par 1 à 2 substituants choisis indépendamment l'un de l'autre dans le groupe constitué par oxo, hydroxy, thioxo, sulfanyle, méthyle, difluorométhyle, trifluorométhyle, 2-hydroxycarbonyl-1,1,2,2-tétrafluoréthyle et 2-méthoxycarbonyl-1,1,2,2-tétrafluoréthyle,
méthyle pouvant être substitué par un substituant méthoxy,
R¹⁰ représente hydrogène, chlore, fluor ou méthyle,
R¹¹ et R¹² ensemble avec les atomes de carbone auxquels ils sont liés forment un hétérocycle à 5 chaînons, l'hétérocycle pouvant être substitué par 1 à 2 substituants choisis indépendamment l'un de l'autre dans le groupe constitué par oxo, chlore, hydroxy, hydroxycarbonyle, méthyle, difluorométhyle, trifluorométhyle, 1,1,2,2,2-pentafluoroéthyle, 2-hydroxycarbonyl-1,1,2,2-tétrafluoréthyle et 2-méthoxycarbonyl-1,1,2,2-tétrafluoréthyle,
R¹³ représente hydrogène, chlore, fluor, méthyle ou méthoxy,
ou un de ses sels, de ses solvates ou des solvates de ses sels.

2. Composé selon la revendication 1, **caractérisé en ce que**
n représente le nombre 1 ou 2,
A représente -N(R²)- ou -CH₂-,
où
R² représente hydrogène ou méthyle,
R¹ représente un groupe de formule 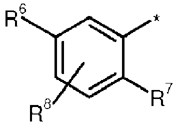 où * est le site de liaison au cycle oxopyridine,
R⁶ représente chlore,
R⁷ représente hydrogène, brome, chlore, cyano, méthyle, difluorométhyle, trifluorométhyle, difluorométhoxy ou trifluorométhoxy,
R⁸ représente hydrogène ou fluor,
R³ représente hydrogène,
R⁴ représente hydrogène,
R⁵ représente un groupe de formule 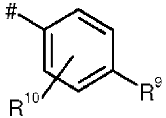 où # est le site de liaison à l'atome d'azote,
R⁹ représente hydroxycarbonyle, oxadiazolyle, pyrazolyle, imidazolyle, triazolyle ou tétrazolyle, oxadiazolyle, pyrazolyle, imidazolyle et triazolyle pouvant être substitués par 1 à 2 substituants choisis indépendamment l'un de l'autre dans le groupe constitué par oxo, hydroxy, méthyle et trifluorométhyle,
R¹⁰ représente hydrogène,
ou un de ses sels, de ses solvates ou des solvates de ses sels.

3. Composé selon l'une quelconque des revendications 1 ou 2, **caractérisé en ce que**
n représente le nombre 1 ou 2,
A représente -CH₂-,
R¹ représente un groupe de formule 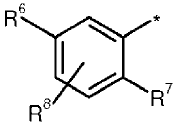 où * est le site de liaison au cycle oxopyridine,
R⁶ représente chlore,
R⁷ représente brome ou cyano,
R⁸ représente hydrogène,
R³ représente hydrogène,
R⁴ représente hydrogène,
R⁵ représente un groupe de formule 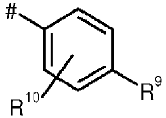 où # est le site de liaison à l'atome d'azote,
R⁹ représente hydroxycarbonyle, oxadiazolyle, pyrazolyle ou tétrazolyle, oxadiazolyle et pyrazolyle pouvant être substitués par 1 à 2 substituants choisis indépendamment l'un de l'autre dans le groupe constitué par oxo, hydroxy et trifluorométhyle,
R¹⁰ représente hydrogène,
ou un de ses sels, de ses solvates ou des solvates de ses sels.

4. Procédé pour la préparation d'un composé de formule (I) ou d'un de ses sels, de ses solvates ou des solvates de ses sels selon la revendication 1, **caractérisé en ce qu'**on transforme soit
[A] un composé de formule 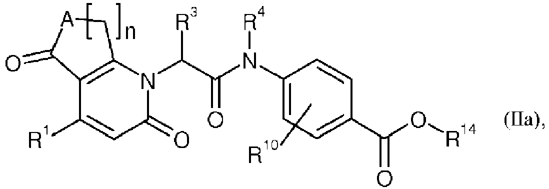 dans laquelle
n, A, R¹, R³, R⁴ et R¹⁰ ont la signification indiquée dans la revendication 1 et
R¹⁴ représente tert-butyle,
avec un acide en un composé de formule 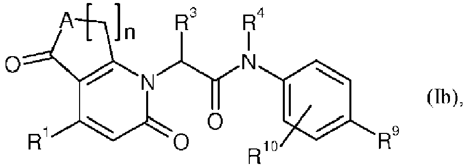 dans laquelle
n, A, R¹, R³, R⁴ et R¹⁰ ont la signification indiquée dans la revendication 1 et
R⁹ représente hydroxycarbonyle,
soit
[B] un composé de formule 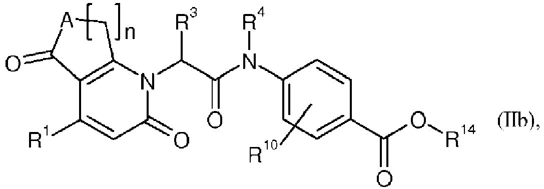 dans laquelle
n, A, R¹, R³, R⁴ et R¹⁰ ont la signification indiquée dans la revendication 1 et
R¹⁴ représente méthyle ou éthyle,
avec une base en un composé de formule 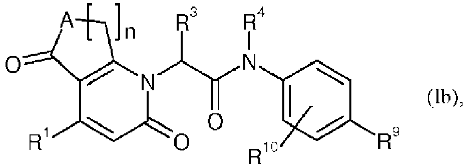 dans laquelle
n, A, R¹, R³, R⁴ et R¹⁰ ont la signification indiquée dans la revendication 1 et
R⁹ représente hydroxycarbonyle,
soit
[C] un composé de formule 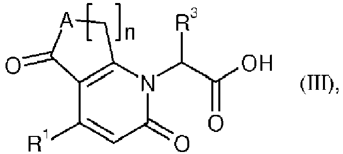 dans laquelle
n, A, R¹ et R³ présentent la signification indiquée dans la revendication 1,
avec un composé de formule 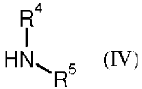 dans laquelle
R⁴ et R⁵ présentent la signification indiquée dans la revendication 1,
en présence d'un réactif de déshydratation en un composé de formule (I), ou
[D] un composé de formule 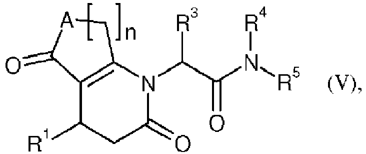 dans laquelle
n, A, R¹, R³, R⁴ et R⁵ présentent la signification indiquée dans la revendication 1,
avec un oxydant.

5. Composé selon l'une quelconque des revendications 1 à 3 destiné au traitement et/ou à la prophylaxie de maladies.

6. Utilisation d'un composé selon l'une quelconque des revendications 1 à 3 pour la préparation d'un médicament destiné au traitement et/ou à la prophylaxie de maladies.

7. Utilisation d'un composé selon l'une quelconque des revendications 1 à 3 pour la préparation d'un médicament destiné au traitement et/ou à la prophylaxie de maladies thrombotiques ou thromboemboliques.

8. Utilisation d'un composé selon l'une quelconque des revendications 1 à 3 pour la préparation d'un médicament destiné au traitement et/ou à la prophylaxie de maladies ophtalmologiques.

9. Utilisation d'un composé selon l'une quelconque des revendications 1 à 3 pour la préparation d'un médicament destiné au traitement et/ou à la prophylaxie d'angio-oedèmes héréditaires ou de maladies inflammatoires de l'intestin, telles que la maladie de Crohn ou la colite ulcéreuse.

10. Médicament contenant un composé selon l'une quelconque des revendications 1 à 3 en combinaison avec un adjuvant inerte, non toxique, pharmaceutiquement approprié.

11. Médicament selon la revendication 10 destiné au traitement et/ou à la prophylaxie de maladies thrombotiques ou thromboemboliques.

12. Médicament selon la revendication 10 destiné au traitement et/ou à la prophylaxie de maladies ophtalmologiques.

13. Médicament selon la revendication 10 pour le traitement et/ou la prophylaxie d'angio-oedèmes héréditaires ou de maladies inflammatoires de l'intestin, telles que la maladie de Crohn ou la colite ulcéreuse.
